(19) Europäisches Patentamt / European Patent Office / Office européen des brevets

(11) **EP 2 145 276 B1**

(12) **EUROPEAN PATENT SPECIFICATION**

(45) Date of publication and mention of the grant of the patent:
**10.06.2020 Bulletin 2020/24**

(21) Application number: **08742637.5**

(22) Date of filing: **07.04.2008**

(51) Int Cl.:
*G16H 50/20* *(2018.01)*

(86) International application number:
**PCT/US2008/004523**

(87) International publication number:
**WO 2008/124138 (16.10.2008 Gazette 2008/42)**

(54) **SYSTEM AND METHOD FOR HANDLING, DIAGNOSE AND PREDICT THE OCCURRENCE OF A MEDICAL CONDITION**

SYSTEM UND VERFAHREN ZUM BEHANDELN, DIAGNOSTIZIEREN UND VORHERSAGEN DES AUFTRETENS EINES MEDIZINISCHEN ZUSTAND

SYSTÈMES ET PROCÉDÉS DESTINÉS À TRAITER, DIAGNOSTIQUER ET PRÉVOIR LA SURVENUE D'UN ÉTAT MÉDICAL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.04.2007 US 922163 P**
**05.04.2007 US 922149 P**
**13.04.2007 US 923447 P**
**09.01.2008 US 10598**

(43) Date of publication of application:
**20.01.2010 Bulletin 2010/03**

(73) Proprietor: **Fundação D. Anna Sommer Champalimaud E**
**Dr. Carlos Montez Champalimaud**
**1400-038 Lisboa (PT)**

(72) Inventors:
• **TEVEROVSKIY, Mikhail**
**Harrison, NY 10528 (US)**
• **VERBEL, David, A.**
**New York, NY 10022 (US)**
• **SAIDI, Olivier**
**Greenwich, CT 06830 (US)**

(74) Representative: **Mewburn Ellis LLP**
**Aurora Building**
**Counterslip**
**Bristol BS1 6BX (GB)**

(56) References cited:
**US-A1- 2005 262 031**

**Description**

**FIELD OF THE INVENTION**

The present disclosure relates to methods and systems that use clinical information, molecular information and computer-generated morphometric information in a predictive model for predicting the occurrence of a medical condition (e.g., disease or responsiveness or unresponsiveness to treatment). For example, in one example, the disclosure relates to methods and systems that use clinical, molecular and morphometric information to treat, diagnose and predict the recurrence of prostate cancer.

**BACKGROUND**

Physicians are required to make many medical decisions ranging from, for example, whether and when a patient is likely to experience a medical condition to how a patient should be treated once the patient has been diagnosed with the condition. Determining an appropriate course of treatment for a patient may increase the patient's chances for, for example, survival and/or recovery. Similarly, predicting the occurrence of an event advantageously allows individuals to plan for the event. For example, predicting whether a patient is likely to experience occurrence (e.g., recurrence) of a disease may allow a physician to recommend an appropriate course of treatment for that patient.

Traditionally, physicians rely heavily on their expertise and training to treat, diagnose and predict the occurrence of medical conditions. For example, pathologists use the Gleason scoring system to evaluate the level of advancement and aggression of prostate cancer, in which cancer is graded based on the appearance of prostate tissue under a microscope as perceived by a physician. Higher Gleason scores are given to samples of prostate tissue that are more undifferentiated [1]. Although Gleason grading is widely considered by pathologists to be reliable, it is a subjective scoring system. Particularly, different pathologists viewing the same tissue samples may make conflicting interpretations.

Conventional tools for assisting physicians in medical diagnostics are limited in scope and application. For example, tools for assisting physicians with decisions regarding prostate cancer treatment after a patient has undergone radical prostatectomy are limited to serum-based PSA screening tests and generalized nomograms. One postoperative nomogram, developed by Kattan et al. U.S. Patent No. 6,409,664, is widely used by urologists and allows prediction of the 7-year probability of disease recurrence for patients treated by radical prostatectomy. This nomogram provides information about the likelihood of biochemical failure only (i.e., an increase in PSA level), and does not predict clinical failure (death). Moreover, this nomogram only predicts whether a patient's condition is likely to recur within 7 years, and does not predict when in that interval the patient's condition might recur. Prognostic variables used in this nomogram include pre-treatment serum PSA levels, Gleason score, and microscopic assessment by a pathologist of prostate capsular invasion, surgical margins, seminal vesicle invasion, and lymph node status. Treatment failure is recorded when there is clinical evidence of disease recurrence, a rising serum PSA, or initiation of adjuvant therapy. However, these nomograms have several limitations. Of the most notable limitations is that even the best of these nomograms performs only slightly better than mid-way between a model with perfect discrimination (concordance index = 1.0) and a model with no discriminating ability (concordance index = 0.5). Furthermore, outcome for the approximately 30% of patients who have nomogram predictions in the mid range (7-year progression-free survival, 30-70%) is uncertain as the prediction is no more accurate than a coin toss.

Techniques in computer-implemented image processing and analysis have emerged that provide significantly increased computational power. In many applications, the ability to extract large amounts of quantitative continuous-valued features automatically from a single image has become a reality. A feature X is said to be continuous- valued if, for some A < B, the set of values for the feature includes all numbers x between A and B. Cancer image analysis systems have been developed for images taken from cytological specimens [2] [3]. However, such systems only capture cells and thus do not utilize all of the architectural information observable at the tissue level, let alone combine that information with clinical and molecular information. Cancer image analysis systems have not been provided for analyzing the structure of different pathological elements at the tissue level, which often plays a more important role in diagnosis (e.g., in Gleason analysis) than the appearance of individual cells. Thus, pathologists have resorted to manual techniques for analyzing the shape and size of the prostate gland to determine the pathologic grade of the cancer [4]. The deficiency of conventional cancer image analysis systems is exacerbated by the fact that tissue images are typically more complex than cellular images and require comprehensive domain expert knowledge to be understood.

US 2005/0262031 A1 discloses methods of systems that use clinical information, molecular information and computer-generated morphometric information in a predictive model for predicting the occurrence or recurrence of a medical condition, such as cancer.

In view of the foregoing, it would be desirable to provide systems and methods for treating, diagnosing and predicting the occurrence of medical conditions, responses and other medical phenomena with improved predictive power. It would also be desirable to provide computer-implemented systems and methods that utilize information at the tissue level to treat, diagnose and predict the occurrence of medical conditions.

## SUMMARY OF THE INVENTION

The invention is defined according to the appended claims.

## BRIEF DESCRIPTION OF THE DRAWINGS

For a better understanding of embodiments of the present invention, reference is made to the following description, taken in conjunction with the accompanying drawings, in which like reference characters refer to like parts throughout, and in which:

Figures 1A and 1B are block diagrams of systems that use a predictive model to treat, diagnose or predict the occurrence of a medical condition;
Figure 1C is a block diagram of a system for generating a predictive model;
Figure 2 shows illustrative results for a patient that may be output by a predictive model;
Figure 3 is flowchart of illustrative stages involved in processing tissue images;
Figure 4 is a flowchart of illustrative stages involved in screening for an inhibitor compound of a medical condition;
Figures 5a and 5b show grayscale digital images of healthy and abnormal prostate tissue specimens, respectively, after image segmentation and classification;
Figure 6 shows various clinical, molecular, and computer-generated morphometric features used by a model to predict prostate cancer recurrence;
Figures 7a and 7b show stained tissue specimens demonstrating the presence of two molecular features, particularly Androgen Receptor (AR) and CD34;
Figure 8 is a graph of a Kaplan-Meier curve demonstrating a classification of patients as being at low-risk, intermediate-risk, or high-risk for experiencing prostate cancer recurrence as predicted by a model based on the features of Figure 6;
Figure 9 shows various clinical, molecular, and computer-generated morphometric features used by a model to predict prostate cancer recurrence;
Figure 10 is a graph of a Kaplan-Meier curve demonstrating a classification of patients as being at low-risk, intermediate-risk, or high-risk for experiencing prostate cancer recurrence as predicted by a model based on the features of Figure 9;
Figure 11 shows various clinical, molecular, and computer-generated morphometric features used by a model to predict overall survivability of prostate cancer;
Figure 12 is a graph of a Kaplan-Meier curve demonstrating a classification of patients as being at low-risk, intermediate-risk, or high-risk of death due to any cause as predicted by a model based on the features of Figure 11;
Figure 13 shows various clinical and computer-generated morphometric features used by a model to predict aggressive disease subsequent to a patient having a prostatectomy;
Figures 14 and 15 show various clinical, molecular, and computer-generated morphometric features used by a model to predict prostate cancer recurrence;
Figure 16 shows various clinical and computer-generated tissue image features used by a model to predict clinical failure in a patient subsequent to radical prostatectomy;
Figure 17 shows various clinical and computer-generated tissue image features used by a model to predict prostate cancer recurrence;
Figures 18a-e illustrate multiplex image segmentation for quantifying nuclear AR in prostate tissue;
Figure 19 shows various clinical and computer-generated tissue image features used by another model to predict clinical failure in a patient subsequent to radical prostatectomy; and
Figure 20 is a line graph showing survival curves for the 51 patients in a model for predicting survival of individuals with non-small cell lung cancer (NSCLC) who have been treated with gefitinib, separated into 2 groups by model score above or below the cut-point of 39, where a score of 39.5 or greater predicted a shortened overall survival time.

## DETAILED DESCRIPTION OF THE PREFERRED EMBODIMENTS

The disclosure herein relates to methods and systems that use computer-generated morphometric information alone or in combination with clinical information and/or molecular information in a predictive model for predicting the occurrence of a medical condition. For example, clinical, molecular and computer-generated morphometric information can be used to predict the recurrence of prostate cancer. The teachings provided herein can also be used to predict the occurrence of other medical conditions such as, for example, other types of disease (e.g., epithelial and mixed- neoplasms including breast, colon, lung, bladder, liver, pancreas, renal cell, and soft tissue) and the responsiveness or unresponsiveness of a patient to one or more therapies (e.g., pharmaceutical drugs). These predictions may be used by physicians or other individuals to, for example, select an appropriate course of treatment for a patient and/or to diagnose a medical condition in the patient.

Disclosed herein is an analytical tool including a support vector machine (SVM) and/or a neural network may be provided that determines correlations between clinical, molecular, and computer-generated morphometric features and a medical condition. The correlated features may form a model that can be used to predict the occurrence or recurrence of the condition. For example, an analytical tool may be used to generate a predictive model based on data for a cohort of patients whose outcomes with respect to a medical condition (e.g., time to recurrence of cancer) are at least partially known. The model may then be used to evaluate data for a new patient in order to predict the occurrence of the medical condition for the new patient. In some situations, only a subset of the three data types (e.g., clinical and morphometric data only) may be used by the analytical tool to generate the predictive model.

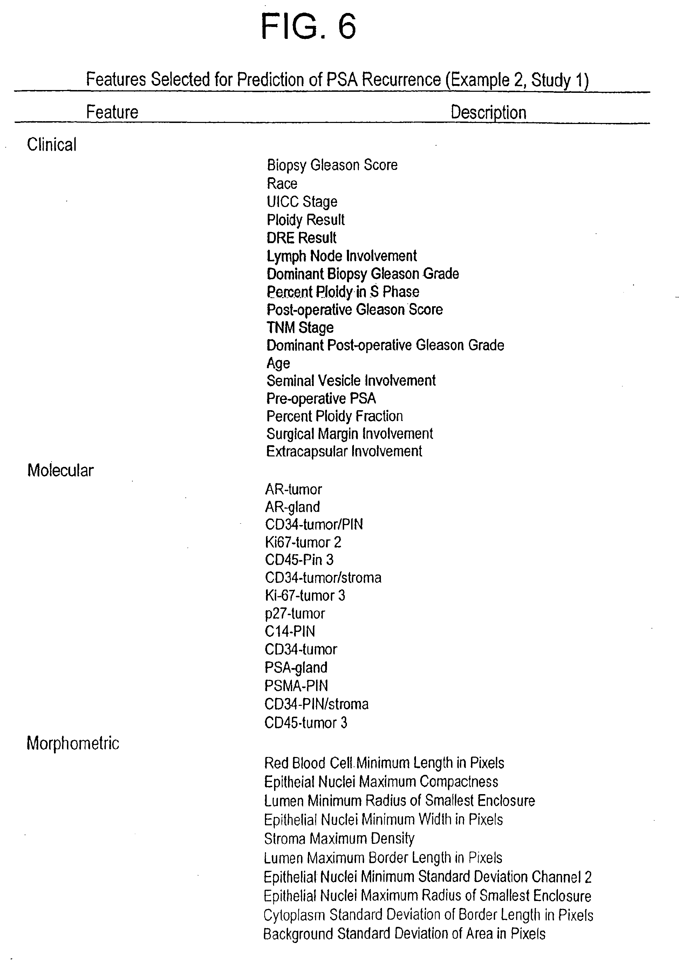

The clinical, molecular, and/or morphometric data used herein may include any clinical, molecular, and/or morphometric data that is relevant to the diagnosis, treatment and/or prediction of a medical condition. Features analyzed for correlations with prostate cancer recurrence and survival in order to generate predictive models are described below in connection with, for example, Tables 1, 2, 4 and/or 6. It will be understood that at least some of these features (e.g., epithelial and mixed-neoplasms) may provide a basis for developing predictive models for other medical conditions (e.g., breast, colon, lung, bladder, liver, pancreas, renal cell, and soft tissue). For example, one or more of the features in Tables 1, 2, 4 and/or 6 may be assessed for patients having some other medical condition and then input to an analytical tool that determines whether the features correlate with the medical condition. Features that increase the ability of the model to predict the occurrence of the medical condition may be included in the final model, whereas features that do not increase (e.g., or decrease) the predictive power of the model may be removed from consideration. Using the features in Tables 1, 2, 4 and/or 6 as a basis for developing a predictive model may focus the resources of physicians, other individuals, and/or automated processing equipment (e.g., a tissue image analysis system) on obtaining patient data that is more likely to be correlated with outcome and therefore useful in the final predictive model. Moreover, the features determined to be correlated with prostate cancer recurrence and survival are shown in Figures 6, 9, and 11. It will be understood that these features may be included directly in final models predictive of prostate cancer recurrence and/or survival, and/or used for developing predictive models for other medical conditions.

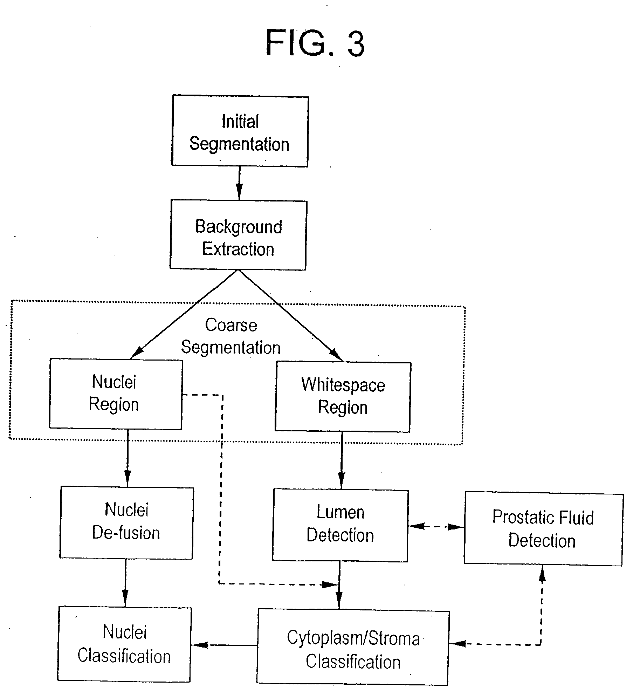

The morphometric data may include computer-generated data indicating various structural and/or spectral properties of, for example, tissue specimens. In one embodiment, the morphometric data may include data for morphometric features of stroma, cytoplasm, epithelial nuclei, stroma nuclei, lumen, red blood cells, tissue artifacts, tissue background, or a combination thereof. In an aspect of the present invention, a tissue image analysis system is provided for obtaining measurements of the morphometric features from a tissue image. Such a system may be the MAGIC™ system which uses the Definiens Cellenger software. Such a system may receive an H&E stained image as input, and may output various measurements of morphometric features for pathological objects in the image. Additional details regarding systems and methods for obtaining morphometric features from an image are described below in connection with Figure 3.

Clinical features may include or be based on data for one or more patients such as age, race, weight, height, medical history, genotype and disease state, where disease state refers to clinical and pathologic staging characteristics and any other clinical features gathered specifically for the disease process at hand. Generally, clinical data is gathered by a physician during the course of examining a patient and/or the tissue or cells of the patient. The clinical data may also include clinical data that may be more specific to a particular medical context. For example, in the context of prostate cancer, the clinical data may include data indicating blood concentration of prostate specific antigen (PSA), the result of a digital rectal exam, Gleason score, and/or other clinical data that may be more specific to prostate cancer. Generally, when any features (i.e., clinical, morphometric and/or molecular) in Tables 1, 2, 4 and/or 6 and/or Figures 6, 9 and/or 11 are applied to medical contexts other than the prostate, features from these Tables and/or Figures that are more specific to the prostate may not be considered. Optionally, features more specific to the medical context in question may be substituted for the prostate-specific features. For example, other histologic disease-specific features/manifestations may include regions of necrosis (e.g., ductal carcinoma in situ for the breast), size, shape and regional pattern/distribution of epithelial cells (e.g., breast, lung), degree of differentiation (e.g., squamous differentiation with non-small cell lung cancer (NSCLC, mucin production as seen with various adenocarcinomas seen in both breast and colon)), morphological/microscopic distribution of the cells (e.g., lining ducts in breast cancer, lining bronchioles in NSCLC), and degree and type of inflammation (e.g., having different characteristics for breast and NSCLC in comparison to prostate).

The molecular features may include or be based on data indicating the presence, absence, relative increase or decrease or relative location of biological molecules including nucleic acids, polypeptides, saccharides, steroids and other small molecules or combinations of the above, for example, glycoroteins and protein-RNA complexes. The locations at which these molecules are measured may include glands, tumors, stroma, and/or other locations, and may depend on the particular medical context. Generally, molecular data is gathered using common molecular biological and biochemical techniques including Southern, Western, and Northern blots, polymerase chain reaction (PCR), immunohistochemistry, and immunofluorescence. Further, in situ hybridization may be used to show both the relative abundance and location of molecular biological features.

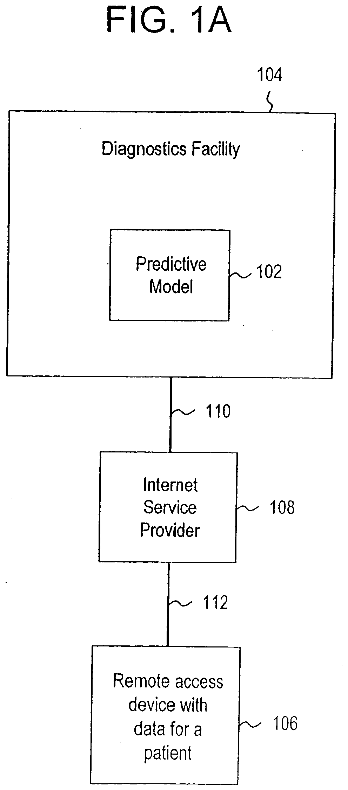

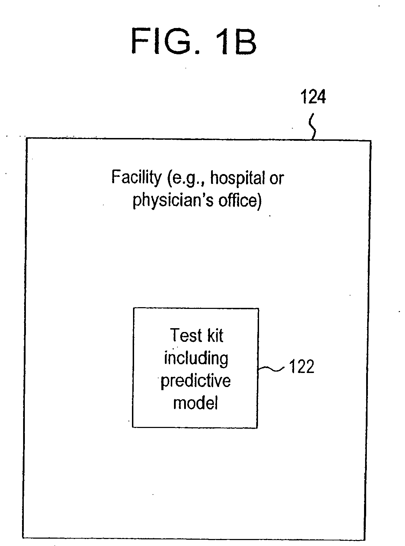

Figures 1A and 1B show illustrative systems that use a predictive model to predict the occurrence of a medical condition in a patient. The arrangement in Figure 1A may be used when, for example, a medical diagnostics lab provides support for a medical decision to a physician or other individual associated with a remote access device. The arrangement in Figure 1B may be used when, for example, a test kit including the predictive model is provided for use in a facility such as a hospital, other medical facility, or other suitable location.

Referring to Figure 1A, predictive model 102 is located in diagnostics facility 104. Predictive model 102 may include any suitable hardware, software, or combination thereof for receiving data for a patient, evaluating the data in order to predict the occurrence (e.g., recurrence) of a medical condition for the patient, and outputting the results of the evaluation. In another embodiment, model 102 may be used to predict the responsiveness of a patient to particular one or more therapies. Diagnostics facility 104 may receive data for a patient from remote access device 106 via Internet service provider (ISP) 108 and communications networks 110 and 112, and may input the data to predictive model 102 for evaluation. Other arrangements for receiving and evaluating data for a patient from a remote location are of course possible (e.g., via another connection such as a telephone line or through the physical mail). The remotely located physician or individual may acquire the data for the patient in any suitable manner and may use remote access device 106 to transmit the data to diagnostics facility 104. In some embodiments, the data for the patient may be at least partially generated by diagnostics facility 104 or another facility. For example, diagnostics facility 104 may receive a digitized version of an H&E stained image from remote access device 106 or other device and may generate morphometric data for the patient based on the image. In another example, actual tissue samples may be received and processed by diagnostics facility 104 in order to generate the morphometric data. In other examples, a third party may receive an image or tissue for a new patient, generate morphometric data based on the image or tissue, and provide the morphometric data to diagnostics facility 104. A suitable image processing tool for generating morphometric data from tissue images and/or samples is described below in connection with Figure 3.

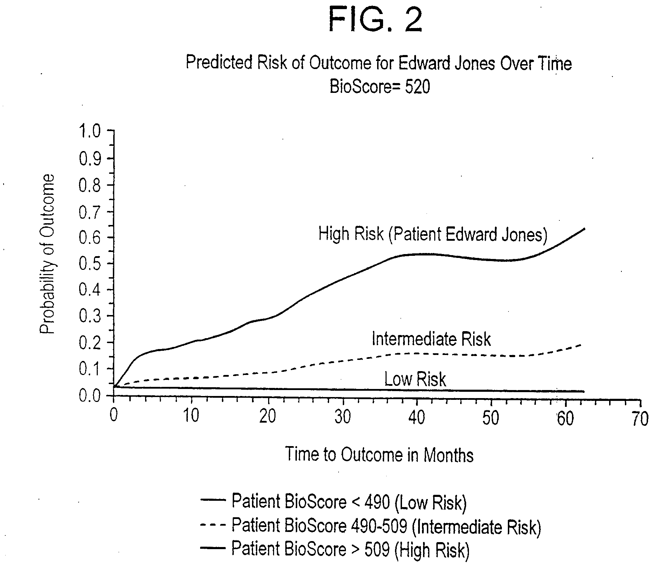

Diagnostics facility 104 may provide the results of the evaluation to a physician or individual associated with remote access device 106 through, for example, a transmission to remote access device 106 via ISP 108 and communications networks 110 and 112 or in another manner such as the physical mail or a telephone call. The results may include a diagnostic "score" (e.g., an indication of the likelihood that the patient will experience one or more outcomes related to the medical condition such as the predicted time to recurrence of the event), information indicating one or more features analyzed by predictive model 102 as being correlated with the medical condition, information indicating the sensitivity and/or specificity of the predictive model, or other suitable diagnostic information or a combination thereof. For example, Figure 2 shows an example of a report for a fictional patient that may be output by the predictive model. As shown, the report maps the patient's probability of outcome (e.g., recurrence of prostate cancer; i.e., y-axis) to time in months (x-axis). In this example, the patient has a score of "520" which places the patient in a high-risk category. Such a report may be used by a physician or other individual to assist in determining a more refined clinical-diagnostic tumor grade, develop an effective means to sub-classify patients and finally generate more accurate (and appropriate) treatment options for the individual patient. The report may also be useful in that it may help the physician or individual to explain the patient's risk to the patient.

Remote access device 106 may be any remote device capable of transmitting and/or receiving data from diagnostics facility 104 such as, for example, a personal computer, a wireless device such as a laptop computer, a cell phone or a personal digital assistant (PDA), or any other suitable remote access device. Multiple remote access devices 106 may be included in the system of Figure 1A (e.g., to allow a plurality of physicians or other individuals at a corresponding plurality of remote locations to communicate data with diagnostics facility 104), although only one remote access device 106 has been included in Figure 1A to avoid over-complicating the drawing. Diagnostics facility 104 may include a server capable of receiving and processing communications to and/or from remote access device 106. Such a server may include a distinct component of computing hardware and/or storage, but may also be a software application or a combination of hardware and software. The server may be implemented using one or more computers.

Each of communications links 110 and 112 may be any suitable wired or wireless communications path or combination of paths such as, for example, a local area network, wide area network, telephone network, cable television network, intranet, or Internet. Some suitable wireless communications networks may be a global system for mobile communications (GSM) network, a time-division multiple access (TDMA) network, a code-division multiple access (CDMA) network, a Bluetooth network, or any other suitable wireless network.

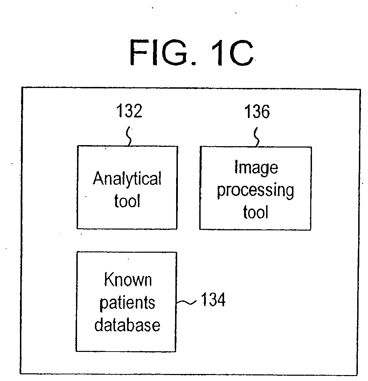

Figure 1B shows a system in which test kit 122 including the predictive model of the present invention is provided for use in facility 124, which may be a hospital, a physician's office, or other suitable location. Test kit 122 may include any suitable hardware, software, or combination thereof (e.g., a personal computer) that is adapted to receive data for a patient (e.g., at least one of clinical, morphometric and molecular data), evaluate the patient's data with a predictive model (e.g., programmed in memory of the test kit), and output the results of the evaluation. For example, test kit 122 may include a computer readable medium encoded with computer executable instructions for performing the functions of the predictive model. The predictive model may be a predetermined model previously generated (e.g., by another system or application such as the system in Figure 1C). In some embodiments, test kit 122 may optionally include an image processing tool capable of generating data corresponding to morphometric features from, for example, a tissue sample or image. A suitable image processing tool is described below in connection with Figure 3. In other embodiments, test kit 122 may receive pre-packaged data for the morphometric features as input from, for example, an input device (e.g., keyboard) or another device or location. Test kit 122 may optionally include an input for receiving, for example, updates to the predictive model. The test kit may also optionally include an output for transmitting data, such as data useful for patient billing and/or tracking of usage, to a main facility or other suitable device or location. The billing data may include, for example, medical insurance information for a patient evaluated by the test kit (e.g., name, insurance provider, and account number). Such information may be useful when, for example, a provider of the test kit charges for the kit on a per-use basis and/or when the provider needs patients' insurance information to submit claims to insurance providers.

Figure 1C shows an illustrative system for generating a predictive model. The system includes analytical tool 132 (e.g., including a support vector machine (SVM) and/or a neural network) and database 134 of patients whose outcomes are at least partially known. Analytical tool 132 may include any suitable hardware, software, or combination thereof for determining correlations between the data from database 134 and a medical condition. The system in Figure 1C may also include image processing tool 136 capable of generating morphometric data based on, for example, a digitized version of an H&E stained tissue image, an actual tissue sample, or both. Tool 136 may generate morphometric data for, for example, the known patients whose data is included in database 134. A suitable image processing tool 136 is described below in connection with Figure 3.

Database 134 may include any suitable patient data such as data for clinical features, morphometric features, molecular features, or a combination thereof. Database 134 may also include data indicating the outcomes of patients such as whether and when the patients have experienced disease recurrence. For example, database 134 may include uncensored data for patients (i.e., data for patients whose outcomes are completely known) such as data for patients who have experienced a recurrence of a medical condition. Database 134 may alternatively or additionally include censored data for patients (i.e., data for patients whose outcomes are not completely known) such as data for patients who have not shown signs of disease recurrence in one or more follow-up visits to a physician. The use of censored data by analytical tool 132 may increase the amount of data available to generate the predictive model and, therefore, may advantageously improve the reliability and predictive power of the model. Examples of support vector machines (SVM) and neural networks (NNci) that can make use of both censored and uncensored data are described below.

In one embodiment, analytical tool 132 may include a support vector machine (SVM). In such an embodiment, tool 132 preferably includes an SVM capable of performing support vector regression on censored data (SVRc). As described in co-pending U.S. Patent Application No. 10/991,240, in SVRc a novel modified loss/penalty function is provided for use within an SVM that may allow the SVM to utilize censored data. Data including clinical, molecular and/or morphometric features of known patients from database 134 may be input to the SVM to determine parameters for a predictive model. The parameters may indicate the relative importance of input features, and may be adjusted in order to maximize the ability of the SVM to predict the outcomes of the known patients. Additional details regarding the use of SVM to determine correlations of features with a medical condition are described in [5] and [6].

The use of SVRc by analytical tool 132 may include obtaining from database 134 multidimensional, non-linear vectors of information indicative of status of patients, where at least one of the vectors lacks an indication of a time of occurrence of an event with respect to a corresponding patient. Analytical tool 132 may then perform regression using the vectors to produce a kernel-based model that provides an output value related to a prediction of time to the event based upon at least some of the information contained in the vectors of information. Analytical tool 132 may use a loss function for each vector containing censored data that is different from a loss function used by tool 132 for vectors comprising uncensored data. A censored data sample may be handled differently because it may provide only "one-sided information." For example, in the case of survival time prediction, a censored data sample typically only indicates that the event has not happened within a given time, and there is no indication of when it will happen after the given time, if at all.

The loss function used by analytical tool 132 for censored data may be as follows:

$$Loss(f(\mathbf{x}), y, s = 1) = \begin{cases} C_s^*(e - \varepsilon_s^*) & e > \varepsilon_s^* \\ 0 & -\varepsilon_s \le e \le \varepsilon_s^* \\ C_s(\varepsilon_s - e) & e < -\varepsilon_s \end{cases},$$

where $e = f(x) - y$; and

$$f(\mathbf{x}) = \mathbf{W}^T \Phi(\mathbf{x}) + b$$

is a linear regression function on a feature space *F*. Here, **W** is a vector in *F*, and $\Phi(\textbf{\textit{x}})$ maps the input **x** to a vector in *F*.

In contrast, the loss function used by tool 132 for uncensored data may be:

$$Loss(f(\mathbf{x}), y, s = 0) = \begin{cases} C_n^*(e - \varepsilon_n^*) & e > \varepsilon_n^* \\ 0 & -\varepsilon_n \le e \le \varepsilon_n^*, \\ C_n(\varepsilon_n - e) & e < -\varepsilon_n \end{cases}$$

where $e = f(\textbf{\textit{x}})-y$

and $\varepsilon_n^* \le \varepsilon_n$ and $C_n^* \ge C_n$.

In the above description, the **W** and *b* are obtained by solving an optimization problem, the general form of which is:

$$\min_{W,b} \quad \frac{1}{2} \, W^T \, W$$

s.t. $y_i - (W^T \phi(x_i) + b) \le \varepsilon$
$(W^T \phi(x_i) + b) - y_i \le \varepsilon$

This equation, however, assumes the convex optimization problem is always feasible, which may not be the case. Furthermore, it is desired to allow for small errors in the regression estimation. It is for these reasons that a loss function is used for SVRc. The loss allows some leeway for the regression estimation. Ideally, the model built will exactly compute all results accurately, which is infeasible. The loss function allows for a range of error from the ideal, with this range being controlled by slack variables $\xi$ and $\xi^*$, and a penalty C. Errors that deviate from the ideal, but are within the range defined by $\xi$ and $\xi^*$, are counted, but their contribution is mitigated by C. The more erroneous the instance, the greater the penalty. The less erroneous (closer to the ideal) the instance is, the less the penalty. This concept of increasing penalty with error results in a slope, and C controls this slope. While various loss functions may be used, for an epsilon-insensitive loss function, the general equation transforms into:

$$\min_{\mathbf{W},b} \quad P = \frac{1}{2} \mathbf{W}^T \mathbf{W} + C \sum_{i=1}^{l} (\xi_i + \xi_i^*)$$

s.t. $y_i - (\mathbf{W}^T \Phi(\mathbf{x}_i) + b) \le \varepsilon + \xi_i$
$(\mathbf{W}^T \Phi(\mathbf{x}_i) + b) - y_i \le \varepsilon + \xi_i^*$
$\xi_i, \xi_i^* \ge 0, i = 1 \cdots l$

For an epsilon-insensitive loss function in accordance with the invention (with different loss functions applied to censored and uncensored data), this equation becomes:

$$\min_{\mathbf{W},b} \quad P_c = \frac{1}{2} \mathbf{W}^T \mathbf{W} + \sum_{i=1}^{l} (C_i \xi_i + C_i^* \xi_i^*)$$

s.t. $y_t - (\mathbf{W}^T \Phi(\mathbf{x}_i) + b) \le \varepsilon_i + \xi_i$
$$(\mathbf{W}^T \Phi(\mathbf{x}_i) + b) - y_i \le \varepsilon_i^* + \xi_i^*$$
$\xi_i^{(*)} \ge 0, i = 1 \cdots l$
where $C_i^{(*)} = s_i C_s^{(*)} + (1 - s_i) C_n^{(*)}$

$$\varepsilon_i^{(*)} = s_i \varepsilon_s^{(*)} + (1 - s_i)\varepsilon_n^{(*)}$$

The optimization criterion penalizes data points whose $y$-values differ from $f(\mathbf{x})$ by more than $\varepsilon$. The slack variables, $\xi$ and $\xi^*$, correspond to the size of this excess deviation for positive and negative deviations respectively. This penalty mechanism has two components, one for uncensored data (i.e., not right-censored) and one for censored data. Both components are, here, represented in the form of loss functions that are referred to as $\varepsilon$-insensitive loss functions.

Additional details regarding systems and methods for performing support vector regression on censored data (SVRc) are described in above-incorporated U.S. Patent Application No. 10/991,240, filed November 17, 2004, and U.S. Provisional Patent Application No. 60/520,939, filed November 18, 2003.

In another embodiment, analytical tool 132 may include a neural network. In such an embodiment, tool 132 preferably includes a neural network that is capable of utilizing censored data. Additionally, the neural network preferably uses an objective function substantially in accordance with an approximation (e.g., derivative) of the concordance index (CI) to train an associated model (NNci). Though the CI has long been used as a performance indicator for survival analysis [7], the use of the CI to train a neural network has not been proposed previously. The difficulty of using the CI as a training objective function in the past is that the CI is non-differentiable and cannot be optimized by gradient-based methods. As described in co-pending U.S. Patent Application No. 11/067,066, filed February 25, 2005 (now U.S. Patent No. 7,321,881), this obstacle may be overcome by using an approximation of the CI as the objective function.

For example, when analytical tool 132 includes a neural network that is used to predict prostate cancer recurrence, the neural network may process input data for a cohort of patients whose outcomes with respect to prostate cancer recurrence are at least partially known in order to produce an output. The particular features selected for input to the neural network may be selected through the use of the above-described SVRc (e.g., implemented with a support vector machine of analytical tool 132) or using another suitable feature selection process. An error module of tool 132 may determine an error between the output and a desired output corresponding to the input data (e.g., the difference between a predicted outcome and the known outcome for a patient). Analytical tool 132 may then use an objective function substantially in accordance with an approximation of the CI to rate the performance of the neural network. Analytical tool 132 may adapt the weighted connections (e.g., relative importance of features) of the neural network based upon the results of the objective function. Additional details regarding adapting the weighed connections of a neural network in order to adjust the correlations of features with a predicted outcome are described in [8] and [9].

The concordance index may be expressed in the form:

$$CI = \frac{\sum_{(i,j)\in\Omega} I(\hat{t}_i, \hat{t}_j)}{|\Omega|}$$

where

$$I(\hat{t}_i, \hat{t}_j) = \begin{cases} 1 : \hat{t}_i > \hat{t}_j \\ 0 : otherwise \end{cases},$$

and may be based on pair-wise comparisons between the prognostic estimates $\hat{t}_i$ and $\hat{t}_j$ for patients $i$ and $j$, respectively. In this example, $\Omega$ consists of all the pairs of patients $\{i,j\}$ who meet the following conditions:

- both patients $i$ and $j$ experienced recurrence, and the recurrence time $t_i$ of patient $i$ is shorter than patient $j$'s recurrence time $t_j$; or
- only patient $i$ experienced recurrence and $t_i$ is shorter than patient $j$'s follow-up visit time $t_j$.

The numerator of the CI represents the number of times that the patient predicted to recur earlier by the neural network actually does recur earlier. The denominator is the total number of pairs of patients who meet the predetermined conditions.

Generally, when the CI is increased, preferably maximized, the model is more accurate. Thus, by preferably substantially maximizing the CI, or an approximation of the CI, the performance of a model is improved. An embodiment of the present invention provides an approximation of the CI as follows:

$$C = \frac{\sum_{(i,j)\in\Omega} R(\hat{t}_i, \hat{t}_j)}{|\Omega|}$$

where

$$R(\hat{t}_i, \hat{t}_j) = \begin{cases} (-(\hat{t}_i - \hat{t}_j - \gamma))^n : \hat{t}_i - \hat{t}_j < \gamma \\ 0 : otherwise \end{cases},$$

and where $0 < \gamma \leq 1$ and $n > 1$. $R(\hat{t}_i, \hat{t}_j)$ can be regarded as an approximation to $I(-\hat{t}_i, -\hat{t}_j)$.

Another approximation of the CI provided by the present invention which has been shown empirically to achieve improved results is the following:

$$C_\omega = \frac{\sum_{(i,j)\in\Omega} -(\hat{t}_i - \hat{t}_j) \bullet R(\hat{t}_i, \hat{t}_j)}{D},$$

where

$$D = \sum_{(i,j)\in\Omega} -(\hat{t}_i - \hat{t}_j)$$

is a normalization factor. Here each $R(\hat{t}_i, \hat{t}_j)$ is weighted by the difference between $\hat{t}_i$ and $\hat{t}_j$. The process of minimizing the $C_\omega$ (or C) seeks to move each pair of samples in $\Omega$ to satisfy $\hat{t}_i - \hat{t}_j > \gamma$ and thus to make $I(\hat{t}_i, \hat{t}_j) = 1$.

When the difference between the outputs of a pair in $\Omega$ is larger than the margin $\gamma$, this pair of samples will stop contributing to the objective function. This mechanism effectively overcomes over-fitting of the data during training of the model and makes the optimization preferably focus on only moving more pairs of samples in $\Omega$ to satisfy $\hat{t}_i - \hat{t}_j > \gamma$. The influence of the training samples is adaptively adjusted according to the pair-wise comparisons during training. Note that the positive margin $\gamma$ in $R$ is preferable for improved generalization performance. In other words, the parameters of the neural network are adjusted during training by calculating the CI after all the patient data has been entered. The neural network then adjusts the parameters with the goal of minimizing the objective function and thus maximizing the CI. As used above, over-fitting generally refers to the complexity of the neural network. Specifically, if the network is too complex, the network will react to "noisy" data. Overfitting is risky in that it can easily lead to predictions that are far beyond the range of the training data.

Figure 3 is a flowchart of illustrative functions of a suitable image processing tool. The functions in Figure 3 relate primarily to the segmentation of tissue images in order to classify pathological objects in the images (e.g., classifying objects as cytoplasm, lumen, nuclei, stroma, background, artifacts, and red blood cells). In one example, the image processing tool may include a light microscope that captures tissue images at 20X magnification using a SPOT Insight QE Color Digital Camera (KAI2000) and produces images with 1600 x 1200 pixels. The images may be stored as images with 24 bits per pixel in Tiff format. Such equipment is only illustrative and any other suitable image capturing equipment may be used without departing from the scope of the present invention. The image processing tool may also include any suitable hardware, software, or combination thereof for segmenting and classifying objects in the captured images, and then measuring morphometric features of the objects. In one embodiment, the image processing tool may include the commercially-available Definiens Cellenger Developer Studio (v. 4.0) adapted to perform the segmenting and classifying of, for example, the various pathological objects described above and to measure various morphometric features of these objects. Additional details regarding the Definiens Cellenger product are described in [10]. The image processing tool may measure various morphometric features of the objects including spectral-based characteristics (red, green, blue (RGB) channel characteristics, such as mean values, standard deviations, etc.), position, size, perimeter, shape (asymmetry, compactness, elliptic fit, etc.) and spatial and intensity relationships to neighboring objects (contrast). The image processing tool may measure these features for every instance of every identified pathological object in the image and may output these features for, for example, evaluation by predictive model 102 (Figure 1A), test kit 122 (Figure 1B), or analytical tool 132 (Figure 1C). Optionally, the image processing tool may also output an overall statistical summary for the image for each of the measured features. Additional details regarding measuring morphometric features of the classified pathological objects are described below in connection with Tables 1 and 2. The following is a description of the functions shown in Figure 3 of the image processing tool.

*Initial Segmentation.* In a first stage, the image processing tool may segment an image (e.g., an H&E stained tissue microarray (TMA) image or an H&E of a whole tissue section) into small groups of contiguous pixels known as objects. These objects may be obtained by a region-growing method which finds contiguous regions based on color similarity and shape regularity. The size of the objects can be varied by adjusting a few parameters [11]. In this system, an object rather than a pixel is typically the smallest unit of processing. Thus, all morphometric feature calculations and operations may be performed with respect to objects. For example, when a threshold is applied to the image, the feature values of the object are subject to the threshold. As a result, all the pixels within an object are assigned to the same class. In one embodiment, the size of objects may be controlled to be 10-20 pixels at the finest level. Based on this level, subsequent higher and coarser levels are built by forming larger objects from the smaller ones in the lower level.

*Background Extraction.* Subsequent to initial segmentation, the image processing tool may segment the image tissue core from the background (transparent region of the slide) using intensity threshold and convex hull. The intensity threshold is an intensity value that separates image pixels in two classes: "tissue core" and "background". Any pixel with an intensity value greater than or equal the threshold is classified as a "tissue core" pixel, otherwise the pixel is classified as a "background" pixel. The convex hull of a geometric object is the smallest convex set (polygon) containing that object. A set S is convex if, whenever two points P and Q are inside S, then the whole line segment PQ is also in S.

*Coarse Segmentation.* In a next stage, the image processing tool may re-segment the foreground (e.g., TMA core) into rough regions corresponding to nuclei and white spaces. For example, the main characterizing feature of nuclei in H&E stained images is that they are stained blue compared to the rest of the pathological objects. Therefore, the difference in the red and blue channels ($R$-$B$) intensity values may be used as a distinguishing feature. Particularly, for every image object obtained in the initial segmentation step, the difference between average red and blue pixel intensity values may be determined. The length/width ratio may also be used to determine whether an object should be classified as nuclei area. For example, objects which fall below a ($R$-$B$) feature threshold and below a length/width threshold may be classified as nuclei area. Similarly, a green channel threshold can be used to classify objects in the tissue core as white spaces. Tissue stroma is dominated by the color red. The intensity difference $d$, "red ratio" $r = R/(R + G + B)$ and the red channel standard deviation $\sigma_R$ of image objects may be used to classify stroma objects.

*White Space Classification.* In the stage of coarse segmentation, the white space regions may correspond to both lumen (pathological object) and artifacts (broken tissue areas) in the image. The smaller white space objects (area less than 100 pixels) are usually artifacts. Thus, the image processing tool may apply an area filter to classify them as artifacts.

*Nuclei De-fusion and Classification.* In the stage of coarse segmentation, the nuclei area is often obtained as contiguous fused regions that encompass several real nuclei. Moreover, the nuclei region might also include surrounding misclassified cytoplasm. Thus, these fused nuclei areas may need to be de-fused in order to obtain individual nuclei.

The image processing tool may use two different approaches to de-fuse the nuclei. The first approach may be based on a region growing method that fuses the image objects constituting nuclei area under shape constraints (roundness). This approach has been determined to work well when the fusion is not severe.

In the case of severe fusion, the image processing tool may use a different approach based on supervised learning. This approach involves manual labeling of the nuclei areas by an expert (pathologist). The features of image objects belonging to the labeled nuclei may be used to design statistical classifiers.

In one embodiment, in order to reduce the number of feature space dimensions, feature selection may be performed on the training set using two different classifiers: the Bayesian classifier and the k nearest neighbor classifier [12]. The leave-one-out method [13] may be used for cross-validation, and the sequential forward search method may be used to choose the best features. Finally, two Bayesian classifiers may be designed with number of features equal to 1 and 5, respectively. The class-conditional distributions may be assumed to be Gaussian with diagonal covariance matrices.

In some embodiments, the input image may include different kinds of nuclei: epithelial nuclei, fibroblasts, basal nuclei, endothelial nuclei, apoptotic nuclei and red blood cells. Since the number of epithelial nuclei is typically regarded as an important feature in grading the extent of the tumor, it may be important to distinguish the epithelial nuclei from the others. The image processing tool may accomplish this by classifying the detected nuclei into two classes: epithelial nuclei and "the rest" based on shape (eccentricity) and size (area) features.

As described above, the image processing tool may measure various morphometric features subsequent to the segmenting and classifying of objects in the image by the tool. These morphometric features may be indicative of one or more properties and/or statistics. The object properties may include both spectral properties (e.g., color channel mean values, standard deviations and brightness) and structural/shape properties (e.g., area, length, width, compactness, density). The statistics may include minimum, maximum, mean and standard deviation and may be computed for each property of an image object. Tables 1 and 2 (appended hereto) show various examples of morphometric features that may be measured in accordance with the present invention. The morphometric features in these tables are named using a convention that indicates the various properties and/or statistics measured by these features. The particular naming convention shown in Tables 1 and 2 is adapted from the commercially-available Definiens software product described above and, therefore, will be understood by one of ordinary skill in the art.

It will be understood that the computer-generated morphometric features shown in Tables 1 and 2 are only illustrative and that any computer-generated morphometric features may be utilized without departing from the scope of the present invention. For example, Tables 1 and 2 include different sets of morphometric features. The reduced and modified set of features in Table 2 (i.e., reduced and modified in comparison to the features of Table 1) resulted from additional experimentation in the field of prostate cancer recurrence and survival from the time that the study involving Table 1 was performed. Particularly, the additional experimentation provided additional insight regarding the types of features which may be more likely to correlate with outcome. The inventors expect that continued experimentation and/or the use of other suitable hardware, software, or combination thereof will yield various other sets of computer-generated features (e.g., a subset of the features in Table 1 (see Tables 10 and 11) or a subset of the features in Table 2) that may correlate with these and other medical conditions.

Referring to Tables 1 and 2, the feature "Lumen.StdDevAreaPxl", "Lumen" indicates a type of image object, "StdDev" indicates a statistic (standard deviation) to be computed using all instances of the identified Lumen, and "AreaPxl" indicates a feature of an object instance (area as a number of pixels) to be evaluated by the statistic. An image processing tool may measure morphometric features for all the objects previously segmented and classified in the image. For example, the image processing tool may measure morphometric features for objects including "Background," "Cytoplasm," "Epithelial nuclei," "Lumen," "Stroma," "Stroma nuclei" and "Red blood cells." "Background" includes portions of the digital image that are not occupied by tissue. "Cytoplasm" refers to the cytoplasm of a cell, which may be an amorphous area (e.g., pink area that surrounds an epithelial nucleus in an image of, for example, H&E stained tissue). "Epithelial nuclei" refers to the nucleus present within epithelial cells/luminal and basal cells of the glandular unit, which appear as "round" objects surrounded by cytoplasm. "Lumen" refers to central glandular space where secretions are deposited by epithelial cells, which appear as enclosed white areas surrounded by epithelial cells. Occasionally, the lumen can be filled by prostatic fluid (which typically appears pink in H&E stained tissue) or other "debris" (e.g., macrophages, dead cells, etc.). Together the lumen and the epithelial cytoplasm and nuclei form a gland unit. "Stroma" refers to a form of connective tissue with different density that maintains the architecture of the prostatic tissue. Stroma tissue is present between the gland units, and appears as red to pink in H&E stained tissue. "Stroma nuclei" are elongated cells with no or minimal amounts of cytoplasm (fibroblasts). This category may also include endothelial cells and inflammatory cells, and epithelial nuclei may also be found scattered within the stroma if cancer is present. "Red blood cells" are small red round objects usually located within the vessels (arteries or veins), but can also be found dispersed throughout tissue.

"C2EN" in the below tables is a relative ratio of nucleus area to the cytoplasm. The more anaplastic/malignant the epithelial cell is, the more area is occupied by the nucleus and the greater the ratio. "EN2SN" is the percent or relative amount of epithelial to stroma cells present in the digital tissue image. "L2Core" is the number or area of lumen present within the tissue. The higher the Gleason grade, the more aggressive cancer is and therefore the less amount of lumen is present. Generally, this is because epithelial cells replicate in an uncontrolled way when cancer occurs, which causes lumen to become filled with the epithelial cells.

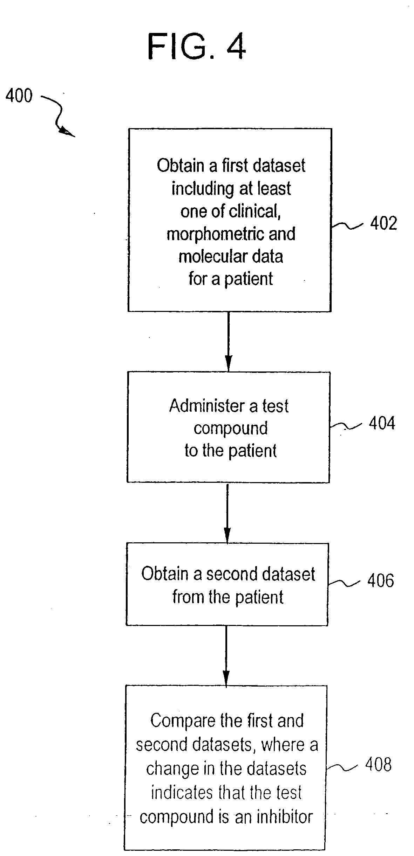

In an aspect of the present invention, systems and methods are provided for screening for an inhibitor compound of a medical condition (e.g., disease). Figure 4 is a flowchart of illustrative stages involved in screening for an inhibitor compound in accordance with an embodiment of the present invention. At stage 402, a first dataset for a patient may be obtained that includes one or more of clinical data, morphometric data and molecular data. A test compound may be administered to the patient at stage 404. Following stage 404, a second dataset may be obtained from the patient at stage 406. The second dataset may or may not include the same data types (i.e., features) included in the first dataset. At stage 408, the second dataset may be compared to the first dataset, where a change in the second dataset following administration of the test compound indicates that the test compound is an inhibitor compound. Stage 408 of comparing the datasets may include, for example, comparing an output generated by a predictive model of the present invention responsive to an input of the first dataset with an output generated by the predictive model responsive to an input of the second dataset. For example, the inhibitor compound may be a given drug and the present invention may determine whether the drug is effective as a medical treatment for a medical condition.

Various illustrative applications of embodiments of the present invention to the prediction of medical conditions will now be described. In a first example, an embodiment of the present invention used clinical and morphometric data to predict the recurrence of prostate cancer. In a second example, an embodiment of the present invention used clinical, morphometric, and molecular data to predict the recurrence of prostate cancer and overall survivability. In a third example, an embodiment of the present invention was used to predict the occurrence of aggressive disease subsequent to a patient prostatectomy. In a fourth example, an embodiment of the present invention was used to predict liver toxicology. In fifth, sixth, and eighth examples, embodiments of the present invention were used to predict prostate cancer recurrence. In seventh and ninth examples, embodiments of the present invention were used to predict clinical failure post prostatectomy. In a tenth example, an embodiment of the present invention was used to predict survival of individuals with non-small cell lung cancer (NSCLC) who have been treated with gefitinib.

**Prostate Cancer Overview**

Prostate cancer is a leading cause of death among men in the United States with an anticipated 230,000 newly diagnosed cases and nearly 30,000 deaths in 2004. The expanded use of serum based screening with PSA has offered physicians the ability to detect prostate cancer at an earlier stage (i.e. T1a-c, T2), either localized to the prostate or regionally spread while only a small percentage are detected at the metastatic stage. The reported benefits of early detection and diagnosis have placed enormous pressure on both the patient and the urologist in selecting the course of treatment. The need for accurate prognosis is critical when selecting initial therapeutic intervention, as the majority of tumors are indolent and require minimal intervention (i.e. 'watchful waiting') while others are more aggressive and early intervention (i.e. radiotherapy / hormonal / adjuvant systemic therapy / clinical trial placement) is recommended. Furthermore, in a randomized trial comparing watchful waiting with radical prostatectomy, only a modest benefit was derived from surgery (6.6% reduction in mortality after prostatectomy) suggesting that better patient stratification measures are needed in order to guide individualized patient care [14].

The natural history of PCa re-emphasizes the challenges facing the patient at the time of their diagnosis [15]. Even though early stage prostate cancer is curable with local therapy, approximately 25 - 40% of men will develop a PSA / biochemical recurrence (BCR). To complicate matters even further, a man with prostate cancer who has had a recurrence can still develop a metastasis some 8 years post PSA / BCR (mean 8 years; median 5 years post BCR), suggesting that identifying this group of patients early in their treatment regimen (both in predicting their time to BCR as well as their propensity to develop metastases) is paramount to their overall survival. Unfortunately, the existing predictive models are limited in their accuracy and are not individualized for the specific patient with respect to their tumor pathology. Although a variety of genetic, environmental and life-style changes have been implicated in the pathogenesis of PCa, at present there is no single biochemical pathway, gene mutation or clinical biomarker which can predict a given patients outcome. Twenty-one years after radical prostatectomy became popular again and 15 years after the widespread use of PSA, urologists still cannot tell patients which treatment for localized disease results in the best clinical disease-free or overall survival.

Prognostic nomograms based only on clinical feature data do in fact provide useful predictions of clinical states and outcomes, but need improvement in both accuracy and universality [16]. Embodiments of the present invention provide a 'Systems Pathology' approach to successfully improve upon the accuracy of a predictive model for PSA / BCR post prostatectomy. This represents an 'individualized' view of the patients own tumor sample, including quantitative assessment of cellular and microanatomic morphometric characteristics, clinical profiles and molecular markers to create a highly accurate and integrative model of prediction. By utilizing domain expertise, highly accurate models for predicting PSA recurrence have been developed. These efforts have validated the utility of systems pathology in generating predictive and prognostic models. Furthermore, the analysis demonstrates that a limited set of clinical variables, molecular biomarkers, and tissue morphometric features can be derived and included in a predictive test used by urologists/pathologists to construct optimal patient treatment plans based on a designated clinical outcome. The selected molecular features which were associated with PSA recurrence suggest convergent roles for mechanisms of growth factor signaling (through the androgen receptor (hereinafter "AR", described below) and cellular coupled vascularization (through CD34). CD34 is a transmembrane glycoprotein which is present on endothelial cells which line vessels in the human body. Further studies are underway to better understand these observations and the potential impact on predicting prostate cancer progression. Also of note were the selected image segmentation and morphometric characteristics which represent in part a highly accurate, non-subjective and quantitative Gleason Score in addition to several novel tissue descriptors which were important in model development and accuracy. The defined morphometric features relating to the Gleason Scoring System include in part the overall appearance of the glandular structures, shape and size (cytoplasmic composition) of the epithelial cells, epithelial cell nuclei and the demonstration of single epithelial cells admixed in the stroma.

The androgen receptor protein (AR) receives naturally occurring androgenic hormones (testosterone and its 5 .alpha.-reduced metabolite, dihydrotestosterone) after these hormones are synthesized by the Leydig cells of the male testes. Particularly, after synthesizing, these hormones circulate throughout the body and bind to the AR. Androgens, acting through the receptor AR, stimulate development of the male genitalia and accessory sex glands in the fetus, virilization and growth in the pubertal male, and maintenance of male virility and reproductive function in the adult. The androgen receptor, together with other steroid hormone receptors, constitute a family of trans-acting transcriptional regulatory proteins that control gene transcription through interactions with specific gene sequences.

Studies on AR with respect to prostate cancer have suggested that a positive correlation may exist between the presence of androgen receptors in cancer cells and their dependence on androgenic hormone stimulation for growth. For example, Sovak et al. U.S. Patent No. 6,472,415 proposes that growth of prostate cancer in early stages is androgen driven and can, at least temporarily, be stopped by androgen deprivation. French et al. U.S. Patent No. 6,821,767 proposes various ways for measuring AR that may allow for the use of androgen receptor assays in the diagnostic evaluation of prostate cancer by physicians. However, these studies have not proposed using measurements of AR in conjunction with automated models that predict the occurrence of prostate cancer, as disclosed herein.

**Example 1: Prediction of Prostate Cancer Recurrence Clinical and Morphometric Data**

A number of raw morphometric features initially as large as five hundred was extracted from each prostate tissue image using the MAGIC tissue image analysis system which is based on Definiens Cellenger software. The full set of raw features was chosen agnostically to avoid disregarding potentially useful features. However, all of these morphometric features were not likely to be equally informative, and a prediction model built based on the full feature set would be likely to have poor predictive performance due to the "curse of dimensionality" [13]. So a dimensionality reduction procedure was applied, and a set of eight morphometric features was finally selected.

A study was conducted based on a subset of 153 patients from a cohort of prostate cancer patients who underwent radical prostatectomy. Measurable prostate specific antigen (PSA) after the operation was used to define prostate cancer recurrence (also referred to as a biochemical recurrence (BCR)). Patients were followed post-operatively. Their recurrence status at their last visit, as well as their follow-up time, was recorded, which generated a set of right-censored data. Gleason scores were measured both pre-operatively from the biopsy specimen and post-operatively using the excised prostate gland. The four specific clinical measures, or features, considered in this study were (1) the biopsy Gleason grade, (2) the biopsy Gleason score, (3) the post-operative Gleason grade, and (4) the post-operative Gleason score.

The morphometric features were analyzed separately from the clinically derived Gleason score feature to predict both the probability and the time to PSA/BCR recurrence. The image and Gleason score (features) were then combined to establish a recurrence and time to recurrence time prediction. Improved prediction accuracy achieved by this joint set of features indicated that the image features indeed provided additional information and thus enhanced the recurrence prediction rate and the overall prediction model.

Because this cohort of patients had right-censored outcome data, survival analysis models had to be built for the prediction of recurrence. In order to avoid the potential bias on different types of data, two survival analysis methods were used: 1) a Cox regression model [17]; and 2) SVRc which is described above and as applied to a support vector machine. The concordance index estimated using 5-fold cross validation was used to measure the models' predictive accuracy [13] [18].

Both methods were applied to three data sets: (1) the Gleason score clinical features alone; (2) the selected morphometric features alone; and (3) the combination of the morphometric features and the Gleason score clinical features. The experimental results are listed in Table 3.

The clinical features selected in this example were BXGGTOT, BXGG1, GGTOT, and GG1 and the morphometric features selected related to epithelial nuclei (Epithelial.Nuclei.MaxCompactness), background (Background.StdDevAreaPxl), and lumen (Lumen.MaxBorderLengthPxl, Lumen.MinRadiusofsmallestenclosinge, Lumen. StdDevBorderLengthPxl, Lumen.SumBorderlengthPxl, Lumen.StdDevAreaPxl, and Lumen.MinCompactness). More particularly, in this example, morphometric features related to the area, border length, and shape (compactness) of the lumen were determined to correlate with disease progression. The smaller and more compact the lumen, the more advanced the cancer was likely to be. Indeed, with more aggressive cancer (Gleason grade 4 and 5), it can be expected that lumen will almost or completely disappear from the tissue. It was also determined that the morphometric feature of compactness of epithelial nuclei correlated with cancer progression, where compactness was calculated by the Definiens Cellenger software as the ratio of the length and width product of the epithelial nuclei to the epithelial nuclei area. This may be because epithelial nuclei invasion into stroma increases as cancer progresses (i.e., tissue with advanced cancer typically includes an abundance of epithelial nuclei). The background-based morphometric feature that was determined to correlate with outcome in this example measured the actual size of the tissue core used in the analysis.

**Table 3 - Comparison of Prediction Accuracy**

|         | Gleason | Image  | Gleason + Image |
|---------|---------|--------|-----------------|
| **Cox**  | 0.6952  | 0.6373 | 0.7261          |
| **SVRc** | 0.6907  | 0.7269 | 0.7871          |

According to Table 3, the predictive performance of the morphometric features is comparable with that of the Gleason scores, and the combination of the morphometric features and the Gleason scores achieves a higher predictive rate, which confirms that the morphometric features extracted by the tissue image analysis system indeed provide extra information beyond the Gleason scores. Therefore, the use of the morphometric measurements can enhance overall recurrence prediction.

**Example 2: Prediction of Prostate Cancer Recurrence and Overall Survival Clinical, Morphometric and Molecular Data**

Two studies were conducted which successfully predicted prostate specific antigen (PSA) recurrence with 88% and 87% predictive accuracies, respectively. By combining clinical, molecular, and morphometric features with machine learning, a robust platform was created which has broad applications in patient diagnosis, treatment management and prognostication. A third study was conducted to predict overall survival of prostate cancer patients, where the outcome of interest was death due to any cause.

A cohort of 539 patients who underwent radical prostatectomy was studied incorporating high-density tissue microarrays (TMAs) constructed from prostatectomy specimens. Morphometric studies were performed using hematoxylin and eosin (H&E) stained tissue sections and molecular biological determinants were assessed with immunohistochemistry (IHC). A predictive model for both PSA recurrence and overall survival was derived from a selected set of features through supervised multivariate learning. Patients with complete non-missing data in each domain were evaluated with a support vector machine for regression developed to handle censored data (SVRc). Predictive performance of the model was estimated using the concordance index (CI) with generated scores used to define risk groups.

Using a cohort of 132 patients, 41 features (including 17 clinical, 14 molecular, and 10 morphometric) were selected which predicted PSA recurrence with 88% accuracy. In a cohort of 268 patients, 10 features (3 clinical, 1 molecular, and 6 morphometric) were found to be predictive of PSA recurrence with 87% accuracy; additionally, 14 features (2 clinical, 1 molecular, and 11 morphometric) were found to be predictive of overall survival with 80% accuracy. Using the log-rank test, significant differences in tumor recurrence and death were observed between risk groups (p<0.0001).

The present study reveals an incremental trend of improved prostate cancer recurrence prediction through the use of a new systems approach combining clinical variables, molecular markers, and tissue histology, analyzed by machine learning.

**Patient Clinical Features.** A cohort of 539 patients who underwent radical prostatectomy was studied. Seventeen clinical features (shown below in Table 4) were retrospectively collected using de-identified patient information, which included patient age, preoperative PSA, and Gleason Grade.

**Table 4. Clinical Features Collected**

| Feature | Description |
| --- | --- |
| age | Age (in years) |
| race | Race |
| prepsa | Prostate specific antigen (ng/dl) |
| tnm | TNM clinical stage |
| uicc | UICC clinical stage |
| dre | Palpable on digital rectal exam |
| ln | Lymph node status |
| svi | Invasion of the seminal vesicles |
| margins | +/- surgical margins |
| ece | Tumor located outside capsule |
| bxggl | Dominant biopsy Gleason Grade |
| bxggtot | Biopsy Gleason Score |
| ggl | Dominant post-operative Gleason Grade |
| ggtot | Post-operative Gleason Score |
| prsltcd | Diploid, Tetraploid, Aneuploid |
| pp_sphas | Percent of cells in ploidy in S phase |
| pp_frac | Ploidy proliferation fraction |

Tissue microarrays (TMAs) were constructed from selected blocks of the prostatectomy specimens. Tissue cores with a diameter of 0.6 mm from each specimen were randomly arrayed in triplicate for each of the recipient paraffin blocks (Beecher Instruments, Silver Spring, MD). Sections (5 $\mu$m) of these TMA blocks were placed on charged poly-lysine-coated slides, and used for morphometric and immunohistochemical (IHC) analyses (see below).

Missing values for clinical features were imputed with flexible additive regression models containing all of the features to estimate the value of the missing feature without reference to outcome, and only those patients with complete clinical (after imputation), morphometric, and molecular data, as well as non-missing outcome information, were further studied. The effective sample size for Study 1 (proof of concept) consisted of 132 patients. The primary classification of interest was whether a patient recurred or not after surgery for prostate cancer. Patients who had two observed consecutive elevations in PSA > 0.2 ng/mL were considered to have recurrent prostate cancer. If a patient did not recur as of his last visit, or the patient outcome was unknown as of his most recent visit (*i.e.* due to loss-to-follow-up), then the patient's outcome was considered censored. Time to recurrence was defined as the time (in months) from radical prostatectomy until PSA (biochemical) recurrence.

Study 2 was performed using 268 patients from the original 539 patient cohort including 129 of the 132 patients from Study 1. Instead of utilizing H&E images derived from TMA cores, whole sections from radical prostatectomies were analyzed. Study 3 examined the same 268-patient cohort but was used to predict overall survival, where the outcome of interest was death due to any cause.

**Image Analysis and Morphometry Studies.** Representative areas of the original tumor tissue retrieved from each patient, either from a tissue core or whole section, were digitized and analyzed using the H&E stained slides. Images were captured with a light microscope at 20X magnification using a SPOT Insight QE Color Digital Camera (KAI2000). Only areas containing greater than 80% tumor were selected for optimal image segmentation and quantitative analysis.

**Molecular Analysis.** A panel of 12 biomarkers including Cytokeratin 18 (luminal cells), Cytokeratin 14 (basal cells), CD45 (lymphocytes), CD34 (endothelial cells), CD68 (macrophages), Ki67 (proliferation), PSA (hK-3, kallikrein), PSMA (growth receptor), Cyclin D1 (cell cycle), p27 (cell cycle), Androgen Receptor (endocrine) and Her-2/neu (signaling) were applied across all 7 TMA blocks with standard chromogenic immunohistochemistry. Antigen retrieval was performed with a 0.01M citrate buffer (pH 6) for 30 min in a pressure cooker for all antibodies. Primary antibodies (shown in Table 5) were diluted in Tris-buffered saline with 0.1% Tween and applied for 16 h at 4 °C followed by biotinylated secondary antibodies (Vector) at 1:1000 dilution for 1 h.

**Table 5. List of Antibodies**

| Biomarker | Clone |
| --- | --- |
| Ki-67 | Clone ki-67 (DAKO) |
| Cytokeratin 18 | Clone DC-10 (Novocastra) |
| CD45 | Clone X16/99 |
| CD68 | Clone 514H2 (Novocastra UK) |
| CD34 | Clone QBEnd 101 (DAKO) |
| AR | Clone AR27 (Novocastra) |
| Cytokeratin 14 | Clone LL002 (Novocastra) |
| Cyclin D1 | Clone P2D11F11 |
| PSA | Clone PA05 (Neomarkers) |
| PSMA | Clone ZMD.80 (Zymed)[P] |
| p27 | Clone DCS72 (Oncogene) |
| Her-2/neu | KIT DAKO[P] |

[P]polyclonal, the rest are monoclonal

Negative control slides received normal mouse serum (DAKO) as the primary antibody. Slides were counterstained with Harris hematoxylin and reviewed by two independent pathologists with all discrepancies resolved by a third pathologist. The recorded IHC data from all 539 patients and their respective triplicate cores included the percentage and intensity (0-3+) of cells which stained for a particular antigen under investigation. Where applicable, these two measures were combined to create a Staining Index for that particular biomarker (Table 6, below, shows an exemplary list of molecular features). A Staining Index was calculated for AR (Androgen Receptor), CK14 (Cytokeratin 14), Cyclin D1, PSA (Prostate Specific Antigen), PSMA (Prostate Specific Membrane Antigen), p27 and Her2/neu while the remaining markers (i.e., Ki67, CK18 (Cytokeratin 18), CD45, CD68) were evaluated based on percentage of positive cells with a given intensity. These biomarkers are further described below. The Staining Index ranged from 0-300, and was calculated as follows: 1*(the percentage of cells staining positive with 1+ intensity for a biomarker) + 2*(the percentage of cells staining positive with 2+ intensity for the biomarker) + 3*(the percentage of cells staining positive with 3+ intensity for the biomarker), where the percentage of cells staining positive refers to the number of positive cells identified per every 100 cells counted. Additional details regarding this staining index are described in [19]. Such a staining index is only illustrative and any other suitable way for measuring molecular features may be used without departing from the scope of the present invention.

In the discussion of biomarkers above, p27 belongs to the family of cell cycle regulators called cyclin-dependent kinase inhibitors, which bind to cyclin-CDK complexes and cause cell cycle arrest in the G1 phase. The biomarker p27 is postulated to promote apoptosis and play a role in terminal differentiation of some tissues. By immunohistochemistry, the loss of nuclear p27 expression is associated with a more aggressive phenotype. Her2/neu is a member of the EGFR family of receptor tyrosine kinases and plays an important role in the pathogenesis of certain human cancers. The over-expression of Her2/neu by immunohistochemistry on cellular membranes has been associated with a more aggressive type of breast cancer. Ki67 is one of many proliferative markers that stains the nucleus with varying degrees of intensity and is utilized to assess a proliferative index or measure of cellular activity of the tumor sample in question. CD45 is a cell surface antigen that is used to identify cells that are destined to become immune cells such as lymphocytes (T cells, B-cells, NK cells etc.). The intensity is believed not to be as important as its distribution / presence and association with other histological elements. CD68 is a cytoplasmic antigen closely associated with lysosomes. It is expressed throughout the monocyte differentiation cascade but is usually more intense in macrophages than monocytes.

**Table 6. Molecular Features**

| Feature | Description |
|---------|-------------|
| atki67t1 | Ki-67 in intensity area 1 (tumor) |
| atki67t2 | Ki-67 in intensity area 2 (tumor) |
| atki67t3 | Ki-67 in intensity area 3 (tumor) |
| atki67p1 | Ki-67 in intensity area 1 (PIN) |
| atki67p2 | Ki-67 in intensity area 2 (PIN) |
| atki67p3 | Ki-67 in intensity area 3 (PIN) |
| atki67a1 | Ki-67 in intensity area 1 (gland) |
| atki67a2 | Ki-67 in intensity area 2 (gland) |
| atki67a3 | Ki-67 in intensity area 3 (gland) |
| atc18t3 | Cytokeratin 18 (tumor) |
| atcd45t3 | CD45 (tumor) |
| atcd68t3 | CD68 (tumor) |
| atcd34p | CD34 (PIN) |
| atcd34s | CD34 (stroma) |
| atcd34t | CD34 (tumor) |
| atcd34tp | CD34 (tumor/PIN) |
| atcd34ts | CD34 (tumor/stroma) |
| atcd34ps | CD34 (PIN/stroma) |
| atc18p3 | Cytokeratin 18 (PIN) |
| atcd45p3 | CD45 (PIN) |
| atc18a3 | Cytokeratin 18 (gland) |
| atcd45a3 | CD45 (gland) |
| arsi | AR (tumor) staining index |
| c14si | Cytokeratin 14 (tumor) staining index |
| cd1si | Cyclin D1 (tumor) staining index |
| psasi | PSA (tumor) staining index |
| psmasi | PSMA (tumor) staining index |
| p27si | p27 (tumor) staining index |
| her2si | Her-2/neu (tumor) staining index |
| arpsi | AR (PIN) staining index |
| c14psi | Cytokeratin 14 (PIN) staining index |
| cdlpsi | Cyclin D1 (PIN) staining index |
| psapsi | PSA (PIN)staining index |
| psmapsi | PSMA (PIN)staining index |
| p27psi | p27 (PIN)staining index |
| her2psi | Her-2/neu (PIN) staining index |

(continued)

| Feature | Description |
| --- | --- |
| arasi | AR (gland) staining index |
| c14asi | Cytokeratin 14 (gland) staining index |
| cdlasi | Cyclin D1 (gland) staining index |
| psaasi | PSA (gland) staining index |
| psmaasi | PSMA (gland) staining index |
| p27asi | p27 (gland) staining index |
| her2asi | Her-2/neu (gland) staining index |

**Analytical and Statistical Studies.** Three studies were conducted: an initial proof of concept analysis (Study 1) with 132 patients and an extended investigation (Study 2 and Study 3) using 268 patients. In both Study 1 and Study 2, the analysis consisted of two steps: identifying features predictive of PSA recurrence and developing a model based on those features, with the ultimate objective of using the model to predict biochemical (PSA) recurrence in future radical prostatectomy patients. The goals of Study 3 were to identify features and develop a model for predicting overall survival post-prostatectomy. Support Vector Regression for Censored data (SVRc) of the type described above was used to develop the resulting models in each of these studies.

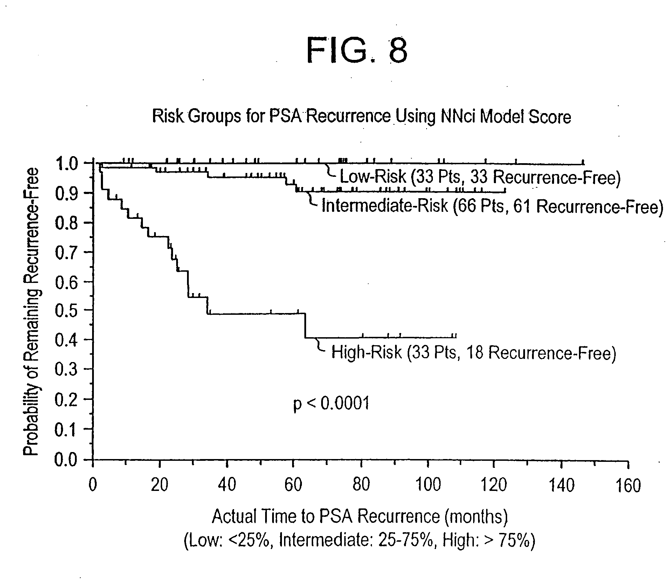

Predictive accuracy of a model was evaluated using the concordance index (CI). In dealing with censored outcomes this is often the metric of choice. The concordance index is based on pairwise comparisons between the prognostic scores of two randomly selected patients who meet any one of the following criteria: both patients experienced the event and the event time of the first patient is shorter than that of the second patient or only the first patient experienced the event and his event time is shorter than the second patient's follow-up time. The CI estimates the probability that a patient with the higher prognostic score from the model will experience the event within a shorter time than a patient with a lower score and is tightly associated with the area under the ROC curve (AUC). Other metrics may also be used to measure the ability of a predictive model. For example, sensitivity and specificity may be used in assessing diagnostics. As another example, a "p-value" may be used that represents the probability that chance alone is responsible for, for example, the observed differences between strata (e.g., see Figures 8, 10, and 12). Therefore, the lower the p-value, the more likely there is a true statistical association with outcome. Typically, the standard is that any p-value less than or equal to 0.05 is statistically significant.

*Study 1.* In this analysis, the above-described SVRc model was applied sequentially to the clinical, molecular, and morphometric data, with the clinical features first serving as an anchor for a "greedy-forward" feature selection ("FS") method via SVRc run on the molecular data. Following this step, a second SVRc greedy-forward feature selection method on the morphometric data was run, using the combination of the clinical and selected molecular features as the anchor. The last step involved running a greedy-backward selection method on the combination of the clinical, selected molecular and selected morphometric features to derive a final model. During feature selection, the criterion to determine whether a feature was entered (or kept) in the model was based on whether the presence (or absence) of that feature increased the concordance index, *i.e.* added predictive information.

The model was evaluated for predictive accuracy using both internal and external validation. Internal validation was performed using five-fold cross-validation. In order to perform external validation, a series of test sets of patients was created from the cohort of patients and predicted outcome was compared to actual outcome for these patients via the concordance index. In applying this two-level validation design, a subset of patients were randomly selected from the full set of patient records and only the remaining patients were used to build the predictive model using the procedure just described. The withheld records were then used to apply to the trained model in order to get a predictive accuracy. These two steps were repeated B times to get B predictive rates where the final predictive rate was the average. Features selected for the final model were those that appeared a sufficient amount of times in the B distinct models created.

Using the selected feature set, a neural network model was developed via directly maximizing the concordance index. Particularly, a neural network (NNci) of the type described above was used, in which network was trained using an objective function substantially in accordance with an approximation of the concordance index. The output of this final model was used to estimate individual future patient risk for PSA recurrence.

*Study 2.* The goals of this study were identical to Study 1; however, different feature selection and validation procedures were used. Instead of using the anchoring approach, all of the features were ranked by their association with time to PSA recurrence (measured by the concordance index) and those features which passed a certain pre-determined threshold (CI ≥ 0.60) were selected. This was done after the number of imaging features was reduced by our domain experts, and these features were then evaluated in a series of n-feature models (e.g. 1-feature, 2-feature, 3-feature, etc.). Using a forward feature selection process, the features that maximized the concordance index of each n-feature model were used in the next n+1-feature model. This process ended once the CI could not be improved by a pre-determined threshold. Then using a backward feature selection process, features were removed in an effort to increase the CI. This process was terminated when the removal of any feature did not improve the CI.

A simple bootstrapping technique was used for feature selection. In this approach, patients were sampled with replacement and used as a training set while the model was evaluated on those not selected. As a comparison, this feature selection method was run using only those features found in the Kattan post-operative nomogram, which is described in Kattan et al U.S. Patent No. 6,409,664, which is hereby incorporated by reference herein in its entirety. The output of the final model was used to estimate individual future patient risk for PSA recurrence.

*Study 3.* The goal of this study was to identify features predictive of overall survival using the same cohort and feature set analyzed in Study 2 as well as the same feature selection method. The output of the final model was used to estimate individual future patient risk for death due to any cause.

## RESULTS

The general approach was to apply systems pathology (the combination of morphometric analyses, molecular signatures and patient clinical profiles) to develop predictive models for PSA recurrence and overall survival in a cohort of prostate cancer patients status post prostatectomy. It is important to note that when clinicopathological features alone from Study 1 were utilized in a standard Cox Model analysis, the accuracy for predicting PSA recurrence was only 59%. It was only after the integration of morphometric and molecular features with SVRc that the level of predictive accuracy was increased to 88%. The following sections describe how this improvement was achieved.

*Study 1.* For the 132 patients in this cohort, the median age at diagnosis was 63 years (min: 40, max: 81), and the median pre-operative PSA was 8.2 ng/dl (min: 1.1, max: 81.9). Based on the prostatectomy samples, 32% had a Gleason score less than 7, 60% were Gleason 7 and the remaining 8% were greater than 7. Sixty-nine patients (52%) were pT2N0M0, 40 patients (30%) pT3aN0M0, and the remaining 23 patients (18%) pT3bN0M0 or pT1-3N+. (Table 7 contains a summary list of clinical characteristics for the three studies).

**Table 7. Clinical Information**

|  | Study 1 | Study 2 and 3 |
|---|---|---|
| N | 132 | 268 |
| Age (years) |  |  |
| Mean | 62 | 62 |
| Median | 63 | 63 |
| Range | 40 - 81 | 40 - 81 |
|  | Study 1 | Study 2 and 3 |
| Race |  |  |
| Caucasian | 120 (90.9%) | 241 (89.9%) |
| Hispanic | 8(6.1%) | 12 (4.5%) |
| African-American | 2 (1.5%) | 9 (3.4%) |
| Unknown | 2 (1.5%) | 6 (2.2%) |
| Pre-operative PSA (ng/dl) |  |  |
| Mean | 12.2 | 10.8 |
| Median | 8.2 | 7.8 |
| Range | 1.1 - 81.9 | 0.9 - 81.9 |
| TNM Stage |  |  |
| pT2N0 | 69 (52.3%) | 157 (58.6%) |
| pT3aN0 | 40 (30.3%) | 72 (26.9%) |
| pT3bN0 | 13 (9.8%) | 22 (8.2%) |
| pT1-3N+ | 10 (7.6%) | 17 (6.3%) |
| UICC Stage |  |  |
| T1a < 5% | 0 (0.0%) | 1 (0.3%) |
| T1b $\geq$ 5% | 0 (0.0%) | 1 (0.3%) |
| T1c not palpable or visible | 49 (37.1%) | 112 (41.8%) |
| T2a $\leq$ ½ lobe | 23 (17.4%) | 58 (21.7%) |

(continued)

| UICC Stage | | |
|---|---|---|
| T2b ≤ 1 lobe | 27 (20.5%) | 45 (16.8%) |
| T2c both lobes | 23 (17.4%) | 34 (12.7%) |
| T3a unilateral ECE | 8 (6.1%) | 15 (5.6%) |
| T3c SV+ | 2 (1.5%) | 2 (0.8%) |
| **DRE Result** | | |
| Non-palpable | 56 (42.4%) | 118 (44.0%) |
| Palpable | 76 (57.6%) | 150 (56.0%) |
| **Lymph Node Involvement** | | |
| Negative | 121 (91.7%) | 250 (93.3%) |
| Positive | 11 (8.3%) | 18 (6.7%) |
| **Seminal Vesicle Involvement** | | |
| No | 113 (85.6%) | 236 (88.0%) |
| Yes | 19 (14.4%) | 32 (12.0%) |
| **Surgical Margins** | | |
| Negative | 108 (81.8%) | 217 (81.0%) |
| Positive | 24 (18.2%) | 51 (19.0%) |
| **Extracapsular Involvement** | | |
| No | 70 (53.0%) | 159 (59.3%) |
| Yes | 62 (47.0%) | 109 (40.7%) |
| **Dominant Biopsy Gleason Grade** | | |
| 1 | 0 (0.0%) | 1 (0.4%) |
| 2 | 24 (18.2%) | 43 (16.0%) |
| 3 | 85 (64.4%) | 184 (68.7%) |
| 4 | 22 (16.7%) | 38 (14.2%) |
| 5 | 1 (0.7%) | 2 (0.8%) |
| **Biopsy Gleason Score** | | |
| 2 | 0 (0.0%) | 1 (0.4%) |
| 3 | 0 (0.0%) | 0 (0.0%) |
| 4 | 6 (4.6%) | 7 (2.6%) |
| 5 | 27 (20.5%) | 56 (20.9%) |
| 6 | 41 (31.1%) | 97 (36.2%) |
| 7 | 48 (36.4%) | 90 (33.6%) |
| 8 | 7 (5.3%) | 13 (4.9%) |
| 9 | 3 (2.3%) | 4 (1.5%) |
| | Study 1 | Study 2 and 3 |
| **Dominant Post-operative Gleason Grade** | | |
| 2 | 3 (2.3%) | 20 (7.5%) |
| 3 | 98 (74.2%) | 201 (75.0%) |
| 4 | 31 (23.5%) | 47 (17.5%) |
| **Post-operative Gleason Score** | | |
| 5 | 6 (4.6%) | 21 (7.8%) |
| 6 | 36 (27.3%) | 86 (32.1%) |
| 7 | 79 (59.9%) | 148 (55.2%) |
| 8 | 10 (7.6%) | 12 (4.5%) |
| 9 | 1 (0.8%) | 4 (0.4%) |

(continued)

| Ploidy | | |
|---|---|---|
| Diploid | 74 (56.1%) | 145 (54.1%) |
| Tetraploid | 54 (40.9%) | 115 (42.9%) |
| Aneuploid | 4 (3.0%) | 8 (3.0%) |
| Percent Ploidy in S Phase (%) | | |
| Mean | 2.3 | 2.4 |
| Median | 1.1 | 1.1 |
| Range | 0.0 - 63.8 | 0.0 - 66.4 |
| Percent Ploidy Fraction | | |
| Mean | 3.4 | 3.5 |
| Median | 2.6 | 2.4 |
| Range | 0.0 - 20.0 | 0.0 - 20.0 |

Twenty (15%) patients experienced PSA recurrence, while the remaining patients (85%) were censored. For censored patients, the median follow-up time was 60.8 months, or just over 5 years. The overall median time to PSA recurrence was not reached. All seventeen clinical features were selected as being predictive of PSA recurrence, with the most informative being annotated as follows (clinicopathological feature and # of times selected by the model): biopsy Gleason grade (112), race (112), UICC clinical stage (110), ploidy (110), and DRE results (109).

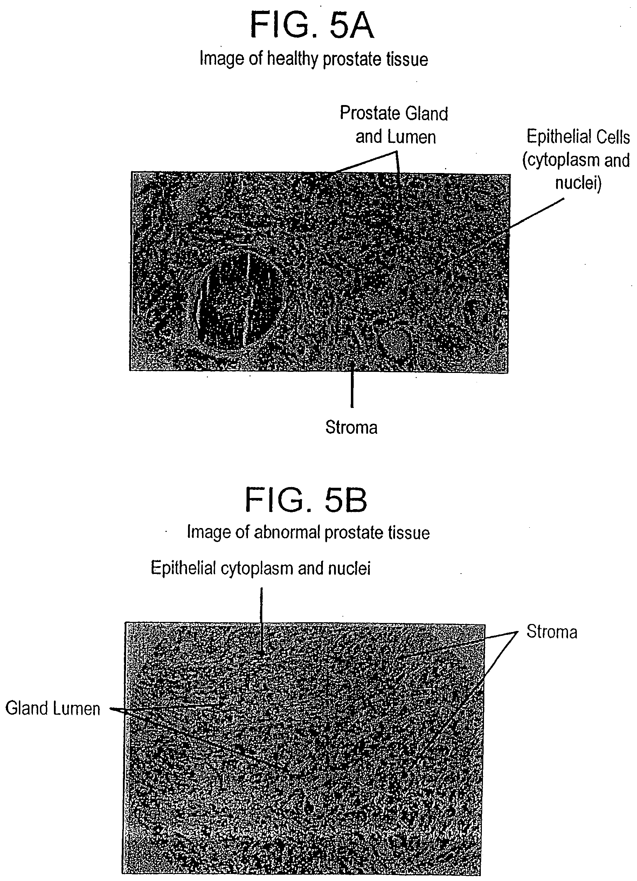

**Image Analysis and Morphometry Studies.** Figures 5a and 5b illustrate digitized images of healthy and abnormal prostate tissue, respectively, obtained after segmentation and classification in accordance with the present invention. Various pathological objects have been labeled in the tissue for illustration. A total of 496 morphometric features (shown in Table 1) were generated by the image analysis software.

Of the 496 morphometric features, the 10 morphometric features shown in Figure 6 were selected as being predictive of PSA recurrence. The morphometric features selected related to the following pathological objects, where the numbers in parentheses next to the features indicate how many times the features were selected as correlated with outcome during generation of the final model: red blood cell, epithelial nuclei, lumen, stroma, cytoplasm, and tissue background (Red Blood Cell Minimum Length in Pixels (20), Epithelial Nuclei Maximum Compactness (17), Lumen Minimum Radius of Smallest Enclosure (14), Epithelial Nuclei Minimum Width in Pixels (11), Stroma Maximum Density (10), Lumen Maximum Border Length in Pixels (10), Epithelial Nuclei Minimum Standard Deviation Channel 2 (10), Epithelial Nuclei Maximum Radius of Smallest Enclosure (10), Cytoplasm Standard Deviation of Border Length in Pixels (10), and Background Standard Deviation of Area in Pixels(10)). More particularly, in this example, the morphometric features of length for red blood cell, radius of smallest enclosure and border length for lumen, border length for cytoplasm, density for stroma (e.g., square root of the area covered by a stroma divided by its radius), and area for background were determined to correlate with outcome. The morphometric features of compactness, width, green channel value, and radius of smallest enclosure for epithelial nuclei (e.g., ellipse with the same area as the object is created and then enlarged until it completely encloses the epithelial nuclei, and the ratio of the radius of the smallest enclosing ellipse to the radius of the original ellipse is computed) were also determined to correlate with outcome.

Various possible reasons for at least some of these correlations are described above in connection with Example 1. For example, the morphometric feature of compactness of the epithelial nuclei may be a reflection of the 'back to back' nature of epithelial cells in a circumferential pattern which would suggest a loss of glandular and lumen formation / differentiation and therefore be consistent with a higher Gleason grade (i.e., higher disease progression). Also, the morphometric feature of the radius of smallest enclosure of the lumen relates to the overall size of the lumen which is dramatically reduced and diminished as the Gleason grade increases.

In addition, the correlations determined in this study may be at least partially explained by the hypothesis that epithelial nuclei typically become less diverse in shape (e.g., more round with less variations) and size (e.g., area and border length) and have less color variation as the epithelial nuclei invade the stroma. This invasion of the stroma may also explain why morphometric features of the stroma have been determined to be correlated with disease progression. Particularly, cancerous images are typically characterized by a small amount of stroma because the stroma area is replaced by epithelial cell cytoplasm as cancer progresses. This causes density values for stroma to be higher because the stroma compactness is reduced and becomes more fractal in shape (the object radius increases more than the area as objects deform and become thinner). Additional reasoning for the correlations determined in this study may be that an abundance of red blood cells traveling through the tissue may reflect some measure of angiogenesis or new blood vessel formation which may be related to disease progression as a means for cells to leave the prostate and seed externally - thus impacting on the clinical outcome of PSA / BCR recurrence.

As stated above, it will be understood that at least some of the particular morphometric features determined by the teachings provided herein to correlate with outcome may depend on, for example, the particular hardware, software, or combination thereof that is used by the present invention to calculate the morphometric features. The Definiens Cellenger software and the particular morphometric features measured by the software described herein are only illustrative and any other hardware, software, or combination thereof may be used without departing from the scope of the invention.

**Molecular Analysis.** Of the 12 biomarkers that were evaluated by IHC, a total of 43 unique features were recorded. (Tables 8a, 8b, and 8c, below, show a summary of the observed biomarker - molecular features).

Table 8a. Cells (%) Staining (+) by Histologic Component and Intensity (Study 1)

| Marker | Tumor | | | PIN | | | Gland | | |
|---|---|---|---|---|---|---|---|---|---|
| | 1+ | 2+ | 3+ | 1+ | 2+ | 3+ | 1+ | 2+ | 3+ |
| Ki-67 | | | | | | | | | |
| Mean ± SD | 23.9 ± 31.38 | 9.8 ± 21.32 | 2.4 ± 4.64 | 25.3 ± 32.50 | 10.3 ± 21.51 | 2.6 ± 3.29 | 1.8 ± 9.96 | 0.0 ± 0.36 | 0.1 ± 0.63 |
| Median | 4.7 | 0.0 | 0.0 | 4.8 | 0.0 | 0.0 | 0.0 | 0.0 | 0.0 |
| Range | 0.0 - 100.0 | 0.0-100.0 | 0.0 - 26.3 | 0.0-100.0 | 0.0-100.0 | 0.0-39.5 | 0.0-96.0 | 0.0-4.0 | 0.0 - 6.3 |
| CK 18 | | | | | | | | | |
| Mean ± SD | NA | NA | 100.0 ± 0.00 | NA | NA | 100.0 ± 0.00 | NA | NA | 100.0 ± 0.00 |
| Median | NA | NA | 100.0 | NA | NA | 100.0 | NA | NA | 100.0 |
| Range | NA | NA | 100.0-100.0 | NA | NA | 100.0 - 100.0 | NA | NA | 100.0-100.0 |
| CD45 | | | | | | | | | |
| Mean ± SD | NA | NA | 0.0 ± 0.04 | NA | NA | 0.0 ± 0.01 | NA | NA | 0.0 ± 0.00 |
| Median | NA | NA | 0.0 | NA | NA | 0.0 | NA | NA | 0.0 |
| Range | NA | NA | 0.0 - 0.4 | NA | NA | 0.0 - 0.1 | NA | NA | 0.0 - 0.0 |
| CD68 | | | | | | | | | |
| Mean ± SD | NA | NA | 0.0 ± 0.01 | NA | NA | NA | NA | NA | NA |
| Median | NA | NA | 0.0 | NA | NA | NA | NA | NA | NA |
| Range | NA | NA | 0.0 - 0.1 | NA | NA | NA | NA | NA | NA |

Table 8b. CD34 Cells (%) Staining (+) by Histologic Component (Study 1)

| | PIN | Stroma | Tumor | Tumor/PIN | Tumor/Stroma | PIN/Stroma |
|---|---|---|---|---|---|---|
| Mean ± SD | 0.0 ± 0.05 | 0.0 ± 0.03 | 0.1 ± 0.21 | 0.0 ± 0.06 | 0.0 ± 0.08 | 0.0 ± 0.05 |
| Median | 0.0 | 0.0 | 0.0 | 0.0 | 0.0 | 0.0 |
| Range | 0.0-0.4 | 0.0 - 0.2 | 0.0 - 0.9 | 0.0 - 0.5 | 0.0 - 0.4 | 0.0 - 0.3 |

Table 8c. Staining Index (0 - 300) by Histologic Component (Study 1)

| Marker | Tumor | PIN | Gland |
|---|---|---|---|
| AR | | | |
| Mean ± SD | 179.8 ± 71.4 | 64.3 ± 75.10 | 22.6 ± 56.86 |
| Median | 200 | 36.5 | 0 |

(continued)

| Marker | Tumor | PIN | Gland |
|---|---|---|---|
| AR | | | |
| Range | 0 - 300 | 0 - 300 | 0 - 300 |
| CK14 | | | |
| Mean ± SD | 2.6 ± 5.83 | 31.2 ± 57.35 | 4.7 ± 20.42 |
| Median | 0 | 0 | 0 |
| Range | 0 - 42 | 0 - 285 | 0 - 150 |
| Cyclin D1 | | | |
| Mean ± SD | 1.5 ± 5.15 | 0.0 ± 0.27 | 0.0 ± 0.0 |
| Median | 0 | 0 | 0 |
| Range | 0 - 33 | 0 - 3 | 0 - 0 |
| PSA | | | |
| Mean ± SD | 128.0 ± 68.85 | 135.7 ± 97.88 | 13.9 ± 41.32 |
| Median | 100 | 111 | 0 |
| Range | 0 - 300 | 0 - 300 | 0 - 201 |
| PSMA | | | |
| Mean ± SD | 0.5 ± 2.97 | 9.5 ± 26.93 | 2.5 ± 15.00 |
| Median | 0 | 0 | 0 |
| Range | 0 - 21 | 0 - 154 | 0-99 |
| p27 | | | |
| Mean ± SD | 4.3 ± 9.61 | 7.0 ± 19.49 | 2.1 ± 12.03 |
| Median | 0 | 0 | 0 |
| Range | 0 - 80 | 0 - 140 | 0 - 120 |
| Her-2/neu | | | |
| Mean ± SD | 4.1 ± 18.50 | 0.1 ± 1.00 | 0.0 ± 0.00 |
| Median | 0 | 0 | 0 |
| Range | 0 -1 46 | 0 - 10 | 0 - 0 |

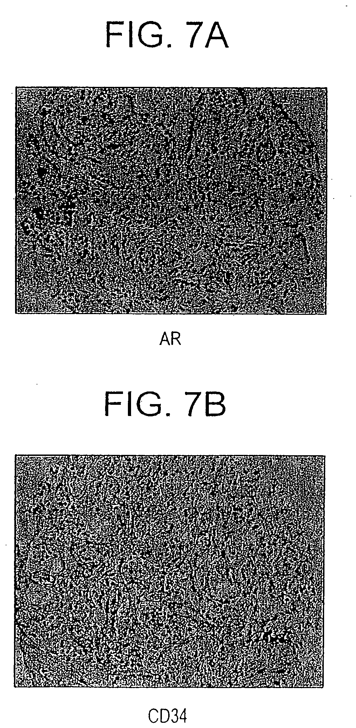

From these 12 antibodies, 8 biomarkers encompassing 14 specific molecular features were selected as being associated with PSA recurrence. Some examples of the more highly selected molecular features are annotated as follows (biomarker - # times selected by the model) and include : AR Staining Index - tumor (93), AR Staining Index - atrophic gland (54), CD34 - associated Tumor / PIN (22), Ki-67 - tumor (18) and CD45 - associated with PIN (17), where PIN is an abbreviation for prostatic intraepithelial neoplasm. Figures 7a and 7b illustrate representative fields demonstrating expression profiles for AR and CD34, respectively. The profile of biomarker expression was noteworthy for the highly selected and somewhat heterogeneous expression patterns of AR and CD34. These markers and their relationship to tumor, atrophic glands (for AR) and Tumor / PIN (for CD34) suggest biological and functional significance impacting on the clinical outcome of PSA recurrence. The second group of selected markers included Ki-67 and CD45 both of which had prominent but overall low selection frequency when compared with AR and CD34.

**Analytical and Statistical Studies.** Using both domain expertise and domain-specific feature selection procedure above where 120 random splits were created for training (N=100) and testing (N=32) the models, the final feature set was reduced to 41 total features of which 17 were clinical, 10 morphometric, and 14 molecular. Figure 5 shows a complete list of the selected features. The 10 morphometric features are described above. The clinical and molecular features are further described below.

**Clinical Features**

1. Biopsy Gleason Score: the summarized Gleason grades (dominant and secondary) which are assigned to the multiple Needle Biopsy Tissue Samples received by a pathologist. The Gleason scoring system was developed to create a standardized, somewhat subjective, means of representing the architecture of prostatic adenocarcinoma by histology with the production of individual grades. The grades range from 1 - 5 based on the degree of differentiation of the glandular units and epithelial cells. The dominant (primary) and sub-dominant (secondary) patterns are added together to create a Gleason Summary. In addition, the features of overall stromal compactness, epithelial cell size and nuclear features are occasionally considered in the overall grading system.

2. Race (e.g., African American, Caucasian, etc.)

3. UICC Stage: International Union against Cancer TNM staging system use to define clinical staging for cancer, where "T" stands for Tumor size, "N" stands for lymph node involvement and "M" stands for metastasis to a distant site.

4. Ploidy Result: DNA content which is a reflection of the overall DNA content within the prostate cancer epithelial cells. Benign cells and well-behaved tumor cells grow and divide in an orderly fashion. In the resting state, they contain one complete set of chromosomes (this is the diploid condition). This complete set of chromosomes consists of 23 chromosomes (or N) from Ma and 23 (N again) chromosomes from Pa (equaling a total of 2N). A cell must double the number of its chromosomes before it can divide, creating two complete sets of chromosomes (this is 4N, or the tetraploid state). After division is completed, each new cell receives half of the genetic material and therefore becomes diploid (2N) once again. If DNA ploidy analysis were to be performed on a group of these cells, one would see that most of the cells would be diploid and a small fraction of them (those getting ready to divide) would be tetraploid. Additionally, in measuring and creating a graph of the amount of genetic material in each cell, one would see a dominant diploid peak and a minor tetraploid peak. The amount of DNA in a cell can be measured by staining it with a dye that binds to the genetic material. The concentration and distribution of this dye (Fuelgen stain) can be measured by image analysis microscopy.

When tumors worsen they tend to not divide as orderly as they once did. Instead of the resting state having a complete set of chromosomes, the resting state may only have a set and a half. Such cells would have a DNA content that was neither diploid nor tetraploid but mid-way between. Plotting these cells on the above-described graph above would yield an aneuploid peak midway between the other two peaks. Studies have shown that tumors that have a significant aneuploid peak do not behave as well as those that do not. This is not surprising because a strong correlation exists between ploidy status and nuclear grade. A nuclear grade can be assessed by any pathologist with enough experience with prostate cancer. The value that DNA ploidy analysis adds is that it is an objective measurement that can be compared between labs using standardized techniques and that can be used to perform a quick check on the approximate accuracy of Gleason scoring. For example, any Gleason score 2+2=4 or 2+3=5 tumor that has an aneuploid peak should potentially be re-evaluated for possible adjustment to the score.

5. DRE Result: Result from a digital rectal exam (e.g., negative or positive) which is utilized to determine extent of disease both within the prostate as well as extra prostatic extension by palpation.

6. Lymph Node Involvement: a measure of the extent to which lymph nodes contain tumor cells (e.g., prostate cancer epithelial cells), which can be assessed either by clinical / surgical inspection or at the time of a prostatectomy.

7. Dominant Biopsy Gleason Grade: See above description of Biopsy Gleason Score. This reflects the dominant Gleason grading pattern seen on either a biopsy or a prostatectomy specimen.

8. Percent Ploidy in S Phase: represents a fraction of the cellular content which is in a proliferative or S phase of the cell cycle and reflects the growth potential of the tumor.

9. Post-operative Gleason Score: Scoring of tissue taken after surgery from various regions of the prostate resection sample.

10. TNM Stage: Tumor, Node and Metastasis based on the UICC criteria post prostatectomy and based on pathologic examination of tissue samples.

11. Dominant Post-operative Gleason Grade: the dominant Gleason grade which represents the most predominant histologic feature present in the prostatectomy specimen.

12. Age

13. Seminal Vesicle Involvement: Invasion of the seminal vesicle by tumor.

14. Pre-operative PSA: PSA level observed prior to surgery

15. Percent Ploidy Fraction: See above description of ploidy result.

16. Surgical Margin Involvement: Involvement of the surgical margins by tumor which reflects the extent to which the bed from which the tumor/prostate was removed at the time of surgery contained tumor cells.

17. Extracapsular Involvement: Extension of the tumor beyond the capsule of the prostate.

**Molecular Features**

1. AR - tumor: Androgen Receptor (AR) Staining Index for a tumor, which is a measure of the percentage and intensity of cells staining positive for AR. With respect to prostate cancer, the staining index may represent the degree of brown reaction product which is detected in the nuclei of epithelial cells in the prostate samples evaluated.

2. AR - gland: AR Staining Index for a tumor, which is present within a glandular structure.

3. CD34 - tumor/PIN: The localization of CD34 to the endothelial cells of vessels which are associated with tumor and PIN.

4. Ki67 - tumor 2: The identification of ki67 positive nuclei in tumor epithelial cell nuclei.

5. CD45 - PIN 3: The identification fCD45 positive lymphocytes in association with PIN.

6. CD34 - tumor/stroma: The localization of CD34 vessels which are associated with tumor.

7. Ki-67 - tumor 3: see above.

8. p27 - tumor: The identification of p27 in the nuclei of tumor epithelial cells.

9. C14 - PIN: The identification of cytokeratin 14 in the (epithelial) basal cells of the glandular unit.

10. CD34 - tumor: The localization of CD34 to vessels which are associated with the tumor.

11. PSA - gland: The identification of PSA to the luminal epithelial cells of the gland unit.

12. PSMA - PIN: The identification of PSMA to the glandular / luminal cells of regions identified as PIN.

13. CD34 - PIN/stroma: The localization of CD34 to vessels associated with PIN.

14. CD45 - tumor 3: The identification of CD45 positive lymphocytes which are associated with tumor.

As each domain of data was analyzed during this process using SVRc, the predictive accuracy of the models increased. Using internal validation, when looking at the clinical data alone, the concordance index was 0.79. By adding features from the molecular domain, the concordance index increased to 0.81. The final model, formed by the addition of the morphometric features, reached a concordance index of 0.84. Each of these internally-validated models was also validated externally (as described above in Materials and Methods) with the same trend being noted. Using NNci on the final selected set of features, the concordance index reached 0.88.

The resulting output of the NNci and the SVRc models can be interpreted as a relative risk estimate of PSA recurrence for an individual patient. Using the quartiles of this score ($\leq$25%, >25%-75%, >75%), risk groups of patients were created; the Kaplan-Meier estimates of recurrence for each risk group according to the NNci model are presented in Figure 8. The groups showed a statistically significant difference in time to PSA recurrence (log-rank test, p-value < 0.0001). The p-value represents the probability that chance alone is responsible for the observed differences between strata (risk groups in these examples). Therefore, the lower the p-value, the more likely you are seeing a true statistical association. Generally, any p-value less than or equal to 0.05 is statistically significant.

*Study 2.* For the 268 patients in this cohort, which contains 129 of the 132 patients analyzed in Study 1, the median age at diagnosis was 63 years (min: 38, max: 81), and the median PSA prior to radical prostatectomy was 7.8 ng/dl (min: 0.9, max: 81.9). Based on the prostatectomy samples, 40% of tumors had a Gleason Score less than 7, while 55% of the prostatectomies had a Gleason 7. The remaining 5% of prostatectomies had a Gleason Score greater than 7. One hundred fifty-seven patients (59%) were diagnosed as having pT2N0M0 disease, 72 patients (27%) as pT3aN0M0, and the remaining 39 patients (14%) as pT3bN0M0 or pT1-3N+. (*See* Table 5, *supra* for details of all analyzed clinicopathological features for this cohort). Thirty-eight (14%) patients experienced PSA recurrence, while the remaining patients (86%) were censored. For censored patients, the median follow-up time was 58.7 months, or just under 5 years. The overall median time to PSA recurrence was not reached. Three clinical features were selected as being predictive of PSA recurrence: TNM clinical stage, surgical margins, and lymph nodes.

**Image Analysis and Morphometry Studies.** Using an updated version of the image analysis software but analyzing the same H&E stained slides, a total of 350 morphometric features were generated (shown in Table 2, above).

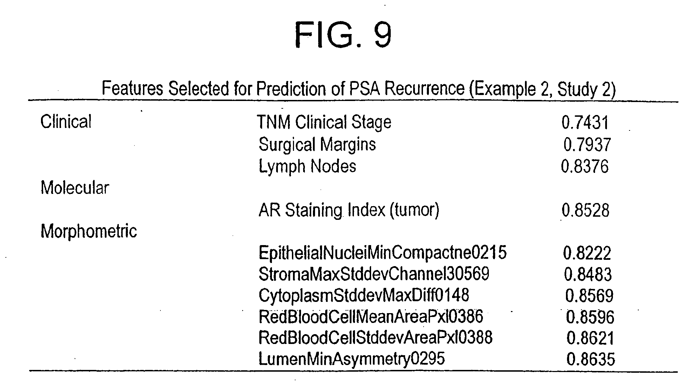

Figure 9 shows that, of the 350 features, 6 morphometric features were selected as being predictive of PSA recurrence, where these morphometric features related to the pathological objects of epithelial nuclei, stroma, cytoplasm, red blood cell, and lumen (i.e., EpithelialNucleiMinCompactne0215, StromaMaxStddevChannel30569, CytoplasmStddevMaxDiff0148, RedBloodCellMeanAreaPxl0386, RedBloodCellStddevAreaPxl0388, and LumenMinAsymmetry0295). More particularly, in this study, the morphometric features of compactness of epithelial nuclei, blue channel value for stroma, max difference for cytoplasm (e.g., minimum mean value belonging to cytoplasm subtracted from its maximum value over all color channels for the cytoplasm, where the result is divided by the object brightness), area for red blood cells, and asymmetry of lumen were selected as being correlated with outcome.

Various possible reasons for at least some of these correlations are described above in connection with Example 1 and/or Study 1. For example, morphometric features including the compactness of the epithelial cells, the variation and disruption of the stroma by infiltrating epithelial cells, and the evidence of reduced lumen size would all provide histologic evidence of a higher Gleason grade (i.e., higher disease progression). A higher Gleason grade suggests a more aggressive prostate tumor which would support metastasis and or extension of tumor supporting PSA recurrence post surgery. In addition, the identification of red blood cells in various formats would suggest an abundance of vessels. The evidence of additional vessels would create a possible route for which epithelial cells could exit the prostate and be distributed in external locations producing PSA.

Clinical and molecular features selected in study 2 are shown in Figure 9 and listed below. Descriptions of these clinical and molecular features are provided above.

**Clinical Features**

1. TNM stage
2. Surgical Margin Involvement
3. Lymph Node Involvement

**Molecular Feature**

1. AR Staining Index (tumor)

Each number in Figure 9 represents the concordance index of a predictive model based on the corresponding feature and all other feature(s) in Figure 9 having smaller number(s). For example, 0.8483 is the CI of a model based on features TNM Clinical Stage, Surgical Margins, EpithelialNucleiMinCompactne0215, Lymph Nodes, and StromaMaxStddevChannel30569. The CI of a model based on the same 5 features plus AR Staining Index (tumor) is 0.8528. In other words, the addition of the AR Staining Index molecular feature to the model increases the predictive power of the model.

**Molecular Analysis.** No additional immunohistochemistry studies were necessary. The data originally collected was used as described in Materials and Methods (see Tables 9a, 9b, and 9c for a complete summary of the molecular features).

**Table 9a. Cells (%) Staining (+) by Histologic Component and Intensity (Study 2 and Study 3)**

| | Tumor | | | PIN | | | Gland | | |
|---|---|---|---|---|---|---|---|---|---|
| **Marker** | **1+** | **2+** | **3+** | **1+** | **2+** | **3+** | **1+** | **2+** | **3+** |
| Ki-67 | | | | | | | | | |
| Mean ± SD | 22.1 ± 30.30 | 7.3 ± 17.04 | 1.9 ± 4.01 | 23.2 ± 31.36 | 7.9 ± 18.16 | 2.0 ± 4.46 | 1.3 ± 7.96 | 1.2 ± 9.78 | 0.3 ± 1.55 |
| Median | 1.3 | 0.0 | 0.0 | 1.0 | 0.0 | 0.0 | 0.0 | 0.0 | 0.0 |
| Range | 0.0 - 100.0 | 0.0 - 100.0 | 0.0 - 26.3 | 0.0 - 100.0 | 0.0 - 100.0 | 0.0-39.5 | 0.0 - 96.0 | 0.0 - 96.5 | 0.0 - 13.0 |
| CK 18 | | | | | | | | | |
| Mean ± SD | NA | NA | 100.0 ± 0.00 | NA | NA | 1.0 ± 0.04 | NA | NA | 100.0 ± 0.00 |
| Median | NA | NA | 100.0 | NA | NA | 100.0 | NA | NA | 100.0 |
| Range | NA | NA | 100.0 - 100.0 | NA | NA | 0.5 - 100.0 | NA | NA | 100.0 - 100.0 |
| CD45 | | | | | | | | | |
| Mean ± SD | NA | NA | 0.0 ± 0.04 | NA | NA | 0.0 ± 0.01 | NA | NA | 0.0 ± 0.00 |
| Median | NA | NA | 0.0 | NA | NA | 0.0 | NA | NA | 0.0 |
| Range | NA | NA | 0.0-0.4 | NA | NA | 0.0 - 0.1 | NA | NA | 0.0 - 0.0 |
| CD68 | | | | | | | | | |
| Mean ± SD | NA | NA | 0.0 ± 0.01 | NA | NA | NA | NA | NA | NA |
| Median | NA | NA | 0.0 | NA | NA | NA | NA | NA | NA |
| Range | NA | NA | 0.0 - 0.1 | NA | NA | NA | NA | NA | NA |

**Table 9b. CD34 Cells (%) Staining (+) by Histologic Component (Study 2 and Study 3)**

|  | PIN | Stroma | Tumor | Tumor/PIN | Tumor/Stroma | PIN/Stroma |
|---|---|---|---|---|---|---|
| Mean ± SD | 0.0 ± 0.04 | 0.0 ± 0.11 | 0.1 ± 0.18 | 0.0 ± 0.08 | 0.0 ± 0.08 | 0.0 ± 0.04 |
| Median | 0.0 | 0.0 | 0.0 | 0.0 | 0.0 | 0.0 |
| Range | 0.0 - 0.4 | 0.0 - 1.7 | 0.0 - 0.9 | 0.0 - 0.6 | 0.0 - 0.4 | 0.0 - 0.3 |

**Table 9c. Staining Index (0 - 300) by Histologic Component (Study 2 and Study 3)**

| Marker | Tumor | PIN | Gland |
|---|---|---|---|
| AR | | | |
| Mean ± SD | 172.1 ± 75.3 | 79.6 ± 82.74 | 28.9 ± 67.25 |
| Median | 200 | 66.0 | 0 |
| Range | 0 - 300 | 0 - 300 | 0 - 300 |
| CK14 | | | |
| Mean ± SD | 2.1 ± 6.32 | 34.4 ± 61.46 | 8.5 ± 32.62 |
| Median | 0 | 0 | 0 |
| Range | 0 - 69 | 0 - 300 | 0-300 |
| Cyclin D1 | | | |
| Mean ± SD | 1.4 ± 6.99 | 0.0 ± 0.21 | 0.0 ± 0.0 |
| Median | 0 | 0 | 0 |
| Range | 0 - 90 | 0 - 3 | 0 - 0 |
| PSA | | | |
| Mean ± SD | 118.3 ± 71.10 | 139.4 ± 97.16 | 22.8 ± 55.14 |
| Median | 100 | 134 | 0 |
| Range | 0 - 300 | 0 - 300 | 0 - 300 |
| PSMA | | | |
| Mean ± SD | 0.2 ± 2.09 | 6.4 ± 21.02 | 2.9 ± 22.94 |
| Median | 0 | 0 | 0 |
| Range | 0 - 21 | 0 - 154 | 0 - 300 |
| p27 | | | |
| Mean ± SD | 3.9 ± 8.20 | 6.4 ± 18.83 | 1.3 ± 8.65 |
| Median | 0 | 0 | 0 |
| Range | 0 - 48 | 0 - 140 | 0 - 120 |
| Her-2/neu | | | |
| Mean ± SD | 3.4 ± 16.69 | 0.2 ± 1.12 | 0.0 ± 0.00 |
| Median | 0 | 0 | 0 |
| Range | 0 - 150 | 0 - 10 | 0 - 0 |

A single molecular feature was selected as being predictive of PSA recurrence: AR Staining Index - tumor.

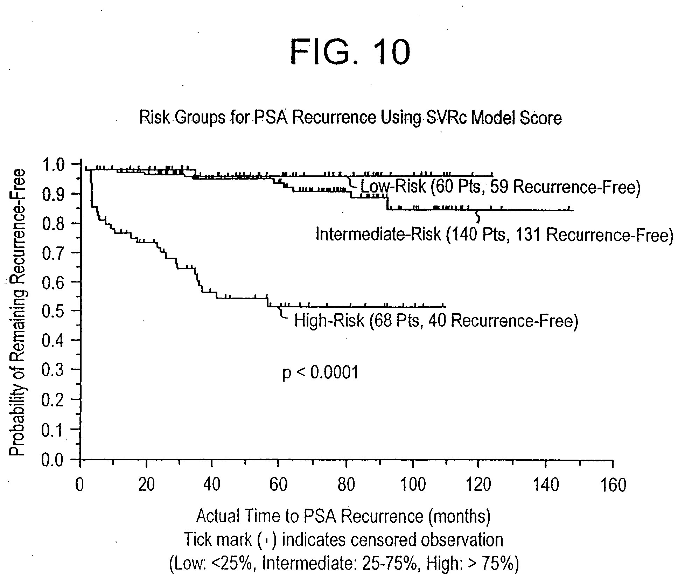

**Analytical and Statistical Studies.** Using domain expertise and simple bootstrapping, the method found a subset of 10 features (3 clinicopathological, 6 morphometric, and 1 molecular) that had a concordance index (CI) of 0.87 (FIG. 9 shows the complete list of selected features). The resulting output of the SVRc model can also be interpreted as a relative risk estimate of PSA recurrence for an individual patient. Using the quartiles of this score (<25%, >25%-75%, >75%), risk groups of patients were created; the Kaplan-Meier estimates of recurrence for each risk group as predicted by the SVRc model are presented in Figure 10. The groups showed a statistically significant difference in time to PSA recurrence (log-rank test, p-value < 0.0001).

*Study 3.* This study used the same cohort as that of Study 2 so that the clinicopathological characteristics of the patients are identical. In terms of outcome, nineteen (7%) patients died due to any cause, while the remaining patients (93%) were alive as of their last visit and censored. For censored patients, the median follow-up time was 64.8 months, or just over 5 years. The overall median time to death was not reached. Two clinical features were selected as being predictive of death due to any cause: TNM clinical stage and patient age.

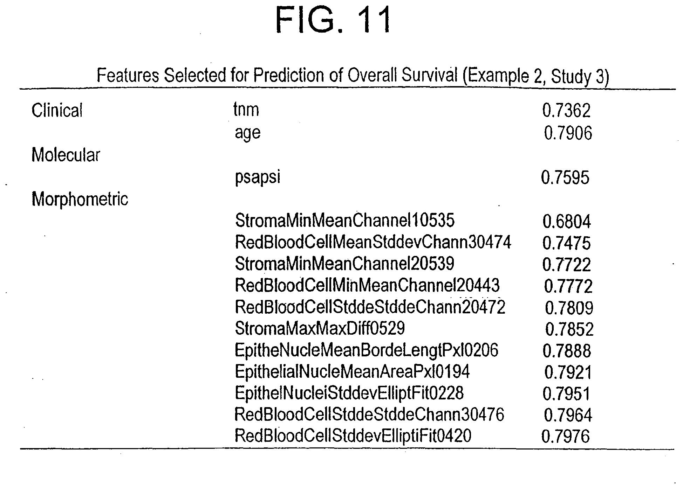

**Image Analysis and Morphometry Studies.** The same set of 350 morphometric features from Study 2 was used in this study. Figure 11 shows that, of the 350 features, 11 morphometric features were selected as being predictive of death due to any cause, where these features related to the pathological objects of stroma, red blood cell, and epithelial nuclei (i.e., StromaMinMeanChannel10535, RedBloodCellMeanStddevChann30474, StromaMinMeanChannel20539, RedBloodCellMinMeanChannel20443, RedBloodCellStddeStddeChann20472, StromaMaxMaxDiff0529, EpitheNucleMeanBordeLengtPxl0206, EpithelialNucleiMeanAreaPxl0194, EpithelNucleiStddevElliptFit0228, RedBloodCellStddeStddeChann30476, and RedBloodCellStddevElliptiFit0420, where "channel" refers to the red (R), green (G), and blue (B) color channels of an image). More particularly, in this study, the morphometric features of mean value of red color channel, mean value of blue color channel and max difference for stroma were determined to be correlated with outcome. The morphometric features of mean and standard deviation of red channel, mean and standard deviation of green channel and elliptic fit for red blood cell were determined to be correlated with outcome. To determine the morphometric feature of elliptic fit, an ellipse with the same area as the red blood cell was created, the area of the red blood cell outside the ellipse was compared with the area inside the ellipse that was not filled with the red blood cell, and a value of 0 was assigned where there was no fit whereas a value of 1 was assigned for a complete fitting object. The morphometric features of border length, area and elliptic fit for epithelial nuclei were determined to be correlated with outcome.

Various possible reasons for at least some of these correlations are described above in connection with Example 1 and/or Study 1. For example, the overall shape of the epithelial nuclei reflects a histologic appearance of a higher Gleason grade. Additionally, in this study, the correlation with respect to stroma may be explained by the understanding that stroma will exhibit a reduced contrast (as measured by the max difference morphometric feature) as cancer progresses due to its interruption with epithelial cells.

**Molecular Analysis.** The same set of molecular features from Study 2 was used in this study. A single feature was selected as being predictive of death due to any cause: PSA Staining Index - atrophic gland.

**Analytical and Statistical Studies.** In this cohort, a total of 14 features (2 clinicopathological, 11 morphometric, and 1 molecular) were selected. The final model had a concordance index (CI) of 0.80. The complete list of selected features are shown in Figure 11 and listed below. The clinical and molecular features selected are listed below. Descriptions of the clinical features are provided above.

## Clinical Features

1. TNM stage
2. age

## Molecular Feature

1. psapsi: refers to the staining index for prostate specific antigen (PSA) in the prostatic intraepithelial neoplasm (PIN).

Each number in Figure 11 represents the concordance index of a predictive model based on the corresponding feature and all other feature(s) in Figure 11 having smaller number(s). For example, 0.6804 is the CI of a model based on StromaMinMeanChannel10535 and 0.7362 is the CI when the model is based on both StromaMinMeanChannel10535 and TNM.

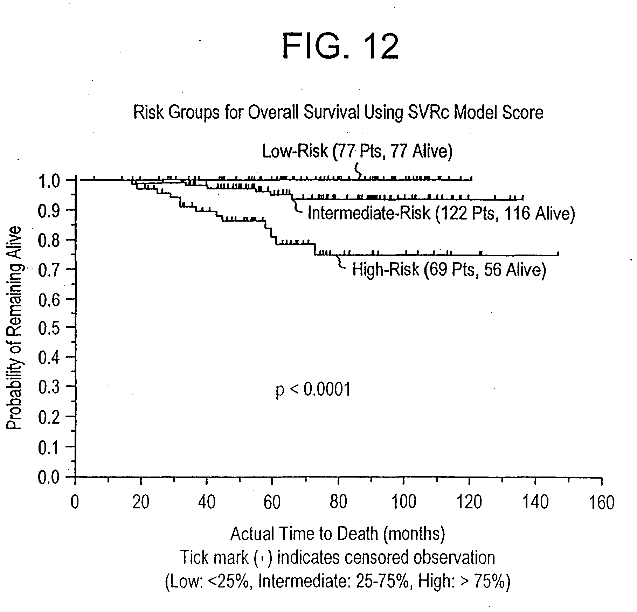

The resulting output of the SVRc model can also be interpreted as a relative risk estimate of death for an individual patient. Using the quartiles of this score (<25%, >25%-75%, >75%), risk groups of patients were created; the Kaplan-Meier estimates of recurrence for each risk group as predicted by the SVRc model are presented in Figure 12. Using the log-rank test, a significant difference in survival was observed between risk groups (p < 0.0001).

## Discussion of Results (Example 2)

The observed reduction of (composite) selected features from Study 1 (41) to Study 2 (10) while retaining the predictive accuracy of the model emphasized the precision and filtering attributes that were achieved through different machine learning methods. The concordance index of the model that was developed in the 268-patient cohort was 0.87; by comparison, when the Kattan nomogram [20] is applied to this cohort it achieved a concordance index of 0.78. Perhaps more striking is the ability of the above model as discussed in Study 2 to correctly classify patients with early PSA recurrences (within 5 years) with a sensitivity of 80%. By comparison, the Kattan nomogram is able to make the same prediction with a sensitivity of only 54%. This further emphasizes the role that such a predictive test would serve in decision making for early intervention. Finally, the output of the model presented can be used to estimate the likelihood of a patient recurring over time, as opposed to offering a single estimate of the probability of a patient recurring within a given number of years without any indication as to when within that time frame.

In Study 3 the objective was to utilize the existing domain knowledge derived from Study 2 and develop a predictive model for overall survival. The successful end result was the ability to predict with 80% accuracy an individual's overall survival and time to death utilizing a total of 14 combined domain features. Although limited by the small number of events (7% dead from any cause) and absence of a comparable published nomogram, the results further support the use of a systems approach for developing these types of predictive tests.

Additional efforts are underway with respect to expanding this 'overall survival' analysis to include clinical measures of poor outcome (i.e., metastasis and or death due to prostate cancer) utilizing a retrospective multi-institutional population with an independent external validation study. In addition, a 'Systems Pathology' approach recently has been initiated to interrogate diagnostic needle biopsies in order to have an impact on treatment issues prior to surgery.

The foregoing example demonstrates that a 'Systems Pathology' platform has been successfully developed which integrates clinical features, tumor tissue morphometrics and molecular analyses. By using domain expertise and support vector regression for censored data (SVRc), features were selected from the three domains and used to develop a predictive model for PSA recurrence and overall survival. It will be understood that this novel 'Systems Pathology' approach has broad application in the field of personalized medicine as it relates to tumor diagnostics, patient prognostication, and as a tool for predicting response to specific therapeutics.

**Example 3: Prediction of Aggressive Disease Subsequent to Prostatectomy Clinical and Morphometric Data**

This study was undertaken to predict aggressive disease (i.e., clinical failure as demonstrated by a positive bone scan representing metastatic prostate cancer to bone) subsequent to a patient having a prostatectomy. Prior to the present invention, no accurate analytical tools existed for providing such a prediction. As described above, the systems pathology approach of the present invention has been shown to accurately predict PSA recurrence. This study demonstrates that the present invention can also be used to accurately predict distant bone metastasis after prostatectomy.

A cohort of 119 patients who underwent radical prostatectomy was studied incorporating tissue microarrays (TMAs) constructed from prostatectomy specimens. Morphometric (i.e., image analysis) studies were performed using hematoxylin and eosin (H&E) stained tissue sections, and biological determinants were assessed with immunohistochemistry (IHC) utilizing a series of biomarkers selected for their potential biological relevance for prostate cancer progression. A predictive model for clinical failure (i.e., positive bone scan) was derived from a selected set of features through supervised multivariate learning. Patients with complete non-missing data (n=116) in each domain were evaluated with a support vector machine for regression developed to handle censored data (SVRc). Predictive performance of the model was estimated using the concordance index (CI) with generated scores used to define risk groups.

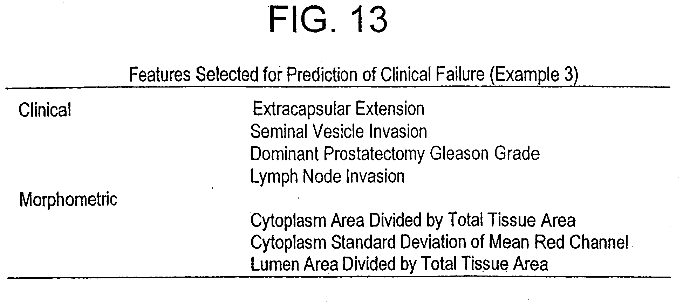

From the 116 patients, a subset of 61 patients was selected based on their clinical features, including 20 individuals with clinical failure as identified by bone metastasis. This cohort was used to create a model for predicting the likelihood of a positive bone scan within 5 years of prostatectomy. The seven features shown in Figure 13 (including four clinical and three morphometric features) were selected which predicted clinical failure with 89 percent accuracy and a sensitivity and specificity of 86 and 85 percent, respectively. The selected morphometric features were related to the pathological objects of cytoplasm and lumen. More particularly, the selected morphometric features were area of cytoplasm divided by the total tissue area, area of lumen divided by total tissue area, and cytoplasm standard deviation of mean red channel. The clinical features are listed below.

**Clinical Features**

1. Extracapsular Extension (ECE)
2. Seminal Vesicle Invasion (SVI)
3. Dominant Prostatectomy Gleason Grade (PGG1)
4. Lymph Node Invasion (LNI)

**Conclusion**

The integration of clinical features with morphometric features resulted in the first, accurate prognostic test for predicting clinical failure within 5 years after prostatectomy. As described, the test can predict with 89% accuracy which patients are most likely to have a clinical failure (and when) within a 5 year period post prostatectomy. The results of adding molecular features to the clinical and morphometric features of the model are currently pending.

**Example 4: Liver Toxicology**

**Morphometric Data**

This study was undertaken to demonstrate image analysis and statistical modeling capabilities in the area of toxicology. Specifically, the study called for the acquisition and analysis of sections of rat liver with the overall objective being to classify the sections as normal or abnormal. Being able to automate this process while simultaneously achieving a high-level of classification accuracy could allow for the creation of a high-throughput platform used to objectively screen for toxicities in preclinical studies.

The study was divided into two phases. The initial phase used a set of 100 rat liver sections as a training set; 80 normal liver sections and 20 abnormal. This set of sections was used to develop an image analysis application using the tissue image analysis system described above as well as perform feature and model selection to classify the sections. The established image analysis process was then applied to an unlabeled set of 100 rat liver sections in the second phase of the study in which the statistical models designed in the training phase were tested.

**Segmentation Accuracy**

The global segmentation accuracy for all objects, as measured by a pathologist's assessment, was 80% - 90%.

**Statistics**

The statistical component of the study involved two steps. The first step involved selecting features from the imaging data generated by the image analysis of the sections. Reducing the number of features used for classification may improve the robustness and reliability of the classification of the sections. The second step involved both training a model using the selected feature set and labels for each section (abnormal, normal) and then testing the model by predicting the classification of an independent set of rat liver sections where the labels were unknown.

**Feature Selection**

The statistical measurements generated for each of the above objects were:

- Number of objects
- Relative area (percent, in relation to total area of image)
- Minimum size (in pixels)
- Maximum size (in pixels)
- Average size (in pixels)
- Standard deviation of the size

Since multiple images were analyzed per section, these measures were themselves averaged across all images for an individual rat liver section. The total number of original features was 378.

Feature selection also involved two steps. The first step utilized domain expertise. A pathologist selected features from the original feature list generated by the image analysis of the sections. The decision to include or exclude features was based on the understanding of the pathology of the liver and potential abnormalities/toxicities that could be encountered. From the original set of 378 features, 90 features were selected using domain knowledge.

These features were then examined using stepwise discriminant analysis to further reduce the number of features for classification. The set of features that made up each class were assumed to be multivariate normal with a common covariance matrix. Features were chosen to enter or leave the model according to the significance level of an F-test from an analysis of covariance, where the features already chosen act as the covariates and the feature under consideration is the dependent variable. A significance level of 0.15 was used.

- Stepwise selection began with no features in the model. At each step, the model was examined.
- If the feature in the model that contributed least to the discriminatory power of the model as measured by Wilks' lambda (the likelihood criterion) failed to meet the criterion to stay, then that feature was removed.
- Otherwise, the feature not in the model that contributed most to the discriminatory power of the model was entered.
- When all features in the model met the criterion to stay and none of the other features met the criterion to enter, the stepwise selection process stopped.

**Classification/Model Training**

The selected features were then entered into a linear discriminant analysis (LDA) which classified each of the liver sections as abnormal or normal. The output of the model was corrected for potential bias via cross-validation.

Neural networks were also explored as a classifier. The selected features were used as the inputs to the neural network model, which is a standard multilayer perceptron (MLP) structure with zero hidden units and direct connection between the input and output layers. The model was trained by trying to directly maximize an approximation to the area under the ROC curve, which is explained below. It was found that the MLP model trained by this criterion achieves better accuracy than an MLP model trained by the typical criteria, *e.g.,* mean square error and cross entropy.

The output from both models were used to create a receiver operating characteristic (ROC) curve by choosing a different value of the model output as a cut point, calculating the sensitivity and specificity for each cut point, and plotting these in a 2-dimensional plot (sensitivity along the y-axis and specificity along the x-axis). The area under the ROC curve (AUC) uses both measures to assess each model's accuracy and can be interpreted as the ability of the model to correctly classify the liver sections as abnormal or normal. Typically, sensitivity and specificity are described in terms of the true positive rate and true negative rate, respectively. Thus in the context of this study, the abnormal class was considered as a 'positive' result, while the normal class was considered as a 'negative' result. Sensitivity, therefore, is the true positive rate, i.e. the proportion of liver sections correctly classified as abnormal; the specificity, on the other hand, is the true negative rate, i.e., the proportion of liver sections correctly classified as normal.

From the ROC curves, selected sensitivities and specificities from the training set are provided in the Results section below.

**Model Testing**

Once developed, the parameters of both the linear discriminant function and the neural network were locked. Upon receipt of the statistical measurements from the test set of rat liver images, both classifiers were applied using an individual cut point estimated using the cross validation results of each of the model outputs respectively. The cut points both corresponded to a sensitivity of 100% and a specificity of 90% (both based on cross validation) for a future industrial-grade application. For the initial evaluation of this external validation set of livers, assessment of the models' accuracies was performed by another party who was unblinded to the true classification of the liver sections. This other party then also provided the test key to verify the results.

**Results**

The area under the ROC curve for both models is very close to 1, indicating almost perfect discrimination between abnormal and normal liver sections. The function derived using LDA has an AUC of 0.99; the function derived using neural networks has an AUC of 0.98.

Also observed in the ROC curves was the sensitivity and specificity of each model, depending on the cut point applied to the model outputs to classify a liver section as abnormal or normal. Table 10 summarizes a selection of sensitivity-specificity pairs.

Table 10

| LDA | | NN | |
|---|---|---|---|
| Specificity | Sensitivity | Specificity | Sensitivity |
| 100% | 65% | 100% | 65% |
| 99% | 75% | 99% | 70% |
| 98% | 100% | | 98% 85% |

Testing

The test key labels were compared with the predicted classifications of the linear discriminant function and those of the neural networks. Based on the key, the results are summarized in Tables 11a and 11b as follows:

Table 11a

**Test Key Label**

|  |  | Abnormal | Normal |  |
|---|---|---|---|---|
| LDA Label | Abnormal | 42 (TP) | 19 (FP) |  |
|  | Normal | 7 (FN) | 32 (TN) |  |
|  |  | 49 | 51 | 100 |

$$\text{Sensitivity} = \text{TP}/(\text{TP}+\text{FN}) \times 100 = 42/(42+7) \times 100 = (42/49) \times 100 = \textbf{86\%}$$

$$\text{Specificity} = \text{TN}/(\text{FP}+\text{TN}) \times 100 = 32/(19+32) \times 100 = (32/51) \times 100 = \textbf{63\%}$$

Table 11b

**Test Key Label**

|  |  | Abnormal | Normal |  |
|---|---|---|---|---|
| NN Label | Abnormal | 36 (TP) | 19 (FP) |  |
|  | Normal | 13 (FN) | 32 (TN) |  |
|  |  | 49 | 51 | 100 |

$$\text{Sensitivity} = \text{TP}/(\text{TP}+\text{FN}) \times 100 = 36/(36+13) \times 100 = (36/49) \times 100 = \textbf{73\%}$$

$$\text{Specificity} = \text{TN}/(\text{FP}+\text{TN}) \times 100 = 32/(19+32) \times 100 = (32/51) \times 100 = \textbf{63\%}$$

The cut point used for the LDA classifier equaled 0.0031; the cut point used for the NN classifier equaled 0.0002. Both correspond to the system requirements of 100% sensitivity and 90% specificity.

**Discussion**

Based on the sensitivity and specificity of each classifier after applying them to the test set, LDA outperformed NN. The LDA classifier achieved a sensitivity of 86% which means that this classifier correctly labeled the abnormal rat liver sections as abnormal 86% of the time, as opposed to the neural network classifier which achieved a sensitivity of 73%. Specificity for both classifiers was 63%. Both the sensitivity and the specificity of each model are lower than previously observed, but this is not surprising as generalizing any classifier to an external set often leads to a drop in its accuracy. This study demonstrated the successful application of technologies for imaging and statistical modeling.

**Example 5: Prediction of Prostate Cancer Recurrence**

**Clinical, Morphometric, and Molecular Data**

Another study was performed to generate a model that predicts time to recurrence of prostate cancer in patients who have undergone radical prostatectomy. As with Example 2, time to recurrence was defined as the time (in months) from radical prostatectomy until PSA (biochemical) recurrence. The sections of prostate tissue used in this study were composed predominantly of tumor but also included benign elements.

This study was based on information for the same 17 clinical features (Table 4) and 43 molecular features (Table 6) evaluated in Example 2. The set of 496 morphometric features (Table 1) were reduced to the 38 features shown in Table 17 (appended hereto) based on, for example, expert knowledge and additional experimentation in the field of prostate cancer recurrence. Clinical, molecular, and morphometric information for 262 patients from the 539 patient cohort described above in connection with Example 2 were evaluated in this study. Other than the filtering that reduced the number of morphometric features from 496 to 38, the main difference between this study and Example 2 is that this study used a SVRc Feature Reduction method for feature selection. SVRc Feature Reduction is described in commonly-owned U.S. Patent Application No. 11/438,789, filed May 22, 2006, which is hereby incorporated by reference herein in its entirety.

## RESULTS

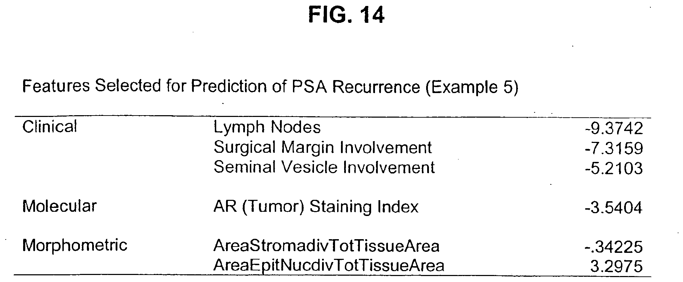

A final model based on 6 features (3 clinical-pathological, 1 molecular, and 2 morphometric) and having a concordance index (CI) of 0.83 was generated as a result of the study. The 6 features included in the model are shown in Figure 14, along with their respective feature contributions to the final model. The three clinical features selected as being predictive of PSA recurrence were seminal vesicle involvement (feature contribution = -5.2103), surgical margin involvement (-7.3159), and lymph node involvement (-9.3742). The one molecular feature selected was the Androgen Receptor (AR) Staining Index present in tumor (-3.5404). These clinical and molecular features are described above in connection with Example 2. The two morphometric features selected were the area occupied by epithelial cell nuclei divided by total tissue area (3.2975) and the area occupied by stroma divided by total tissue area (-.34225). For example, total tissue area may include the sum (in pixels with 1920000 being the maximum where the image size is 1200x1600 pixels) of the areas of cytoplasm, epithelial nuclei, lumen, red blood cells, stromal nuclei, stroma, and artifacts. Possible reasons for the selection of morphometric features related to epithelial nuclei and stromal cells are also described below in connection with the validation study.

The final training model had a sensitivity of 82% and specificity of 81% for correctly predicting prostate cancer recurrence prior to 5 years. The resulting output of the SVRc model can also be interpreted as a relative risk estimate of PSA recurrence for an individual patient. Using the quartiles of this score (<25%, >25%-75%, >75%), risk groups of patients were created, and Kaplan Meier estimates of recurrence for each risk group were generated. The groups showed a statistically significant difference in time to PSA recurrence (log-rank test, p-value < 0.0001).

### Validation Study

The final model was validated with an external cohort consisting of 61 patients. The final model produced a CI of 0.80, sensitivity of 91% and specificity of 70% for the validation for identifying patients at risk for experiencing prostate cancer recurrence within the first 5 years.

To further understand the significance of the two morphometric features selected in the final model, Kaplan-Meier curves were generated for each morphometric feature. It was observed that increasing amounts of stroma (p=0.004) and epithelial nuclei (although not statistically significant, p=0.28) were independently associated with a favorable outcome. This raised the possibility that the quantitative measurements derived from these image patterns may represent more objective determinants of the Gleason grading system.

This study demonstrated that only a limited set of clinical, molecular, and morphometric features may be required to create a clinically useful predictive test. This reduction of features was accomplished while also retaining the predictive accuracy of the model.

### Example 6: Prediction of Prostate Cancer Recurrence

### Clinical, Morphometric, and Molecular Data

Yet another study was performed to generate a model that predicts time to recurrence of prostate cancer in patients who have undergone radical prostatectomy. As in Examples 2 and 5, time to recurrence was defined as the time (in months) from radical prostatectomy until PSA (biochemical) recurrence. The sections of prostate tissue used in this study were composed predominantly of tumor but also included benign elements.

This study was based on information for the same 17 clinical features (Table 4) and 43 molecular features (Table 6) evaluated in Examples 2 and 5. The set of 496 morphometric features (Table 1) were reduced to the 33 features shown in Table 18 (appended hereto). Clinical, molecular, and morphometric information for the same 262 patients from Example 5 were evaluated in this study. This study used the same SVRc Feature Reduction method referenced above in Example 5.

## RESULTS

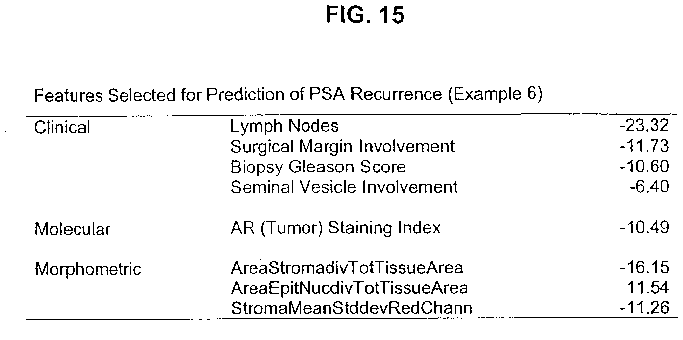

A final model based on 8 features (the same 6 features selected in Example 5 plus 1 additional clinical-pathological feature and 1 additional morphometric feature) and having a concordance index (CI) of 0.86 was generated as a result of the study. The 8 features included in the model are shown in Figure 15, along with their respective feature contributions to the final model. The additional clinical feature selected in this study was biopsy Gleason Score (-10.60), described above in connection with Example 2. The additional morphometric feature selected in this study was the variation in texture within stroma as expressed in the red channel (-11.26). This feature, which indicates variation in stromal texture based on its staining properties, most likely reflects the biochemical attributes of stroma associated with tumor as opposed to benign elements.

## Validation Study

The final model was validated with an external cohort consisting of 366 patients. The final model produced for the validation a CI of 0.82, sensitivity of 96% and specificity of 72% for identifying patients at risk for experiencing prostate cancer recurrence within the first 5 years. Table 12 below shows the observed clinical features for the training and validation cohorts. Tables 13a-c show the observed biomarker-molecular features from the training cohort.

**Table 12.**

| Characteristic | Training | Validation |
|---|---|---|
| N | 262 | 61 |
| Age (years) | | |
| Mean | 62 | 61 |
| Median | 63 | 62 |
| Range | 38-81 | 42-74 |
| Race | | |
| Caucasian | 235 (89.7%) | 58 (95.1%) |
| African-American (Hispanic and Non-Hispanic) | 21 (8.0%) | 2 (3.3%) |
| Other/Unknown | 6(2.3%) | 1 (1.6% |
| Pre-operative PSA (ng/mL) | | |
| Mean | 10.7 | 12.9 |
| Median | 7.8 | 10.0 |
| Range | 0.9-81.9 | 2.0-69.5 |
| Pathologic TNM Stage | | |
| T2N0 | 158 (60.3%) | Not Collected |
| T3aN0 | 70 (26.7%) | Not Collected |
| T3bN0 | 17 (6.5%) | Not Collected |
| T1-3N+ | 17 (6.5%) | Not Collected |
| Missing | 0 | Not Collected |
| UICC Stage | | |
| T1a < 5% | 1 (0.4%) | Not Collected |
| T1b ≥ 5% | 1 (0.4%) | Not Collected |
| T1c not palpable or visible | 113 (43.1%) | Not Collected |
| T2a ≤ ½ lobe | 54 (20.7%) | Not Collected |
| T2b ≤ 1 lobe | 43 (16.4%) | Not Collected |
| T2c both lobes | 33 (12.6%) | Not Collected |
| T3a unilateral ECE | 15 (5.7%) | Not Collected |
| T3c SV+ | 2 (0.8%) | Not Collected |
| Missing | 0 | Not Collected |
| Digital Rectal Exam Result | | |
| Non-palpable | 122 (46.6%) | 32 (52.5%) |
| Palpable | 140 (53.4%) | 29 (47.5%) |
| Missing | 0 | 0 |
| Lymph Node Involvement | | |
| Negative | 246 (93.9%) | 56 (91.8%) |
| Positive | 16 (6.1%) | 5 (8.2%) |
| Missing | 0 | 0 |

(continued)

| Seminal Vesicle Involvement | | |
|---|---|---|
| No | 233 (88.9%) | 51 (83.6%) |
| Yes | 29 (11.1%) | 10(16.4%) |
| Missing | 0 | 0 |
| Surgical Margins | | |
| Negative | 216 (82.4%) | 36 (59.0%) |
| Positive | 46 (17.6%) | 25 (41.0%) |
| Extracapsular Involvement | | |
| No | 159 (60.7%) | 43 (70.5%) |
| Yes | 103 (39.3%) | 18 (29.5%) |
| Missing | 0 | 0 |
| Dominant Biopsy Gleason Grade | | |
| 1 | 1 (0.4%) | 0 (0.0%) |
| 2 | 43 (16.4%) | 0 (0.0%) |
| 3 | 181 (69.1%) | 39 (63.9%) |
| 4 | 36 (13.7%) | 22 (36.1%) |
| 5 | 1 (0.4%) | 0 (0.0%) |
| Missing | 0 | 0 |
| Biopsy Gleason Score | | |
| 2 | 1 (0.4%) | 0 (0.0%) |
| 3 | 0 (0.0%) | 0 (0.0%) |
| 4 | 7 (2.7%) | 0 (0.0%) |
| 5 | 56 (21.4%) | 3 (4.9%) |
| 6 | 97 (37.0%) | 27 (44.3%) |
| 7 | 85 (32.4%) | 20 (32.8%) |
| 8 | 13 (5.0%) | 8 (13.1%) |
| 9 | 3 (1.2%) | 3 (4.9%) |
| Missing | 0 | 0 |
| Dominant Specimen Gleason Grade | 20 (7.6%) | 0 (0.0%) |
| 2 | 198 (75.6%) | 34 (55.7%) |
| 3 | 44 (16.8%) | 23 (37.7%) |
| 4 | 0 (0.0%) | 4 (6.6%) |
| 5 | | |
| Specimen Gleason Score | | |
| 5 | 21 (8.0%) | 1 (1.6%) |
| 6 | 86 (32.8%) | 8 (13.1%) |
| 7 | 144 (55.0%) | 37 (60.7%) |
| 8 | 11 (4.2%) | 7 (11.5%) |
| 9 | 0(0.0%) | 8 (13.1%) |
| Ploidy | | |
| Diploid | 141 (53.8%) | Not Collected |
| Tetraploid | 113 (43.1%) | Not Collected |
| Aneuploid | 8 (3.1%) | Not Collected |
| Missing | 0 (0.0%) | Not Collected |
| Percent Ploidy in S Phase (%) | | |
| Mean | 2.4 | Not Collected |
| Median | 1.2 | Not Collected |

(continued)

| Percent Ploidy in S Phase (%) | | |
|---|---|---|
| Range | 0.0 - 66.4 | Not Collected |
| Percent Ploidy Fraction | | |
| Mean | 3.4 | Not Collected |
| Median | 2.4 | Not Collected |
| Range | 0.0-20.0 | Not Collected |

**Table 13a. Percentage of Cells Staining, by Histologic Component and Staining Intensity (Training Set)**

| Marker | Tumor | | | PIN | | | Atrphic Gland | | |
|---|---|---|---|---|---|---|---|---|---|
| | 1+ | 2+ | 3+ | 1+ | 2+ | 3+ | 1+ | 2+ | 3+ |
| Ki-67 | | | | | | | | | |
| Mean ± SD | 22.0 ± 30.4 | 7.2 ± 17.1 | 1.8 ± 4.0 | 23.0 ± 31.5 | 7.8 ± 18.3 | 2.0 ± 4.5 | 1.3 ± 8.05 | 1.2 ± 9.9 | 0.3 ± 1.6 |
| Median | 0.7 | 0.0 | 0.0 | 1.0 | 0.0 | 0.0 | 0.0 | 0.0 | 0.0 |
| Range | 0.0-100.0 | 0.0-100.0 | 0.0-26.3 | 0.0-100.0 | 0.0-100.0 | 0.0-39.5 | 0.0-96.0 | 0.0-96.5 | 0.0-13.0 |
| CK18 | | | | | | | | | |
| Mean ± SD | NA | NA | 100.0 ± 0.04 | NA | NA | 100.0 ± 0.04 | NA | NA | 100.0 ± 0.00 |
| Median | NA | NA | 100.0 | NA | NA | 100.0 | NA | NA | 100.0 |
| Range | NA | NA | 50.0-100.0 | NA | NA | 50.0-100.0 | NA | NA | 100.0-100.0 |
| CD45 | | | | | | | | | |
| Mean ± SD | NA | NA | 0.0 ± 0.04 | NA | NA | 0.0 ± 0.01 | NA | NA | 0.0 ± 0.00 |
| Median | NA | NA | 0.0 | NA | NA | 0.0 | NA | NA | 0.0 |
| Range | NA | NA | 0.0-0.4 | NA | NA | 0.0-0.1 | NA | NA | 0.0-0.0 |
| CD68 | | | | | | | | | |
| Mean ± SD | NA | NA | 0.0 ± 0.01 | NA | NA | NA | NA | NA | NA |
| Median | NA | NA | 0.0 | NA | NA | NA | NA | NA | NA |
| Range | NA | NA | 0.0-0.1 | NA | NA | NA | NA | NA | NA |

**Table 13b. Percentage of Cells with CD34 Staining, by Histologic Component (Training)**

|  | PIN | Stroma | Tumor | Tumor/PIN | Tumor/Stroma | PIN/Stroma |
|---|---|---|---|---|---|---|
| Mean ± SD | 0.0 ± 0.04 | 0.0 ± 0.11 | 0.1 ± 0.18 | 0.0 ± 0.07 | 0.0 ± 0.08 | 0.0 ± 0.04 |
| Median | 0.0 | 0.0 | 0.0 | 0.0 | 0.0 | 0.0 |
| Range | 0.0-0.4 | 0.0-1.7 | 0.0-0.9 | 0.0-0.5 | 0.0-0.4 | 0.0-0.3 |

**Table 13c. Staining Index by Histologic Component (Training Set)**

| Marker | Tumor | PIN | Gland |
|---|---|---|---|
| **AR** | | | |
| Mean ± SD | 171.8 ± 75.9 | 79.9 ± 83.3 | 29.5 ± 67.9 |
| Median | 200 | 66.0 | 0 |
| Range | 0-300 | 0-300 | 0-300 |
| **CK14** | | | |
| Mean ± SD | 2.2 ± 6.4 | 35.2 ± 62.0 | 8.3 ± 32.6 |
| Median | 0 | 0 | 0 |
| Range | 0-69 | 0-300 | 0-300 |
| **Cyclin D1** | | | |
| Mean ± SD | 1.4 ± 7.1 | 0.0 ± 0.21 | 0.0 ± 0.0 |
| Median | 0 | 0 | 0 |
| Range | 0-90 | 0-3 | 0-0 |
| **PSA** | | | |
| Mean ± SD | 117.9 ± 71.2 | 140.5 ± 97.4 | 22.4 ± 54.9 |
| Median | 100 | 134 | 0 |
| Range | 0-300 | 0-300 | 0-300 |
| **PSMA** | | | |
| Mean ± SD | 0.3 ± 2.1 | 5.8 ± 19.8 | 3.0 ± 23.2 |
| Median | 0 | 0 | 0 |
| Range | 0-21 | 0-154 | 0-300 |
| **p27$^{Kipl}$** | | | |
| Mean ± SD | 3.9 ± 8.2 | 6.6 ± 19.0 | 1.3 ± 8.7 |
| Median | 0 | 0 | 0 |
| Range | 0-48 | 0-140 | 0-120 |
| **Her-2/neu** | | | |
| Mean ± SD | 3.5 ± 16.9 | 0.2 ± 1.1 | 0.0 ± 0.0 |
| Median | 0 | 0 | 0 |
| Range | 0-150 | 0-10 | 0-0 |

**Example 7: Prediction of Clinical Failure**

**Subsequent to Radical Prostatectomy**

**Clinical, Morphometric, and Molecular Data**

This study generated a model for predicting clinical failure in prostate cancer patients who have undergone radical prostatectomy. In this study, clinical failure was defined as the development of metastatic disease and/or androgen-independent disease (e.g., bone scan positive for metastases or a PSA rise while on ADT after surgery). This is in contrast to the clinical failure study described in Example 2, in which clinical failure was defined as death due to any cause.
Clinical information and morphometric information for 345 patients were evaluated in this study. Ten (10) clinical features and 27 morphometric features similar to the clinical and morphometric features shown in Tables 4 and 6 were evaluated. Eleven (11) molecular features were also evaluated. However, these molecular features were generated by a computer from images of tissue subject to immunofluorescence (IF) detection, and not based on IHC as with the molecular features described in Example 2.

More particularly, Alexa fluorochrome labeled antibodies for the androgen receptor (AR), racemase (AMACR), cytokeratin 18 (CK18), TP73L (p63), and high molecular weight keratin were used, along with DAPI, in a 'quint-plex' assay. Based on the distinctive spectral profiles of the fluorochromes, antigen-specific gray-scale images were acquired. Scripts were generated for the Definiens Cellenger product to localize the individual antigens. From antigen distribution and intensity, the scripts identified cell types and cellular compartments (e.g. luminal epithelial cells, epithelial/stromal nuclei) and quantified AR and AMACR in prostate tumor, benign glands, and stroma. Namely, scripts for the Definiens Cellenger product were generated that segmented DAPI and CK18 images into valid nuclei and cytoplasm objects, respectively. These scripts classified an image object as either nuclei or cytoplasm based on an intensity threshold, where the intensity threshold was a linear function of the biomarker gray scale image characteristics (e.g., average intensity, standard deviation, quantiles). Each script/linear function was specific to a given biomarker and was designed using supervised learning (threshold settings by an expert) and linear regression between expert thresholds and image characteristics. The identified cytoplasm objects served as anchor objects for the nuclei split into epithelial and stromal objects. AR and AMACR biomarkers were also segmented using spatial and intensity co-localizations. The spatial localization identified a biomarker within specified compartment: epithelial and stromal nuclei for the AR and epithelial cells (cytoplasm) for the AMACR. The threshold function similar to those used for DAPI and CK18 allowed further classification of the biomarker signal within a compartment as either valid or noise. Patients were excluded if their cores were missing from the array or if the sample contained only stroma.

Because the majority of patients had multiple cores from which IF features were extracted, a procedure was devised for aggregating the feature values across multiple cores, for each patient and feature. Four candidate functions (min, max, median, and mean) were considered (e.g., if a patient had 3 cores, the function min returns the lowest value among the 3 cores). For a given feature, each of these functions was applied to aggregate the core values of each patient in the training set; then the concordance index for each aggregation function was calculated to evaluate it as a predictor of clinical failure. The best aggregating function for a feature was considered to be the one whose concordance index was farthest from random (0.5). Table 14 below lists the 11 IF features and the corresponding selected aggregating functions, and Table 15 describes the features. The aggregating function selected was then used to generate a single value for each IF feature for each patient.

**Table 14.**

| IF feature | Selected aggregating function |
|---|---|
| EpitarposamaMeanMeanChannel30017 | mean |
| EpitarposamaMeanMeanChannel30021 | median |
| Mamacrpostotalarea0045 | max |
| Mcytoplasmamacrpostotalarea0050 | max |
| SumMen02_10totalarea | not applicable; this is a derived feature whose value is the average of the aggregated values of 10 primary features |
| Mepithnucleiarposamacrpostot0085 | max |
| RelAEpithNucARpos | median |
| RelAEpithNucARposAMACRpos | max |
| MeanEpithNucARPosIntensity | min |
| MeanEpithNucARPosAMACRPosIntens | max |
| NormSimpleAverageTotalEpithLum | min |

**Table 15**

| Feature | Description |
|---|---|
| EpitarposamaMeanMeanChannel30017 | Mean intensity of the DAPI objects classified as Epithelial Nuclei positively expressed with AR biomarker and located in the cytoplasm negative with respect to the AMACR biomarker |
| EpitarposamaMeanMeanChannel30021 | Mean intensity of the DAPI objects classified as Epithelial Nuclei negatively expressed with AR biomarker and located in the cytoplasm positive with respect to the AMACR biomarker |

(continued)

| Feature | Description |
|---|---|
| Mamacrpostotalarea0045 | Total area of the AMACR objects with the intensity above noise/signal threshold |
| Mcytoplasmamacrpostotalarea0050 | Total area of the cytoplasm (CK18) objects positively expressed with AMACR |
| SumMen02_10totalarea | In every DAPI object classified as an epithelial nucleus and positively expressed with AR biomarker the sum of normalized AR object intensity values $\tilde{Y}_k$ is calculated: $$I = \sum_k \tilde{Y}_k$$ Where $\tilde{Y}_k = Y_k/Y.$ is ratio of the actual AR object intensity $Y_k$ and the local (with respect to an AR image) noise vs. signal intensity threshold $Y.$ values. The $I$ values are associated with total amount of the protein detected by the biomarker. The values of $I$ are subject for binning. The epithelial nuclei are classified into $M = 11$ bins. Every bin characterizes a certain amount of the AR in the epithelial nuclei. The feature is area of the epithelial nuclei with $20 \leq I < 30$. The area values are divided on to total area of the epithelial nuclei detected on the image |
| Mepithnucleiarposamacrpostot0085 | Total area of the epithelial nuclei positively expressed with AR and positive with respect to the AMACR |
| RelAEpithNucARpos | Relative area of the epithelial nuclei with **AR+** with respect to the total epithelial nuclei area |
| RelAEpithNucARposAMACRpos | Fraction (relative area) of the **AR+** epithelial nuclei with **AMACR+** with respect to the **AR+** epithelial nuclei |
| MeanEpithNucARPosIntensity | Value of the summed intensity ($I$ values) for the AR objects in epithelial nuclei calculated as average across $I$ bin values. The feature can be interpreted as average amount of the AR expressed in the epithelial nuclei in an image (region of interest) |
| MeanEpithNucARPosAMACRPosIntens | Value of the summed intensity ($I$ values) for the AR objects in the **AR+** and **AMACR+** epithelial nuclei estimated through mean value of the intensity. The feature is proportional to the average amount of the AR expressed in the epithelial nuclei located in the cytoplasm positive with respect to the AMACR marker. |
| NormSimpleAverageTotalEpithLum | Concept of luminance - total light energy emitted by object - was introduced. For every DAPI objects classified as an epithelial nucleus luminance was calculated (mean AR intensity multiplied on object area). The feature is an estimate of the AR amount in the epithelial nuclei calculated via luminance values. |

All the IF features except 'Nuclear AR present within epithelial cells that are AMACR negative' displayed association with clinical failure in univariate analysis based on concordance index (CI $\leq$ 0.4 or CI $\geq$ 0.6).

**RESULTS**

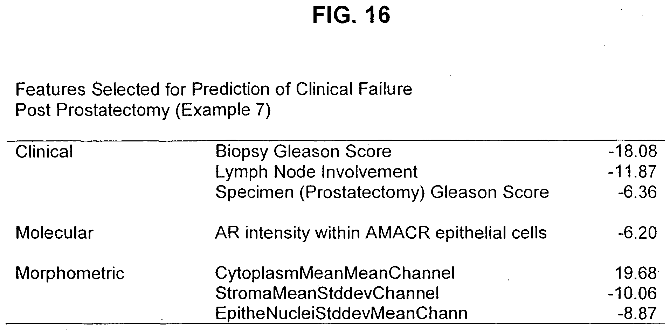

The model resulting from this study was based on 7 features (3 clinical, 1 molecular, and 3 morphometric) and had a concordance index (CI) of 0.91, sensitivity of 95%, and a specificity of 80% on the training cohort. The 7 features included in the model are shown in Figure 16. The 3 clinical features selected as being predictive of clinical failure post-prostatectomy were Biopsy Gleason Score, Lymph Node Involvement, and Specimen (Prostatectomy) Gleason Score. The 3 morphometric features were mean intensity of epithelial cytoplasm as expressed in the blue channel (CytoplasmMeanMeanChannel60060), variation in texture within the stroma as expressed in the Red channel (StromaMeanStddevChannel40310), and variation in texture between epithelial nuclei as expressed in the Red channel (EpitheNucleiStddevMeanChann40157). As described above, the feature related to stromal texture is based on its staining properties and most likely reflects the biochemical attributes of stroma associated with tumor as opposed to benign elements. Additionally, typically when the cytoplasm color changes from light blue to dark blue it reflects changes in the tissue due to cancer development, namely epithelial cell invasion into stroma areas. Regarding texture variations inside epithelial nuclei (folded/unfolded chromatin texture and nucleoli), cancer development is typically characterized by an increased number of the epithelial nuclei with unfolded chromatin texture as well as an increased number of nucleoli, which results in higher values for this feature. The 1 molecular feature selected was AR intensity within epithelial cells that were AMACR positive.

**Validation**

The final model was validated with an independent cohort consisting of 319 patients. The final model produced for the validation a CI of 0.85, sensitivity of 89% and specificity of 77% for predicting clinical failure after prostatectomy. Table 16 below shows the observed clinical features for the training and validation cohorts.

**Table 16. Clinical Information**

| Characteristic | Training | Validation |
|---|---|---|
| Race | | |
| White (Hispanic and Non-Hispanic) | 328 (95.1) | 301 (94.4) |
| African-American (Hispanic and Non-Hispanic) | 11 (3.2) | 9 (2.8) |
| Other/Unknown | 6 (1.7) | 9 (2.8) |
| Pre-operative PSA (ng/ml) | | |
| Mean | 11.4 | 11.1 |
| Median | 7.9 | 8.2 |
| Range | 0.5-100.0 | 1.1-56.2 |
| Clinical TNM Stage | | |
| T1a/b | 4 (1.2) | 4 (1.3) |
| T1c | 172 (49.9) | 148 (46.4) |
| T2a | 64 (18.6) | 59 (18.5) |
| T2b | 29 (8.4) | 26 (8.2) |
| T2c | 67 (19.4) | 77 (24.1) |
| T3 | 9 (2.6) | 5 (1.6) |
| Lymph Node Involvement | | |
| Negative | 332 (96.2) | 308 (96.6) |
| Positive | 13 (3.8) | 11 (3.4) |
| Seminal Vesicle Involvement | | |
| No | 316 (91.6) | 291 (91.2) |
| Yes | 29 (8.4) | 28 (8.8) |
| Surgical Margins | | |
| Negative | 228 (66.1) | 199 (62.4) |
| Positive | 117 (33.9) | 120 (37.6) |
| Extracapsular Involvement | | |
| No | 242 (70.1) | 222 (69.6) |
| Yes | 103 (29.9) | 97 (30.4) |
| Dominant Biopsy Gleason Grade | | |
| 1 | 2 (0.6) | 1 (0.3) |
| 2 | 22 (6.4) | 20 (6.3) |
| 3 | 264 (76.5) | 247 (77.4) |
| 4 | 56 (19.4) | 51 (16.0) |
| 5 | 9 (2.6) | 0 (0.0) |
| Biopsy Gleason Score | | |
| 2 | 1 (0.3) | 0 (0.0) |
| 3 | 2 (0.6) | 1 (0.3) |
| 4 | 7 (2.0) | 12 (3.8) |
| 5 | 28 (8.1) | 27 (8.5) |

(continued)

| Biopsy Gleason Score | | |
|---|---|---|
| 6 | 186 (53.9) | 159 (49.8) |
| 7 | 93 (27.0) | 97 (30.4) |
| 8 | 23 (6.7) | 20 (6.3) |
| 9 | 5 (1.5) | 3 (0.9) |
| 10 | 0 (0.0) | 0 (0.0) |
| Dominant Specimen Gleason Grade | | |
| 2 | 9 (2.6) | 8 (2.5) |
| 3 | 269 (78.0) | 243 (76.2) |
| 4 | 62 (18.0) | 63 (19.8) |
| 5 | 5 (1.5) | 5 (1.6) |
| Specimen Gleason Score | | |
| 5 | 16 (4.6) | 16 (5.0) |
| 6 | 112 (32.5) | 102 (32.0) |
| 7 | 182 (52.8) | 167 (52.4) |
| 8 | 22 (6.4) | 18 (5.6) |
| 9 | 12 (3.5) | 16 (5.0) |
| 10 | 1 (0.3) | 0 (0.0) |

In another aspect, based on an evaluation of a subset of patients who were treated with ADT post-prostatectomy, it has been determined that increasing levels of AR may be associated with a shortened time to clinical failure post treatment with ADT. Thus, measurement(s) of AR content in the prostatectomy specimen could be used to predict response to an androgen suppression type of therapy. To summarize, AR content at the time of the prostatectomy specimen and prior to any form of treatment may be used to predict not only progression of disease but also potentially response to treatment.

### Example 8: Prediction of Prostate Cancer Recurrence

### Clinical, Morphometric, and Molecular Data

Another study was performed to generate a model that predicts time to recurrence of prostate cancer in patients who have undergone radical prostatectomy. As in Examples 2, 5, and 6, time to recurrence was defined as the time (in months) from radical prostatectomy until PSA (biochemical) recurrence.

An expanded cohort of 682 patients was evaluated. This study used clinical features comparable to those used in Example 6 (*see* Table 19). 101 patients (14.8%) experienced PSA recurrence, defined as two consecutive PSA measurements >0.2 ng/mL. Median follow-up of patients with no recurrence observed was 74 months. The overall median time to recurrence was not reached. The same morphometric features shown in Table 18 were evaluated, although in this study Adobe Imageready 7.0 was used to digitally outline and color-mask infiltrative tumor only regions within individual cores for subsequent image segmentation. The molecular features were generated through quantitative multiplex immunofluorescence (IF), generally as described above in connection with Example 7. Additional details regarding IF quantitation are provided below.

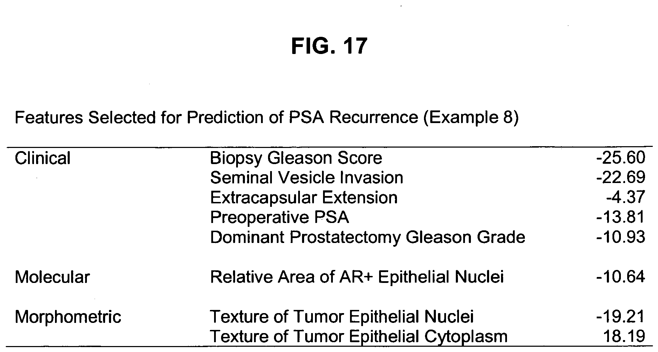

The model again predicted PSA recurrence with high accuracy, the concordance index being 0.77, sensitivity 72% and specificity 74%. As shown in FIG. 17, one molecular feature was selected corresponding to the relative area of AR+ epithelial nuclei. Five clinical features, including seminal vesicle invasion, biopsy Gleason score, extracapsular extension, preoperative PSA, and dominant prostatectomy Gleason grade, as well as two morphometric features, texture of epithelial nuclei and texture of cytoplasm in tumor only regions, were also selected. In FIG. 17, a negative weight indicates that the presence of each feature (or higher value of a continuous feature) was associated with a shorter time to PSA recurrence, whereas a positive weight indicates the opposite. These weights illustrate the respective contribution of each feature in the complete model.

The morphometric features selected for inclusion in the model describe textural properties of the epithelial nuclei and cytoplasm regions using color variations (standard deviation of intensity). Biological processes associated with cancer progression such as unfolded chromatin texture, and appearance of nucleoli in the epithelial nuclei, result in changes in texture of nuclear image objects. The latter increases the value of color standard deviation. Textural properties of cytoplasm areas are due to structural changes in the tissue, and invasion of epithelial cells into stroma when tissue progresses from benign to malignant stages.

Quantitative Immunofluorescence (IF)

The features quantified by the immunofluorescent image analysis in this study are shown and described in Tables 14 and 15 (*see* Example 7). Of note, a feature such as the intensity of AR is associated with concentration of the antigen and was expressed using 12 bits, as opposed to 8 bits for (0-255), RGB- (red,green,blue) limited value, thus allowing for an expanded dynamic range. Among the measurements for AR, one feature, the amount of AR in epithelial cell nuclei (including tumor and non-tumor elements) relative to the total DAPI area of all epithelial nuclei, was highly concordant with the IHC staining index for AR in tumor (Spearman rank correlation coefficient=0.44; p=0.0011). This feature was also independently associated with PSA recurrence when analyzed univariately. Therefore, the quantitative immunofluorescent data support AR IHC biomarker results, and provide a more objective means of assigning a value to AR expression in cellular compartments.

High resolution gray-scale tiff images (1280 x 1024 pixels) were individually processed with an object-oriented method which identified the intensity level for AR within epithelial and stromal nuclei in TMA cores. Tumor elements were not distinguished from normal elements; rather, only stromal and epithelial cells were distinguished. CK18 expression served as the topographical marker for all prostate epithelial cells. Individual 'morphologic' layers were overlaid, i.e. DAPI for nuclei, CK18 for epithelial cells and AR, and, by employing discrete Alexa fluorochromes and preestablished thresholds to remove background and non-specific binding, AR intensity was accurately defined. Additional description regarding establishing thresholds for automated localization and quantification of protein multiplexes via multiplex fluorescence imaging are provided below. The analysis equated protein concentration to absolute positive pixel counts with individual features reflecting a series of morphologic correlates, including nuclear (DAPI-stained) area occupied by AR and the relative amount of AR protein present within epithelial and stromal nuclei. AMACR positive epithelial cells were used as an additional descriptor for AR content and derived features were assessed as part of the overall model development.

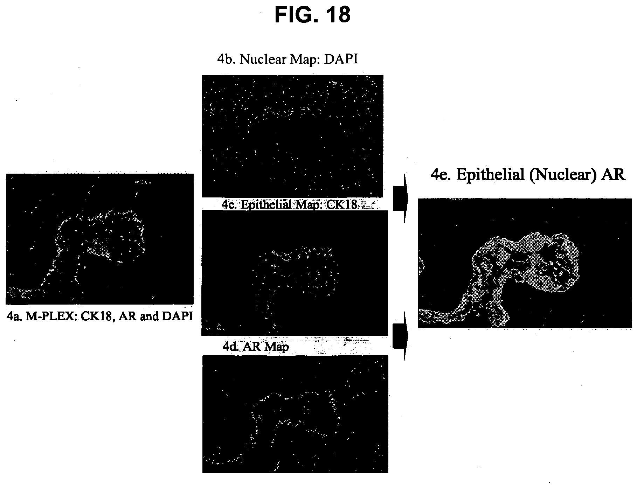

FIGS. 18a-e illustrate multiplex image segmentation for quantifying nuclear AR in prostate tissue. Figure 18a is a standard multi-fluorochrome image that was deconstructed to produce 3 gray-scale images (FIGS. 18b-d) representing discrete morphologic (i.e. DAPI, CK18) and AR maps. Figure 18e is a segmented image representing the nuclear AR within epithelial cells quantified by the script. All of these figures are shown in black and white for reproducibility. More specifically, FIG. 18a is a multiplex immunofluorescent image of prostate tissue stained with DAPI for nuclei (not shown), CK18 labeled with Alexa 488 for epithelial cells (typically shown in green) and AR labeled with Alexa 568 in stroma and epithelial cells (typically shown in red). By applying spectral optics to separate the respective fluorochromes, individual gray-scale images were created for nuclei (FIG. 18b), epithelial cells (FIG. 18c), and AR (FIG. 18d). Using methods based on pixel and object classification, a composite color image was rendered (FIG. 18e; shown here in black and white), which segments the AR present within DAPI-stained nuclei that are within CK18-positive epithelial cells as well as the AR that is present within nuclei that are CK18 negative (stroma). The segmented image was the basis for quantifying the AR in epithelial nuclei as well as the AR within the stroma. Parameters calculated included overall nuclear area involved with AR, mean and maximum intensity, and distribution (% positive cells).

Feature Selection

In the expanded study of Example 8, a SVRc Bootstrap Feature Selection method was employed. An initial filtering step removed features that did not univariately correlate with the outcome of interest. Next, N different splits (in this study, N=25) were made of the training data; in each split approximately two-thirds of that total training instances was randomly assigned to a training subset and approximately one-third of the total training instances was randomly assigned to a testing subset.

The method began with a "greedy-forward" feature selection process starting with all the features that passed the initial filter. For each feature, N models were built on the training subsets across all the splits, and validated on the N respective testing subsets. The overall performance for each feature was averaged across the N runs. The feature with the best overall performance was selected. In the next step, each feature was added to the previously selected feature(s) and again N models were built and tested across the splits. The feature whose addition resulted in the best overall performance was selected. The method continued in this fashion until there were no more features that would improve the performance.

Subsequently, a "greedy-backward" feature selection approach was employed. Each feature was removed, and N models without that feature across the splits were built and tested. The feature whose removal resulted in the best overall performance was removed, and the procedure was repeated until the model performance ceased to improve due to the removal of features. This step reduced model complexity and removed features that may have initially been significant, but their information contribution was encapsulated within a feature added subsequently.

Finally, the complete SVRc model was trained using all the selected features on the complete training data.

In other embodiments, models may be provided for predicting the probability of prostate cancer progression to bone metastasis and/or PSA rise after androgen deprivation therapy, either at the time of diagnostic needle biopsy or after radical prostatectomy.

**Example 9: Prediction of Clinical Failure**

**Subsequent to Radical Prostatectomy**

**Clinical, Morphometric, and Molecular Data**

Another study was conducted to generate a model for predicting clinical failure (CF) in prostate cancer patients who have undergone radical prostatectomy. As with Example 7, clinical failure was defined as the development of metastatic disease and/or androgen-independent disease.

A training set of 373 patients with 32 clinical failure events was evaluated to generate the model through supervised multivariate machine learning (SVRc with bootstrap feature selection). The model had a CI of 0.92, sensitivity of 90%, and specificity of 91% for predicting clinical failure within 5 years after prostatectomy. The model was validated on an independent cohort of 385 patients with 29 clinical failure events, and yielded a CI of 0.84, sensitivity of 84%, and specificity of 85%. High levels of androgen receptor predicted shorter time to castrate PSA rise after androgen deprivation therapy.

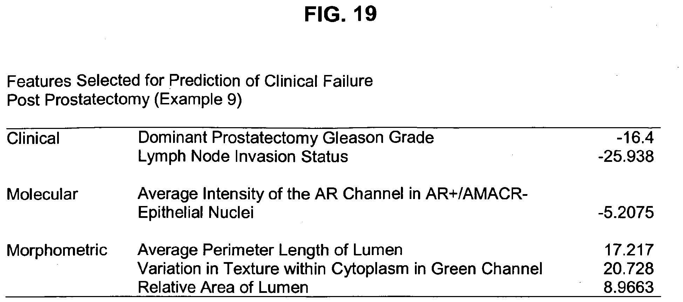

As shown in FIG. 19, the model includes the clinical features of dominant prostatectomy Gleason grade and lymph node invasion status, and the morphometric features of average perimeter length of lumen, relative area of lumen, and variation in texture within cytoplasm in the green channel. The model also includes the molecular feature of average intensity of the AR channel in AR+/AMACR- epithelial nuclei, which was generated by quantitative multiplex IF. In FIG. 19, a negative weight indicates that the presence of each feature (or higher value of a continuous feature) was associated with a shorter time to clinical failure, whereas a positive weight indicates the opposite. These weights illustrate the respective contribution of each variable in the complete model.

In the study, patients who received treatment either before prostatectomy or immediately after but before biochemical recurrence were excluded, leaving 881 patients in the full cohort. The cohort was randomized and evenly split between training and validation sets with similar numbers of clinical failure events. Clinical failure was prespecified as unequivocal radiographic or pathologic evidence of metastasis (including skeletal disease or soft tissue disease in lymph nodes or solid organs) or a rising PSA in a castrate state, or death attributed to prostate cancer. The time to CF was defined as the time from radical prostatectomy to the first of these events. If a patient did not experience CF as of his last visit, or the patient outcome at the time of his most recent visit was unknown, then the patient's outcome was considered censored.

Only patients with complete clinical, morphometric, and molecular data, as well as non-missing outcome information, were further studied. As a result, the number of evaluable patients was 373 in the training set and 385 in the validation set (*see* Tables 20 and 21). The characteristics of these 758 patients were similar to those of the full cohort of 881 patients. The morphometric features were extracted from images taken from patient cores, generally as described in connection with Example 8 (e.g., digitally masking cores with a labeling tool (Adobe 7.0) to include only tumor elements for quantitation). Similarly, the molecular features were generated using multiplex quantitative IF, generally as described in connection with Example 8.

In the training set of 373 patients, 33 (8%) had CF after prostatectomy: 24 with a positive bone scan prior to possible adjuvant therapy, and 9 with a castrate rise in PSA. All but one of the CF patients had received hormonal therapy. The one patient who did not receive hormonal therapy underwent salvage radiotherapy. In addition, there was one patient who received both radiotherapy and hormonal therapy. These 373 patients were followed for a median of 76 months after prostatectomy; the overall median time to CF was not reached. Nine clinical features were included as variables for selection by the model (Table 20). In the validation cohort consisting of 385 patients with a median follow-up of 72 months, twenty-nine (7.5%) of the patients had CF, 22 with a positive bone scan prior to possible adjuvant therapy, and 7 with a castrate rise in PSA. All CF patients had received hormonal therapy and 7 also received salvage radiotherapy. The 6-feature model was applied with the outcome data blinded.

From tumor masked digitized images of each patient's H&E-stained tumor cores, 27 morphometric features were generated (Table 22), reflecting the color and texture of the staining as well as the overall area of the epithelial and stromal cells in the prostate tissue sample. In univariate analysis, 12 features displayed an association with CF based on the concordance index of each feature (CI≤0.4 or CI≥0.6) (Table 20).

AR and AMACR in specific cell types (epithelial tumor cells) were quantified to generate molecular features by utilizing spectral imaging combined with simultaneous multiple antigen assessment and image analysis. Of the immunofluorescence features related to levels of AR and AMACR, eleven of the immunofluorescence features displayed association with CF in univariate analysis (CI ≤ 0.4 or CI ≥ 0.6) and were included in the model for selection (Table 20).

Regarding the final model, noteworthy is the role identified for tumor differentiation (i.e. Gleason grade) and evidence of metastasis (i.e. lymph node involvement) in predicting prostate cancer progression. The three morphometric features of the model illustrate the importance of histochemical staining as a surrogate for biochemical or metabolic properties within the different cell types and overall tissue architecture associated with Gleason grade. Pathologists have traditionally acknowledged the importance of color when distinguishing benign from reactive or malignant cell types, but have been unable to standardize and objectively quantify it. With the current model, the textural color properties of the epithelial cytoplasm were more strongly associated with CF than was the prostatectomy Gleason grade, suggesting the importance of cellular biochemical properties in progression of disease. Individual Kaplan-Meier curves for the three imaging features illustrated the ability to accurately stratify patients. Regarding molecular features, only the normalized average brightness/intensity of AR within AMACR-negative epithelial cells was selected for inclusion in the model. The negative weight for this feature (FIG. 19) indicates that an increasing amount of AR was associated with a shorter time to CF. Of note, preoperative PSA and Gleason sum information were not selected for inclusion the model. Through the SVRc analysis, these variables were supplanted by image analysis features that reflect differentiation and color characteristics linked to the biochemical properties of the prostate cancer.

It is believed that the highly accurate predictions of this CF model will allow the development of more informed and appropriate treatment plans. This would include the possibility of administering ADT, which is currently the first line of systemic therapy against advanced prostate cancer, radiation therapy, and/or chemotherapy earlier where there is a high risk of recurrence. In addition, early identification of patients at high risk creates the opportunity to increase surveillance for progression. The model predictions may also be useful in guiding treatment decisions after biochemical recurrence. Some patients with biochemical recurrence may not require immediate intervention, as the interval between biochemical recurrence and clinically significant events is typically years and also quite variable. Furthermore, many men diagnosed with biochemical recurrence today are likely to have an indolent disease course. Knowledge of the probability of aggressive disease, e.g. disease that will progress to CF within 5 years of surgery, may help the patient and his physician decide whether biochemical recurrence should trigger aggressive therapy.

Given the importance of AR in this clinical progression model, a study was conducted to determine if AR content in the prostatectomy specimen could be used to predict time to castrate rise in PSA after androgen deprivation therapy (ADT). Sixty-three of the 881 patients had received ADT without prior CF. Thirty-two of these patients (51%) subsequently had CF. Using outcome data on the 63 patients, it was investigated whether any of the immunofluorescence features associated with CF were also associated with response to ADT. The following four features were significantly ($p < 0.05$ chi-square test) associated with reduced time to castrate PSA rise, all of them relating to increased AR nuclear levels in tumor epithelial cells:

| Feature | P value |
| --- | --- |
| AR intensity in AMACR(-) Epi Cells | 0.0021 |
| AR intensity in AMACR(+) Epi Cells | 0.0003 |
| Mean AR intensity in AMACR(+) Epi Cells | 0.0285 |
| Total AR intensity in all Epi Cells (normalized) | 0.018 |

By using the AR content of the tumor sample, the urologist may be able to predict utility of ADT and even for an individual patient and adjust therapeutic regimens accordingly. Patients with high scores in the current CF model and high levels of AR face a particular dilemma: an elevated probability of clinical progression accompanied by a decreased likelihood of durable response to ADT and possibly to salvage radiotherapy. These patients may be good candidates for therapies, such as histone deacetylase inhibitors, that target AR or its cofactors. Thus, it is believed that accurate quantitation of the androgen receptor will aid in guiding therapeutic decision-making. Such approaches could even improve selection for clinical trials and potentially serve as measures of treatment effect, specifically for therapies, such as histone deacetylase inhibitors, that target the AR or AR co-factors.

**Automated Localization and Quantification of Protein Multiplexes**

**Via Multispectral Fluorescence Imaging**

A system and method have been developed for automated localization and quantification of the expression of protein biomarkers in immunofluorescence (IF) microscopic images. The system and method discriminate biomarker signal from background, where signal may be the expression of any of the many biomarkers or counterstains used in IF. The system and method are based on supervised learning and represent the biomarker intensity threshold as a function of image background characteristics. The utility of the system and method are demonstrated in predicting prostate cancer recurrence in patients undergoing prostatectomy. Specifically, features representing androgen receptor (AR) expression are shown to be statistically significantly associated with poor outcome in univariate analysis. AR features are also shown to be valuable for multivariate recurrence prediction.

Introduction

Protein biomarkers are widely used in histopathology for cancer diagnosis, prognosis, and therapeutic response prediction. They provide information on the expression levels of proteins in the cells, allowing for the detection of particular cell activities associated with the disease state.

One method for protein expression quantitation is IF, where a protein is localized by introducing an antibody labeled with a fluorescent dye into the tissue that binds to the target protein [21]. The stained slide is illuminated under a fluorescence microscope with a light source having a specific wavelength. This excitation light is absorbed by the fluorescent dye causing it to emit light of a longer wavelength. The intensity of the emitted light is a measure of the concentration of the target protein. In multiplexing, the tissue is labeled with several antibodies at the same time. The antibodies are labeled with fluorescent dyes having distinct spectral characteristics. Separation of multiple biomarkers is accomplished via multispectral imaging of the tissue followed by spectral unmixing to obtain images that represent the expression of individual antibodies.

Conventionally, IF images are interpreted by pathologists based on their perceived intensity levels of the objects of interest (e.g., nuclei) in the tissue. This practice is labor-intensive and suffers from intra- and inter-observer variations. The development of automated systems enables a low-cost, objective alternative to visual scoring of IF images.

Quantitation of a biomarker is achieved in two stages. First, a compartment relevant to the biomarker is detected. Then, the signal is separated from the background within the compartment. These tasks are often accomplished via intensity thresholding. Interactive thresholds [22]-[24] are often followed by computer measurements to quantify biomarker expression. Gordon *et al.* [23] identified proteins in single-cell images as sets of contiguous pixels above a threshold three standard deviations from the background. Mode of the image histogram together with interactive thresholding is used for identifying background pixels in the AQUA system [24]. Rao *et al.* [25] utilize manual epithelial nuclei delineation to mark the compartment of interest for quantitation. Limited reproducibility of interactive methods and poor performance of histogram thresholding on images with low signal-to-background ratios are the primary disadvantages of the existing techniques.

What is proposed is a system and method for biomarker localization and quantification addressing the above limitations. The system and method are fully automated and therefore reproducible. The system and method quantify a biomarker signal within its relevant subcellular compartment, which makes the system and method robust against low signal-to-background ratios.

The system and method are based on an approach for discrimination of the biomarker signal from background. Supervised learning is used to model the intensity threshold for signal discrimination as a function of image background characteristics. The utility of the proposed system and method is demonstrated on prostate cancer prognosis, although it will be understood that the system and method have many additional uses.

Multiplex Image Analysis

The proposed system and method are described in connection with the IF multiplex assay described above, which includes the nuclear counterstain 4'-6-diamidino-2-phenylindole (DAPI) along with cytokeratin 18 (CK18), $\alpha$-methylacyl-CoA-racemase (AMACR) and androgen receptor (AR).

Image analysis platform

The quantification system was designed using the Definiens Enterprise Image Intelligence Suite™ [26]. Image_segmentation into valid biological objects (e.g., nuclei) is a multistage process based on *object-oriented image segmentation.* In this paradigm, objects rather than pixels are the smallest units on which image processing operations and feature calculations are performed. For example, when an intensity threshold is applied to an image, object intensities are subjected to the threshold. The intensity of an object is the average intensity of all pixels belonging to that object.

Two methods are utilized to obtain the primitives [26]. The multiresolution segmentation method finds primitives based on color similarity between pixels and object shape regularity. The quadtree segmentation method uses color similarity only. A scale parameter controls the size of the objects in both methods.

Biomarker localization and quantification

In the developed approach, the first step is spatial colocalization of the biomarker within the corresponding_subcellular compartment. For instance, epithelial nuclei and cytoplasm are subcellular compartments for AR and AMACR, respectively. The next step is to discriminate true biomarker signal from background in the corresponding compartment primitives. Background consists of autofluorescence and non-specific binding of the fluorescent dye to the tissue. Objects of the subcellular compartment are classified as positive with respect to the biomarker if they contain a certain amount of the true signal. Biomarker_quantification is the final step of analysis where we measure properties (e.g., area) of the classified objects.

Biomarker Threshold Model

The next stage of IF image analysis is the discrimination of the true biomarker signal from background using an intensity threshold. The threshold $T$ is considered to be a function of biomarker background characteristics. In order to find this function the objects $B$ are split into two nonoverlapping classes: colocalized in a compartment $B_c$ and objects $B_b$ outside of the compartment. The objects $B_b$ are considered as the background which are described by the following features: mean $\mu_b$, standard deviation $\sigma_b$, and $\alpha$ -th percentiles $q_{b,\alpha}$ of the object intensities, where $\alpha$ = 5,10,...,95. Assuming a linear relationship between the threshold T and the background characteristics, we have

$$T = \boldsymbol{\beta}^T \mathbf{X}, \qquad\qquad (1)$$

where $\beta = [\beta_0,\beta_1,...,\beta_p]^T$ $\beta$ are model parameters, and $\mathbf{X} = [1, X_1,...,X_p]^T$ are background features. Note that in (1), $T$ is changes according to the properties of each image.

Multivariate linear regression is used to determine $\beta$. A set of images is selected for training the model. For each image: a) objects $B_c$ and $B_b$ are identified; b) background features are extracted; and c) expert intensity threshold exp $T$ is determined by a pathologist. The training set is used to train the model to predict the expert thresholds.

To avoid model overfitting, redundant and statistically insignificant features are excluded from training. One of two features is redundant if their pairwise correlation coefficient $r$ satisfies $r \geq 0.85$. The feature having the stronger univariate correlation with expert thresholds is used for multivariate regression. Confidence intervals for the regression coefficients are calculated and coefficients whose confidence intervals contain zero are considered statistically insignificant and are eliminated. Parameters $\beta$ are computed with the remaining features.

Subcellular Compartments for Colocalization

Basic subcellular tissue compartments contain true signal originating from the target AR and AMACR antigens. Epithelial nuclei and cytoplasm constitute the basic compartments mentioned above. These are segmented positive objects from the DAPI and CK18 images, respectively. The next step is to split the nuclei objects into epithelial and stroma cells by overlaying them with the cytoplasm.

It is important in cancer recurrence prediction to distinguish epithelial nuclei located in the invasive cancer areas. The latter are recognized as cytoplasm areas containing AMACR expression. Those epithelial nuclei are called AMACR+, as contrary to AMACR- ones.

The *initial* primitive objects are obtained through the color quadtree segmentation of the CK18 and AMACR images. These objects are re-segmented into coarser objects using multiresolution segmentation. Then the threshold (3) from Table 23 is applied to identify the initial *cytoplasm* or *CK18+* objects. Note that the intensity standard deviation $b \sigma$ in (3) is computed over all initial CK18 objects. Once the initial CK18+ objects are identified, neighborhood analysis is used to refine the class labels. Small background objects whose border length with cytoplasm relative to their total border length is 0.6 or more are reclassified as cytoplasm. Small isolated cytoplasm objects are labeled as background.

The primitive objects are overlaid with the DAPI image. Threshold (2) from Table 23 is used to classify primitives in DAPI+ and background objects. This classification is further refined by a neighborhood analysis similar to that described above. The DAPI+ objects are small fragments of real nuclei depicted in the DAPI image.

DAPI+ primitives are merged into nuclei using an iterative method consisting of region growing and object classification. Finally, nuclei objects are classified into epithelial and stroma nuclei based on their colocalization with CK18+ objects.

Positive and Negative Biomarker Expressions

AMACR+ cytoplasm areas are found in two steps: a) overlaying the primitive objects on the AMACR image with the CK18+ areas; b) applying threshold (5) in Table 23 to the colocalized objects. Cytoplasm objects whose intensity exceeds (5) are classified as AMACR+. Epithelial nuclei are additionally labeled as AMACR+ and AMACR- depending on their association with positive and negative cytoplasm, respectively.

Epithelial cells are the basic subcellular compartments for AR expression. Threshold model (4) in Table 23 is applied to AR objects colocalized in the epithelial nuclei. An epithelial nucleus is classified as AR positive (AR+), if the area of its AR+ objects is more than 0.1% of its total area. Otherwise, it is classified as AR negative (AR -).

Biomarker Quantification

Once biomarkers are colocalized and classified, quantitative features representing object-based intensity and area characteristics are generated from the colocalized biomarkers. Representative intensity features include mean and total AR intensity within AMACR positive/negative epithelial cells recognized in an image. Representative area features include the area of AR+ epithelial cells relative to the area of all epithelial cells and fraction of the AMACR+ epithelial cells among all AR+ cells.

Results

The utility of the proposed system for IF image analysis was demonstrated in predicting prostate cancer recurrence for patients who had undergone prostatectomy. Two types of adverse outcome were considered for prediction. One outcome was prostate-specific antigen (PSA) recurrence (PSAR) corresponding to a significant rise in the patient's serum PSA levels. The other outcome was clinical failure (CF) corresponding to significant disease progression as measured by criteria such as distant metastasis.

Tissue microarray cores with at least 80% of their tissue area covered with tumor were obtained for 682 and 758 patients in the PSAR and CF prediction tasks, respectively. The cores were labeled with the DAPI counterstain, and the CK18, AR, and AMACR biomarkers, and were imaged using the CRI Nuance™ multispectral imaging system [27]. For each core, a single 12-bit 1280×1024-pixel grayscale_image was acquired at the emission peak wavelength of the DAPI counterstain. For each of the CK18, AR, and AMACR biomarkers, an image stack was acquired for a range of wavelengths encompassing the emission spectrum of the corresponding fluorescent dye. The resulting image stacks were unmixed using the Nuance™ system to obtain three images, each corresponding to one of the biomarkers.

On a set of 60 multiplex images from the training set, expert thresholds for the DAPI counterstain, and the CK18,_AR and AMACR biomarkers were selected by a certified pathologist and threshold models were trained as described above and shown in Table 23. The models were validated by visual examination of the images with the largest discrepancies between their expert and predicted thresholds. The AR and AMACR biomarkers were quantitated on the images and features representing the area of AR+ epithelial nuclei relative to the total area of epithelial nuclei as well as the intensity of AR+ epithelial nuclei were obtained. Similar features were also computed for AR+ AMACR+ and AR+ AMACR- epithelial nuclei.

The available patients were grouped into training and validation sets of 342 and 340 patients for PSAR prediction and 373 and 385 patients for CF prediction. In PSAR prediction, the relative area of AR+ epithelial nuclei showed_the highest (among all AR features) univariate concordance index (CI) of 0.37 (p-value < 0.001) on the training set. In CF prediction, the intensity of AR+ AMACR+ epithelial nuclei achieved the highest univariate CI of 0.30 (p-value <0.001).

Additionally, multivariate models were trained for the two prediction tasks in the context of the *systems pathology* paradigm. In this paradigm, disparate information from patient's clinical (e.g., age), histological (via image features extracted from hematoxylin and eosin-stained tissue_specimens [28]), and molecular (via features measuring the expression of protein biomarkers) profiles are combined in a supervised learning framework to predict cancer recurrence. The resulting PSAR model is described above in Example 8. The resulting CF model is described above in Example 9.

Accordingly, provided herein is a system and method for the automated localization and quantification of any protein biomarker's expression in IF multiplexed microscopic images. True biomarker signal is discriminated from background by approaching biomarker image thresholding as a supervised learning problem, modeling the threshold as a function of image background characteristics. The application of this system is demonstrated, for example, to the IF multiplex component of a prostate cancer prognostic assay. Features representing the area and intensity of AR expression were shown to have significant univariate correlation. In addition, the novel, reproducible and robust features were selected as informative in competition with other predictive factors in a multivariate environment, thereby validating the utility of the system in practical applications.

**Table 23. Threshold models for the multiplex biomarkers**

| Biomarker | Model | Eq. |
|---|---|---|
| DAPI | $T = 0.86\sigma_b + 1.1q_{b,5}$ -32.0 | (2) |

(continued)

| Biomarker | Model | Eq. |
|---|---|---|
| CK18 | $T = 0.55\,\sigma_b + 220$ | (3) |
| AR | $T = 5.1q_{b,5} + 0.39q_{b,95} + 1100$ | (4) |
| AMACR | $T = 2.1\mu_b - 0.82q_{b,25} + 1800$ | (5) |

**Example 10: Prediction of Survival of Patients with Non-Small Cell Lung Cancer Treated with Gefitinib; Clinical, Morphometric, and Molecular Data**

Another study was performed to generate a model that predicts survival of individuals with non-small cell lung cancer (NSCLC) who have been treated with gefitinib. Prior to the present invention, no accurate analytical tools existed for providing such a prediction. As described above, the systems pathology approach of the present invention has been shown to accurately predict PSA recurrence and clinical failure after prostatectomy. This study demonstrates that the present invention can also be used to accurately predict survival post gefitinib treatment.

The abundant expression of epidermal growth factor receptor (EGFR) in a variety of solid tumors, including non-small-cell lung cancer (NSCLC), has made it an attractive target for selective molecular therapeutics, specifically tyrosine kinase inhibitors such as gefitinib (IRESSA). Although initial results for gefitinib in pretreated patients were promising, in two pivotal Phase III trials of gefitinib in combination with two other agents, patients with NSCLC treated with gefitinib did not demonstrate significantly better overall or progression-free survival compared with the placebo group. Furthermore, in the Phase III, placebo-controlled IRESSA Survival Evaluation in Lung Cancer (ISEL) trial, gefitinib monotherapy was associated with some improvement in overall survival; however, the data did not reach statistical significance. Positive responses to gefitinib have been associated with activating mutations in the ATP pocket of EGFR, as have clinical-demographic features such as race (Asian), gender (female), smoking history (non-smoking), good performance status, and tumor pathology (adenocarcinoma-bronchoalveolar variant). These results have suggested that specific subgroups of patients with NSCLC are most likely to respond to gefitinib. The data, however, has been complicated by a low EGFR mutation frequency (specifically in Caucasian populations; U.S. ~10-15%), which contrasts with the observation that approximately 45-50% of patients treated with gefitinib (in limited studies) have experienced some form of clinical benefit. Another challenge is the lack of comparative data on EGFR mutations among patients randomized to gefitinib versus no treatment and versus chemotherapy. Furthermore, the response rate seems highly dependent upon the demographics of the group being studied (e.g. gender ratios, ethnicity, and detailed smoking history), suggesting that response criteria are more complicated than previously thought. Of importance, recent evidence from the placebo-controlled ISEL trial, suggests that a high EGFR gene copy number was predictive of clinical benefit and survival. These findings illustrate some of the complexity observed in attempts to understand the response criteria.

A cohort of 109 patients was evaluated. Specifically, diagnostic tumor samples from with advanced refractory NSCLC treated with gefitinib were evaluated . Tumor samples were evaluated by EGFR DNA mutation analysis, EGFR immunohistochemistry, histologic morphometry, and quantitative immunofluorescence for 15 markers. A support vector regression mathematical model was utilized to integrate six clinical features (gender, smoking history, age at diagnosis, tumor histology and ECOG performance status) with histology and quantitative biomarker multiplexing.

Patients and Tissues

The initial cohort consisted of 284 United States patients with advanced refractory NSCLC treated with gefitinib 250 mg orally each day. Five clinical features were analyzed: gender, smoking history, age at diagnosis, histology, ECOG performance status,(scale ranges from 0 (healthy) to 5 (death from disease). (Table 24). Unstained slides (including fine needle aspirates, cell pellets, and cytospins) and/or paraffin blocks from the diagnostic specimen were evaluated with hematoxylin and eosin (H&E) for tumor content. All biomarkers were analyzed without knowledge of clinical outcome.

EGFR Tyrosine Kinase Domain Mutation Analysis

Two sequential 20-$\mu$m thick sections from each paraffin block or a minimum of 8 unstained sections from paraffin slides were analyzed. Genomic DNA was obtained from de-paraffinated tissue samples, after incubation with proteinase K and subsequent chloroform extraction and ethanol precipitation. EGFR mutations were analyzed primarily by DNA sequencing of exons 19, 20, and 21, and secondarily using the amplification refractory mutation system (ARMS), specifically, allele-specific polymerase chain reaction (PCR) to detect the exon 21 L858R point mutation and the most common exon 19 deletion (del G2235-A2249). Patients were considered mutation-positive if a mutation in the tyrosine kinase domain was detected by either ARMS or gene sequencing in both forward and reverse directions in at least two independent PCR products from tumor DNA.

Histologic Morphometry

H&E-stained slides were prepared from the original blocks or unstained sections. One to six images from representative areas of tumor were acquired with an Olympus bright-field microscope at 20X magnification using a SPOT Insight QE Color Digital Camera (KAI2000). Image analysis software classified image objects as histopathological cellular elements exhibiting red, green, and blue color channel values, generic shape features (e.g. area, length), and spatial relationship properties (e.g. relative amounts of lumen with respect to total tissue), from which statistics were generated. Due to differences in sample preparation (i.e. cytospin, needle biopsy, and tissue resection) fixation, staining, and tissue quality, several different scripts were developed for image segmentation.

EGFR Immunohistochemistry

EGFR protein was analyzed by immunohistochemistry using the EGFR pharmDX kit (DAKO, Glostrup, Denmark). Samples were considered positive if >10% of tumor cells had staining. A staining index was calculated for each sample by multiplying each intensity level (0-3) by the percentage of cells at that intensity level; the index thus ranges from 0 to 300.

Mutiplex (M-Plex™) Biomarker Assessment

Fifteen antibodies including: Cytokeratin 18, Ki67, Caspase-3 activated, CD34, phosphorylated EGFR, phosphorylated ERK, phosphorylated AKT, PTEN, cyclin D1, phosphorylated mTOR, PI3-K, VEGF, VEGFR-2, and phosphorylated VEGFR-2 were selected and grouped into six multiplex formats. Each antibody represented an assessment of morphologic components (e.g. cytokeratin 18 [CK18], CD34), signaling pathways, or biologic processes of proliferation, apoptosis, and angiogenesis.

Each antibody was evaluated on a series of cell lines and/or control lung cancer tissue samples by immunohistochemistry (IHC). Each antibody was titrated using both immunohistochemical and immunofluorescent standard operating procedures. Antibodies were then advanced through simplex and multiplex immunofluorescent formats. The 15 antibodies were organized into 6 multiplex formats based on antigen retrieval systems, isotype, and prioritization with respect to biological application. These m-plex formats are listed in Table 27.

**Table 27. Antibodies and multiplex immunofluorescence groupings.**

| M-plex Group | Antibody | Vendor | Cat. # | Isotype | Dilution | Fluorescent Label |
|---|---|---|---|---|---|---|
| 1 | Cytokeratin 18 | Vector | VC-C414 | Mouse IgG 1 | 1:250 | 1° - 488 |
| | Cyclin D1 | BioCare Med. | CP236B | Rabbit IgG | 1:100 | 1° - 568 |
| 2 | Cytokeratin 18 | Vector | VC-C414 | Mouse IgG1 | 1:250 | 1° - 488 |
| | Ki-67 | Ventana | 790-2910 | Mouse IgG 1 | 1:75 of pre-diluted | 2° - 555 |
| | PI3 Kinase | Cell Signaling | 3821 | Rabbit IgG | 1:20 | 1° - 568 |
| | Caspase-3 activated | Chemicon | ab3623 | Rabbit IgG | 1:5 | 2° - 594 |
| 3 | Cytokeratin 18 | Vector | VC-C414 | Mouse IgG1 | 1:200 | 1° - 488 |
| | CD-34 | Dako | M7165 | Mouse IgG1 | 1:50 | 2° - 555 |
| | p-mTOR | Cell Signaling | ab2971 | Rabbit IgG | 1:50 | 1° - 568 |
| | pERK | Cell Signaling | ab4376 | Rabbit IgG | 1:5 | 2° - 594 |
| 4 | Cytokeratin 18 | Vector | VC-C414 | Mouse IgG1 | 1:200 | 1° - 488 |

(continued)

| M-plex Group | Antibody | Vendor | Cat. # | Isotype | Dilution | Fluorescent Label |
|---|---|---|---|---|---|---|
| | pAKT S473 | Abeam | ab4802 | Rabbit IgG | 1:20 | 1° - 555 |
| | PTEN | NeoMarkers | MS1797 | Mouse IgG1 | 1:50 | 2° - 568 |
| | KDR | Upstate | 07-158 | Rabbit IgG | 1:450 | 2° - 594 |
| 5 | Cytokeratin 18 | Vector | VC-C414 | Mouse IgG 1 | 1:250 | 1° - 488 |
| | VEGF | Abeam | ab1316 | Mouse IgG1 | 1:50 | 2° - 555 |
| | pKDR | Upstate | 07-374 | Rabbit IgG | 1:150 | 2° - 594 |
| 6 | Cytokeratin 18 | Vector | VC-C414 | Mouse IgG 1 | 1:10 | 2° - 488 |
| | EGFR | Dako | K1492 | Mouse IgG 1 | pre-diluted | 1° - 555 |
| | pEGFR (Y1068) | Abeam | ab5644 | Rabbit IgG | 1:250 | 1° - 594 |

Deparaffinization and rehydration of tissue samples was performed as per standard operating procedures. Antigen retrieval was performed by boiling the slides in a microwave oven for 7.5 minutes in IX Reveal Solution (BioCare Medical). The slides were allowed to cool for 20 minutes at room temperature and then were washed twice for 3 minutes in phosphate-buffered saline (PBS).

The tissue samples underwent the following pre-hybridization treatment steps. To help permeate the cellular structures of the tissue, the samples were incubated in PBT (PBS + 0.2% Triton X-100) at room temperature for 30 minutes, followed by three rinses of 3 minutes each in PBS. To help reduce tissue autofluorescence, the samples were incubated in acid alcohol (1% HCl in 70% ethanol) at room temperature for 20 minutes, followed by three rinses of 3 minutes each in PBS. Blocking of nonspecific binding sites was performed by incubating the slides in IF Blocking Reagent (0.5 mg/ml BSA in PBS) at room temperature for 20 minutes. No washes were performed between the blocking step and the subsequent hybridization step.

A cocktail of one mouse IgG1 antibody and one rabbit IgG antibody was made using previously determined titers in IF Blocking Reagent. Approximately 100 μl of this antibody cocktail was applied to the tissue sample, and the antibodies and tissue samples were allowed to hybridize in a humidity chamber at room temperature for 1 hour. Hybridization was followed by two rinses of 5 minutes each in PBT followed by two rinses of 3 minutes each in PBS.

For the labeling step, a cocktail of the appropriate Zenon Alexa Fluor Rabbit IgG label and of the appropriate Zenon Alexa Fluor Mouse IgG1 label (Invitrogen / Molecular Probes) for the above hybridized antibodies was made in IF Blocking Reagent at the concentrations recommended by the manufacturer (1:50 dilution for each Fab fragment). Approximately 100 μl of this labeling cocktail was applied to the tissue samples, and the tissue samples were incubated in a humidity chamber at room temperature for 30 minutes. The labeling reaction was followed by two rinses. The hybridization and labeling steps as described above were repeated for each subsequent group of antibodies included in the multiplex. Up to two more rounds of antibody hybridization and labeling were possible. It must be noted that to obtain discrimination between differing antibodies, no Zenon Alexa Fluor label, whether rabbit- or mouse-specific, was used more than once.

A fixation step was performed by incubating the samples in 10% formalin at room temperature for 10 minutes, followed by two rinses of 3 minutes each in PBS.
Approximately 25.0 μl of *SlowFade* Gold antifade reagent with DAPI mounting solution was applied to the samples, which were then cover-slipped. Samples were stored at -20°C until analysis could be performed.

Multiplex fluorescent images were acquired using a CRI Nuance multispectral camera (Cambridge Research and Instrumentation, Inc.) mounted on a Nikon 90i automated fluorescence microscope and controlled by MetaMorph on-line software. The resulting images were saved as quantitative grayscale TIFF images (1280 by 1024 pixels). For the selected regions of interest, DAPI nuclear counterstain was recorded at 480 nm wavelength using a bandpass DAPI filter (Chroma). Alexa 488 was captured between 520 and 560 nm in 10-nm intervals using an FITC filter (Chroma). Alexa 555, 568, and 594 were recorded between 570 and 670 nm in 10-nm intervals using a custom-made longpass filter (Chroma), while Alexa 647 was recorded between 640 and 720 nm in 10-nm intervals using a second custom-made longpass filter (Chroma). Representative regions for each dye were allocated in order to create a spectral library for the spectral un-mixing process.

The image analysis scripts overlay morphologic attributes (i.e. DAPI for nuclei, CK18 for epithelial cells), and use the Alexa fluorochrome signature to differentiate individual antibodies while removing background to optimize signal-to-noise ratios. The intensity profiles generate features, including the mean, maximum, and standard deviation for each labeled antibody. Specific relationships of individual markers with their activated forms (e.g. pKDR:KDR, pEGFR:EGFR), and the intensity of a specific marker in cells that express another marker (e.g. phosphorylated mTOR in pERK-positive epithelial cells) are generated through the script feature analysis software.

Antibody Quality Control

To confirm specificity of the pEGFR(Y1068) and pERK antibodies, the A431 NSCLC cell line was treated with epidermal growth factor (EGF). Cells were washed twice with ice-cold PBS and then lysed with lysis buffer (0.5% Triton X-100, 50 mmol/L Tris pH 7.6, 300 mmol/L NaCl, 2 mmol/L $Na_3VO_4$) containing phenylmethylsulfonyl fluoride (1 mmol/L), leupeptin (25 $\mu$g/mL), aprotinin (25 $\mu$g/mL), and pepstatin A (3.5 $\mu$g/mL) for 25 minutes at 4C. Following standard protocols for immunoprecipitation, IP ECL Plus Chemiluminescent western blots were performed with both EGF-activated and non-activated cell lines. Similar tests were performed with pKDR in HUVEC cell lines activated with VEGF. In addition, for both EGF and VEGF, treated and non-treated cells were processed for routine IHC.

Generation of Immunofluorescent Features

Immunofluorescent scripts for each antibody: antigen pair were developed using control lung cancer tissue samples. The scripts were designed according to the expected cellular distribution of the individual proteins and utilize the acquired grayscale image to identify both the area and subsequent fluorescent intensity for each marker. The preliminary scripts generated >400 features (many of them redundant), which were reduced to 84 features for entry in the models.

Statistical Analysis

Time to death was defined as the time from initiation of treatment with gefitinib to date of death or date of last follow-up and was reported in weeks. Patients with no death recorded were censored at the date of last follow-up. A support vector regression for censored data with feature reduction (SVRc-FR) was applied on clinical variables and features generated by M-Plex™, histologic morphometry, and EGFR immunohistochemistry. SVRc is an adaptation of traditional SVR for accommodating censored data. To accomplish this, a modified loss/penalty function was defined that allows processing of right-censored and non-censored data. The SVRc-FR algorithm has an initial filtering step to remove features that are not univariately correlated with the outcome of interest, based on the concordance index (CI). The CI is the probability of predicting, in the correct order, the times for two randomly chosen patients, where both died, or one died before the last follow-up of the censored patient. The CI ranges from 0 to 1, with 0.5 indicating a random association. CI's further from 0.5 thus indicate stronger associations with outcome. Only features with CI <0.4 or >0.6 were retained. The Cox model was used as a comparator for multiple features including those selected by SVRc-FR. Using a stepwise approach, the entry criterion for a feature was a p-value $\leq$ 0.15. To stay in the model, a feature had to have a p-value $\leq$ 0.05.

Results

Of the 284 patients, tissue samples from 109 (38%) had sufficient tumor (>50%) for further analyses. From the 109 patient samples, EGFR mutation results were obtained from 87 while 51 patients had complete data (i.e. clinical, immunohistochemistry and immunoflourescence) for inclusion in a final model. The demographics for the total cohort (284 patients) and final model (51patients) are contained in Table 24.

Table 24. Characteristics of patients in the final predictive model compared with total patient cohort.

| Characteristic | Patients in model 51 patients: N (%) | Full Cohort 284 patients: N (%) |
| --- | --- | --- |
| Performance Status | | |
| 0 | 2 (3.9) | 13 (4.58) |
| 1 | 39 (76.5) | 203 (71.48) |
| 2 | 10 (19.6) | 68 (23.94) |
| Gender | | |
| Female | 22 (43.1) | 126 (44.37) |
| Male | 29 (56.9) | 158 (55.63) |

(continued)

| Histology | | |
|---|---|---|
| Adenocarcinoma | 31 (60.8) | 138 (48.59) |
| Bronchoalveolar | 1 (2.0) | 5 (1.76) |
| Large Cell | 5 (9.8) | 36 (12.68) |
| Mixed | 2 (3.9) | 20 (7.04) |
| NSCL | 5 (9.8) | 30 (10.56) |
| Squamous | 7 (13.7) | 55 (19.37) |
| Smoking History | | |
| No | 8 (15.7) | 45 (15.85) |
| Yes | 43 (84.3) | 239 (84.15) |
| Chemotherapy Regimens | | |
| 0 | 1 (2.0) | 19 (6.69) |
| 1 | 19 (37.3) | 106 (37.32) |
| 2 or 3 | 25 (49.0) | 135 (47.54) |
| 4, 5, or 6 | 6 (11.8) | 24 (8.45) |
| Age | | |
| Mean +/- SD | 60.8 +/- 12.83 | 63.02 +/- 11.29 |
| Median | 62.0 | 64.0 |
| Range | 24.0 - 82.0 | 24.0 - 86.0 |
| Survival | | |
| Deaths | 50 (98.0) | 268 (94.37) |
| Censored | 1 (2.0) | 16 (5.63) |
| Median Survival Time, weeks | 25.0 | 27.0 |

*Note: The mean values for continuous features are comparable*

EGFR Mutation Status

EGFR mutation analysis, based on published sequences for mutations in exons 19-21,4,10 was performed for the 87 patients who had clinical outcome data and sufficient material for DNA analysis. Four patients (5%) had EGFR tyrosine kinase domain mutations in exons 19, 20, or 21. Two of these patients exhibited in-frame deletions in exon19 (delL747-S752insV and deIL747P753insS), one had a somatic mutation (T790M) in exon 20, and the fourth had an L858R mutation in exon 21. All 4 patients with EGFR mutation were female and had been diagnosed with adenocarcinoma. Two of these patients had achieved partial response and 2 progressed (Table 25). The T790M mutation, which was present in a patient who had short survival (4 weeks), was previously shown to be associated with drug resistance. The median overall survival for all patients analyzed for mutations was 24 weeks, and the 1-year overall survival was 26%. Because of the low frequency of EGFR mutations, mutation status was not used as a variable in subsequent model development.

Table 25. Response Status for Patients with EGFR Mutations.

| Patient Number | Exon | Mutation | Objective Response | Time to Death, wks |
|---|---|---|---|---|
| 46 | 19 | del L747-S752insV | Partial response | 65 |
| 127B | 19 | Del L747-P753insS | Partial response | 92 |
| 69 | 21 | L858R | Progression | 22 |
| 196 | 20 | T790M | Progression | 4 |

EGFR Immunohistochemistry

Immunohistochemistry was performed on 60 patient samples for which >50% tumor was present. In brief, EGFR protein heterogeneity in tumor only regions was evaluated, with 14 samples (23%) exhibiting a staining index ≥200 suggestive of increased expression, while 20 samples (30%) had a staining index of 0. The tumor staining index was the only variable included in the predictive model. Immunohistochemistry of EGFR protein was analyzed using % of positive cells multiplied by intensity (0-3+) to generate a staining index (H score) on patients with sample available for analysis (Table 28).

Table 28. Detailed results from EGFR immunohistochemistry.

| Protocol Number DM05010 | | | | | |
|---|---|---|---|---|---|
| DM Slide No. | Total positive cells, % | + cells, % | ++ cells, % | +++ cells, % | H score* |
| 1 | 95 | 25 | 35 | 35 | 200 |
| 2 | 80 | 30 | 30 | 20 | 150 |
| 3 | 100 | 0 | 10 | 90 | 290 |
| 4 | 10 | 10 | 0 | 0 | 10 |
| 5 | 10 | 5 | 5 | 0 | 15 |
| 6 | 0 | 0 | 0 | 0 | 0 |
| 7 | 80 | 10 | 40 | 20 | 150 |
| 8 | 60 | 20 | 40 | 0 | 100 |
| 9 | 0 | 0 | 0 | 0 | 0 |
| 10 | 100 | 0 | 20 | 80 | 280 |
| 11 | 75 | 35 | 25 | 15 | 130 |
| 12 | 100 | 0 | 10 | 90 | 290 |
| 13 | 30 | 20 | 10 | 0 | 40 |
| 14 | 40 | 10 | 20 | 10 | 80 |
| 15 | 100 | 10 | 40 | 50 | 240 |
| 16 | 0 | 0 | 0 | 0 | 0 |
| 17 | 0 | 0 | 0 | 0 | 0 |
| 18 | 90 | 10 | 40 | 40 | 210 |
| 19 | 40 | 15 | 15 | 10 | 75 |
| 20 | 90 | 20 | 30 | 40 | 200 |
| 21 | 40 | 10 | 20 | 10 | 80 |
| 22 | 0 | 0 | 0 | 0 | 0 |
| 23 | 0 | 0 | 0 | 0 | 0 |
| 24 | 0 | 0 | 0 | 0 | 0 |
| 25 | 100 | 0 | 0 | 100 | 300 |
| 26 | 0 | 0 | 0 | 0 | 0 |
| 27 | 90 | 20 | 20 | 50 | 210 |
| 28 | 40 | 20 | 10 | 10 | 70 |
| 29 | 0 | 0 | 0 | 0 | 0 |
| 30 | 0 | 0 | 0 | 0 | 0 |
| 31 | 100 | 0 | 0 | 100 | 300 |
| 32 | 90 | 10 | 10 | 70 | 240 |
| 33 | 30 | 10 | 20 | 0 | 50 |
| 34 | 0 | 0 | 0 | 0 | 0 |
| 35 | 70 | 20 | 50 | 0 | 120 |
| 36 | 0 | 0 | 0 | 0 | 0 |
| 37 | 50 | 20 | 20 | 10 | 90 |
| 38 | 0 | 0 | 0 | 0 | 0 |
| 39 | 20 | 20 | 0 | 0 | 20 |

(continued)

| Protocol Number DM05010 | | | | | |
|---|---|---|---|---|---|
| DM Slide No. | Total positive cells, % | + cells, % | ++ cells, % | +++ cells, % | H score* |
| 40 | 0 | 0 | 0 | 0 | 0 |
| 41 | 30 | 10 | 10 | 10 | 60 |
| 42 | 30 | 10 | 10 | 10 | 60 |
| 43 | 90 | 20 | 60 | 10 | 170 |
| 44 | 0 | 0 | 0 | 0 | 0 |
| 45 | 40 | 30 | 10 | 0 | 50 |
| 46 | 0 | 0 | 0 | 0 | 0 |
| 47 | 10 | 10 | 0 | 0 | 10 |
| DM Slide No. | Total positive cells, % | + cells, % | ++ cell, % | +++ cells, % | H score* |
| 48 | 0 | 0 | 0 | 0 | 0 |
| 49 | 90 | 10 | 10 | 70 | 240 |
| 50 | 70 | 30 | 30 | 10 | 120 |
| 51 | 70 | 10 | 40 | 20 | 150 |
| 52 | 90 | 10 | 10 | 70 | 240 |
| 53 | 10 | 10 | 0 | 0 | 10 |
| 54 | 60 | 50 | 10 | 0 | 70 |
| 55 | 0 | 0 | 0 | 0 | 0 |
| 56 | 30 | 10 | 10 | 10 | 60 |
| 57 | 0 | 0 | 0 | 0 | 0 |
| 58 | 0 | 0 | 0 | 0 | 0 |
| 59 | 90 | 10 | 20 | 60 | 230 |
| 60 | 10 | 0 | 10 | 0 | 20 |
| 61 | 80 | 10 | 60 | 10 | 160 |
| *H Score = (% cells scored as +++) x 3 + (% cells ++) x 2 + (% cells +) x 1. Maximum H score is 300. | | | | | |

H&E Image Analysis

Selected digitized images from 109 H&E-stained patient samples were processed with imaging software. The software segments and classifies individual tissue - cellular elements, segregating epithelial nuclei, epithelial cytoplasm, stroma as well as alveolar lumens. Elements are classified using spectral 'color' characteristics, shape, and spatial relationships between tissue objects. Lumen, for example, is classified from the overall association of white space surrounded by alveolar 'tumor' epithelial cells. Thirty-nine imaging features were generated and entered into the predictive models.

Table 29 lists the 39 H&E features extracted from the individual patient samples, including whole sections, needle biopsies, and aspirates.

Table 29. Histologic morphometry features.

| Feature Name | Description |
|---|---|
| CytoplasmMeanMeanChannel40058 | Mean of all cytoplasm objects' pixel value, red channel |
| CytoplasmMeanMeanChannel50059 | Mean of all cytoplasm objects' pixel value, green channel |
| CytoplasmMeanMeanChannel60060 | Mean of all cytoplasm objects' pixel value, blue channel |
| CytoplasmMeanStddevChannel40066 | Mean of all cytoplasm objects' pixel value std, red channel |
| CytoplasmMeanStddevChannel50067 | Mean of all cytoplasm objects' pixel value std, green channel |
| CytoplasmMeanStddevChannel60068 | Mean of all cytoplasm objects' pixel value std, blue channel |

(continued)

| Feature Name | Description |
|---|---|
| CytoplasmStddevMeanChannel40081 | SD of all cytoplasm objects' pixel value mean, red channel |
| CytoplasmStddevMeanChannel50082 | SD of all cytoplasm objects' pixel value mean, green channel |
| CytoplasmStddevMeanChannel60083 | SD of all cytoplasm objects' pixel value mean, blue channel |
| EpithelialNucleiMeanAreaPx10093 | Mean of all epithelial nuclei objects' area |
| EpithelNucleiMeanMeanChanne40104 | Mean of all epithelial nuclei objects' pixel value mean, red channel |
| EpithelNucleiMeanMeanChanne50105 | Mean of all epithelial nuclei objects' pixel value mean, green channel |
| EpithelNucleiMeanMeanChanne60106 | Mean of all epithelial nuclei objects' pixel value mean, blue channel |
| EpitheNucleiMeanStddevChann40112 | Mean of all epithelial nuclei objects' pixel value SD, red channel |
| EpitheNucleiMeanStddevChann50113 | Mean of all epithelial nuclei objects' pixel value SD, green channel |
| EpitheNucleiMeanStddevChann60114 | Mean of all epithelial nuclei objects' pixel value SD, blue channel |
| EpitheliaNucleiStddevAreaPx10116 | SD of all epithelial nuclei objects' area |
| EpitheNucleiStddevMeanChann40127 | SD of all epithelial nuclei objects' pixel value mean, red channel |
| EpitheNucleiStddevMeanChann50128 | SD of all epithelial nuclei objects' pixel value mean, green channel |
| EpitheNucleiStddevMeanChann60129 | SD of all epithelial nuclei objects' pixel value mean, blue channel |
| StromaNucleiMeanAreaPx10300 | Mean of all stroma nuclei objects' area |
| StromaNucleiMeanMeanChannel40311 | Mean of all stroma nuclei objects' pixel value mean, red channel |
| StromaNucleiMeanMeanChannel50312 | Mean of all stroma nuclei objects' pixel value mean, green channel |
| StromaNucleiMeanMeanChannel60313 | Mean of all stroma nuclei objects' pixel value mean, blue channel |
| StromaNucleiMeanStddevChann40319 | Mean of all stroma nuclei objects' pixel value std, red channel |
| StromaNucleiMeanStddevChann50320 | Mean of all stroma nuclei objects' pixel value SD, green channel |
| StromaNucleiMeanStddevChann60321 | Mean of all stroma nuclei objects' pixel value SD, blue channel |
| StromaNucleiStddevAreaPx10323 | SD of all stroma nuclei objects' area |
| StromaNucleiStddevMeanChann40334 | SD of all stroma nuclei objects' pixel value mean, red channel |
| StromaNucleiStddevMeanChann50335 | SD of all stroma nuclei objects' pixel value mean, green channel |
| StromaNucleiStddevMeanChann60336 | SD of all stroma nuclei objects' pixel value mean, blue channel |
| AreaCytopdivTotTissueArea | Area of cytoplasm divided by the total tissue area |
| AreaEpitNucdivTotTissueArea | Area of epithelial nuclei divided by the total tissue area |
| AreaLumendivTotTissueArea | Area of lumen divided by the total tissue area |
| AreaLymphdivTotTissueArea | Area of lymphoid divided by the total tissue area |
| AreaStrNucdivTotTissueArea | Area of stroma nuclei divided by the total tissue area |
| AreaStromadivTotTissueArea | Area of stroma divided by the total tissue area |
| NormTotalNucleiArea | Total nuclei unit area divided by the total tissue area |
| NormGlandTissueArea | Total gland unit area divided by the total tissue area |

SD, standard deviation

Quantitative M-Plex™ Immunofluorescence

All 15 antibodies used in exhibited the expected cellular localization and distribution when evaluated using control tissue samples. Tumor specimens from 59 patients were assessed using the 15 antibodies as listed in the methods section. Representative regions of interest for each antibody were selected based on the amount and quality of the tumor within the sample, avoiding regions of necrosis and cellular debris. A minimum of three fields were acquired per patient sample in each multiplex analysis, and all images were processed using the immunofluorescence software to generate 84 quantitative features. Activated caspase-3 and phosphorylated mTOR were found focally within infiltrating lymphocytes and tumor epithelial cells. In contrast, cyclin D1 was predominantly present within nuclei of tumor epithelial cells. Of interest, pKDR was found diffusely within islands of tumor epithelial and endothelial cells.

The 84 features (Table 30) represent the algorithm output (mean, max and standard deviation as it relates to both intensity and area) from the individual biomarkers with respect to the compartment they are found in (i.e. DAPI=nuclear; nuclear for cyclin D1, Ki67, PTEN, and phosphorylated ERK (pERK); cytoplasmic for CK18, caspase-3a, and VEGF; nuclear/cytoplasmic for phosphorylated AKT; membrane for EGFR and KDR; and cytoplasmic/membrane for phosphorylated KDR (pKDR), phosphorylated mTOR, PI3K, and phosphorylated EGFR.), distribution of the signal (i.e.

pixels as geometric Dot's vs. pixels within an Object such as a nucleus, cytoplasm) and relationship to one another (e.g. EGFR to pEGFR; p-mTOR to pERK).

Table 30. Quantitative immunofluorescence features.

| Feature Name |
| --- |
| Dapiwithcyc10002 |
| Dcyclkmean500007 |
| Dcyclvar1000013 |
| Ocyclmean500027 |
| Ocyclvar1000030 |
| Ocyclvar500032 |
| Oquantile95cyc10038 |
| Ratio1 00cyc0041 |
| Ratio50cyc0043 |
| B1 EgfrEgfrmean500004 |
| B1EgfrEgfrq950010 |
| B1 EgfrEgfrvar1 000016 |
| B2pEgfrmean500023 |
| B2pEgfrvar1000035 |
| CorrB1 EgfrEgfrB2pEgfr0040 |
| OB1 Egfrmean500053 |
| OB1Egfrq950059 |
| OB1 Egfrvar1000065 |
| OB2pEgfrmean500072 |
| OB2pEgfrq950077 |
| OB2pEgfrvar1000083 |
| VaroverlapB 1Egfr0094 |
| VaroverlapB2pEgfr0095 |
| B1 Vegfmean500004 |
| B1Vegfq950010 |
| B1 Vegfvar1000016 |
| B2pKdrmean500023 |
| B2pKdrq950029 |
| B2pKdrvar1000035 |
| CorrB1 VegfB2pKdr0040 |
| OB1 Vegfmean500053 |
| OB1 Vegfvar1000065 |
| OB2pKdrmean500072 |
| OB2pKdrq950077 |
| OB2pKdrvar1000083 |
| VaroverlapB2pKdr0095 |
| B3Caspasemean 1000040 |
| B3Caspasemean500042 |
| B3Caspaseq950048 |
| B3Caspaseratio 1000049 |
| B3Caspaseratio500051 |
| B3Caspasevar1000054 |
| B4Pi3kmean500061 |
| B4Pi3kq950067 |
| B4Pi3kratio1000068 |
| B4Pi3kratio500070 |
| B4Pi3kvar1000073 |

(continued)

| Feature Name |
| --- |
| Corrb 1B2Ki670078 |
| OB2Ki67mean500122 |
| OB2Ki67q950127 |
| OB2Ki67ratio1000128 |
| OB2Ki67ratio500130 |
| OB2Ki67var1000133 |
| VarB3Caspase0144 |
| VarB4Pi3k0145 |
| B3KDRmean500042 |
| B3KDRq950048 |
| B3KDRratio 1000049 |
| B3KDRratio500051 |
| B3KDRvar1000054 |
| B4pAKTmean500061 |
| B4pAKTq950067 |
| B4pAKTratio 1000068 |
| B4pAKTratio500070 |
| B4pAKTvar1000073 |
| Corrb1 B2pTEN0078 |
| OB2pTENmean500122 |
| OB2pTENq950127 |
| OB2pTENratio1000128 |
| OB2pTENratio500130 |
| OB2pTENvar1000133 |
| VarB3KDR0144 |
| VarB4pAKT0145 |
| MeanVesselArea0030 |
| StdVesselArea0034 |
| EpitInPmtoWithPerkMinMeaCha40026 |
| Dmptormean500036 |
| Dperkmean500039 |
| Dperkvar1000042 |
| Dpmtormean0048 |
| Dpmtorquantile950056 |
| Dpmtorvar1000058 |
| Dquantile95perk0065 |
| Dvarperk0066 |

Predictive Model Development

Using an integrative 'systems pathology' approach, eight models of overall survival were developed, based on clinical variables plus different combinations of the following features: EGFR immunohistochemistry (molecular), histologic morphometry (morphometric), and immunofluorescence (molecular). Note that because some patients were missing data for some of the feature domains, as additional domains are added, the number of patients included is reduced from a maximum of 284 to a minimum set of 51. Because none of the 6 clinical features passed the CI filter, SVRc-FR could not be applied to the dataset with clinical features only (cohort of 284 patients).

Table 31. Performance and features chosen in the 8 models constructed by SVRc-FR.

| Dataset | Number of patients | Number of patients alive | CI of best model | Features chosen (in order of importance in the model) | Contribution of feature |
|---|---|---|---|---|---|
| Clinical | 284 | 16 | NA | no features passed the CI filter | NA |
| Clinical + IHC | 55 | 1 | 0.62 | ps_m | -5.35 |
| Clinical + IF | 56 | 2 | 0.72 | ps_m | -3.85 |
| | | | | B3 Caspasemean 1000040 | -3.22 |
| | | | | Ocyclvar1000030 | -2.29 |
| | | | | B1Vegfvar1000016 | -2.24 |
| | | | | VaroverlapB2pKdr0095 | -1.99 |
| Clinical + IHC + IF | 52 | 1 | 0.73 | ps_m | -4.75 |
| | | | | VaroverlapB2pKdr0095 | -2.63 |
| | | | | B3Caspasemean1000040 | -2.52 |
| | | | | Dcyclkmean500007 | -2.21 |
| Clinical + H&E | 53 | 2 | 0.67 | ps_m | -4.72 |
| | | | | AreaLumendivTotTissueArea | 2.46 |
| Clinical + IHC + H&E | 51 | 1 | 0.68 | ps_m | -4.77 |
| | | | | AreaLumendivTotTissueArea | 2.51 |
| Clinical + IF + H&E | 53 | 2 | 0.75 | ps_m | -3.50 |
| | | | | Dcyclkmean500007 | -2.49 |
| | | | | AreaLumendivTotTissueArea | 2.36 |
| | | | | B3Caspasemean1000040 | -1.89 |
| | | | | VaroverlapB2pKdr0095 | -1.86 |
| | | | | EpitInPmtoWithPerkMinMeaCha40026 | -1.80 |
| | | | | B2pKdrvar1000035 | -1.54 |
| | | | | Ocyclvar1000030 | -1.54 |
| | | | | VarB3Caspase0144 | -1.41 |
| Clinical + IHC + IF + H&E | 51 | 1 | 0.74 | ps_m | -4.77 |
| | | | | B3Caspasemean1000040 | -2.51 |
| | | | | Ocyclvar1000030 | -2.32 |
| | | | | B2pKdrvar1000035 | -2.17 |
| | | | | Dcyclkmean500007 | -2.08 |
| | | | | AreaLumendivTotTissueArea | 1.91 |

To assess the utility of the systems pathology approach, the above eight models were trained using data from the subset of patients with complete information for all of the feature domains (n = 51). Since IHC did not pass the CI filter, excluding this feature did not change the model (e.g., the clinical + IHC model is equivalent to the clinical model). Table 32 presents the results of SVRc-FR on the 51-patient cohort. Performance status was the only clinical variable that passed the CI filter. The only H&E feature selected was the relative area of lumen. The addition of IF features to the clinical features improved the CI. The addition of the H&E features to the clinical data also improved the CI, but slightly less than IF did.

Table 32. SVRc-FR models constructed from the cohort of 51 patients with complete information.

| Dataset | CI of best model | Features chosen (in order of importance in the model) | Contribution of feature |
|---|---|---|---|
| Clinical | 0.62 | ps_m | -4.766 |

(continued)

| Dataset | CI of best model | Features chosen (in order of importance in the model) | Contribution of feature |
|---|---|---|---|
| Clinical + IF | 0.73 | ps_m | -4.766 |
|  |  | B3 Caspasemean 1000040 | -2.4688 |
|  |  | Ocyctvart1000030 | -2.2744 |
|  |  | B2pKdrvar1000035 | -2.1261 |
|  |  | Dcyclkmean500007 | -2.0558 |
| Clinical + H&E | 0.68 | ps_m | -4.766 |
|  |  | AreaLumendivTotTissueArea | 2.5081 |
| Clinical + IF + H&E | 0.74 | ps_m | -4.766 |
|  |  | B3Caspasemean1000040 | -2.5079 |
|  |  | Ocyclvar1000030 | -2.3232 |
|  |  | B2pKdrvar1000035 | -2.1663 |
|  |  | Dcyclkmean500007 | -2.0788 |
|  |  | AreaLumendivTotTissueArea | 1.9107 |

The Cox results for the different models are presented in Table 33. These are multivariable analyses based on a Cox model with stepwise selection of variables. All features that had a p-value <= 0.15 were entered as input into the Cox model. In order to stay in the model, a feature needed a p-value <= 0.05.

A hazard ratio greater than 1 indicates a negative effect upon the survival (higher value of the feature predicts shorter survival time); a hazard ratio less than 1 indicates that the feature has a protective effect (higher value of the feature predicts longer survival time). Based on these results, performance status was found to be a significant clinical predictor of survival. Of the IF features, often the same antibody was selected in Cox results and SVRc models; however, the selected feature captured an attribute different from the attribute selected using SVRc-FR. Of the H&E features, the Cox model found that the mean area of the epithelial nuclei and the relative area of the cytoplasm were predictive of survival.

Table 33. Cox results.

| Dataset | DF | Parameter Estimate | Standard Error | Chi-Square | Pr > ChiSq | HR |
|---|---|---|---|---|---|---|
| **Clinical** |  |  |  |  |  |  |
| ps_m | 1 | 0.73059 | 0.12598 | 33.6295 | <0.0001 | 2.076 |
| sex_m | 1 | 0.30755 | 0.12428 | 6.1234 | 0.0133 | 1.360 |
| **Clinical + IHC** |  |  |  |  |  |  |
| ps_m | 1 | 1.11823 | 0.32541 | 11.8089 | 0.0006 | 3.059 |
| **Clinical + IF** |  |  |  |  |  |  |
| ps_m | 1 | 2.76611 | 0.55914 | 24.4737 | <0.0001 | 15.897 |
| sex_m | 1 | -0.90167 | 0.39445 | 5.2254 | 0.0223 | 0.406 |
| smk_hist_m | 1 | 2.51627 | 0.55970 | 20.2116 | <0.0001 | 12.382 |
| Ocyclmean500027 | 1 | 0.00399 | 0.00125 | 10.1507 | 0.0014 | 1.004 |
| B1EgfrEgfrq950010 | 1 | -0.00143 | 0.0003890 | 13.5689 | 0.0002 | 0.999 |
| B1Vegfmean500004 | 1 | -0.0005077 | 0.0001250 | 16.4936 | <.0001 | 0.999 |
| B1Vegfvar1000016 | 1 | 0.00108 | 0.0004208 | 6.5637 | 0.0104 | 1.001 |
| OB2pKdrmean500072 | 1 | 0.0004915 | 0.0001789 | 7.5490 | 0.0060 | 1.000 |
| B3Caspaseq950048 | 1 | -0.00166 | 0.0005254 | 9.9609 | 0.0016 | 0.998 |
| Corrb1B2Ki670078 | 1 | -5.34023 | 1.10152 | 23.5036 | <0.0001 | 0.005 |
| VarB3Caspase0144 | 1 | 0.00643 | 0.00170 | 14.2993 | 0.0002 | 1.006 |
| Corrb1B2pTEN0078 | 1 | 1.56428 | 0.76844 | 4.1439 | 0.0418 | 4.779 |
| OB2pTENvar1000133 | 1 | -0.01540 | 0.00423 | 13.2270 | 0.0003 | 0.985 |

(continued)

| | | DF | | | | | HR |
|---|---|---|---|---|---|---|---|
| **Clinical + IF** | | | | | | | |
| Dperkmean500039 | | 1 | 0.00140 | 0.0003034 | 21.1775 | <0.0001 | 1.001 |
| **Clinical + IHC + IF** | | | | | | | |
| ps_m | | 1 | 1.38468 | 0.41388 | 11.1932 | 0.0008 | 3.994 |
| Dcyclvar1000013 | | 1 | 0.00164 | 0.0005454 | 9.0105 | 0.0027 | 1.002 |
| B1Vegfmean500004 | | 1 | -0.0001949 | 0.0000782 | 6.2195 | 0.0126 | 1.000 |
| EpitInPmtoWithPerkMinMeaCha40026 | | 1 | 0.00378 | 0.00174 | 4.7192 | 0.0298 | 1.004 |
| **Clinical + H&E** | | | | | | | |
| ps_m | | 1 | 1.10707 | 0.33782 | 10.7395 | 0.0010 | 3.025 |
| smk_hist_m | | 1 | 1.70880 | 0.49665 | 11.8381 | 0.0006 | 5.522 |
| EpithelialNucleiMeanAreaPx10093 | | 1 | -0.00958 | 0.00329 | 8.4502 | 0.0037 | 0.990 |
| **Clinical + IHC + H&E** | | | | | | | |
| ps_m | | 1 | 1.25964 | 0.35019 | 12.9383 | 0.0003 | 3.524 |
| smk_hist_m | | 1 | 1.59729 | 0.49377 | 10.4646 | 0.0012 | 4.940 |
| CytoplasmMeanMeanChannel40058 | | 1 | -0.01083 | 0.00550 | 3.8834 | 0.0488 | 0.989 |
| EpithelialNucleiMeanAreaPx10093 | | 1 | -0.01150 | 0.00349 | 10.8336 | 0.0010 | 0.989 |
| **Clinical + IF + H&E** | | | | | | | |
| Dcyclvar1000013 | | 1 | 0.00200 | 0.0005022 | 15.8982 | <0.0001 | 1.002 |
| OB2pKdrvar1000083 | | 1 | 0.0006294 | 0.0002266 | 7.7146 | 0.0055 | 1.001 |
| OB2pTENratio500130 | | 1 | -21.08066 | 8.03902 | 6.8764 | 0.0087 | 0.000 |
| **Clinical + IHC + IF + H&E** | | | | | | | |
| ps_m | | 1 | 1.48368 | 0.41363 | 12.8661 | 0.0003 | 4.409 |
| smk_hist_m | | 1 | 1.04319 | 0.47848 | 4.7534 | 0.0292 | 2.838 |
| Dcyclvar1000013 | | 1 | 0.00186 | 0.0006154 | 9.1377 | 0.0025 | 1.002 |
| B1Vegfmean500004 | | 1 | -0.0002506 | 0.0000807 | 9.6380 | 0.0019 | 1.000 |
| EpitInPmtoWithPerkMinMeaCha40026 | | 1 | 0.00398 | 0.00184 | 4.6619 | 0.0308 | 1.004 |
| AreaCytopdivTotTissueArea | | 1 | 2.55288 | 0.99480 | 6.5855 | 0.0103 | 12.844 |

DF, degrees of freedom; HR, hazard ratio.

A detailed description of the model based on all four feature domains is described below. Complete data for each of the input domains were available for 51 patients. Their demographic characteristics were similar to those of the overall cohort (Table 24).

The features selected in the final model are listed in Table 26, in order of their importance in the model. A positive contribution for a feature means that higher values predict a better outcome (longer survival time), and a negative contribution means that higher values predict a worse outcome. The feature whose contribution is furthest from zero is deemed the most important.

The final model performed with a CI of 0.74. By comparison, the model utilizing only clinical data produced a CI of 0.62; performance status was the only clinical feature selected. Thus, adding immunofluorescence and H&E features increased the ability to predict survival by 12%.

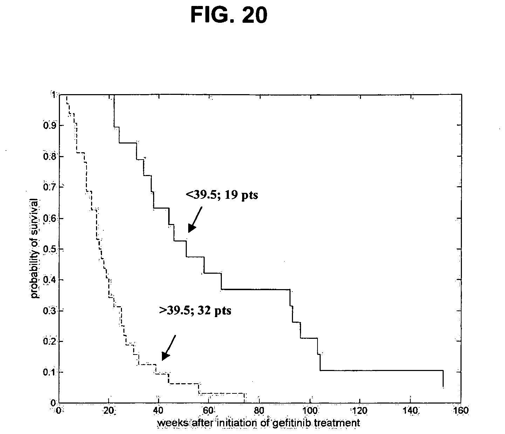

Model scores were analyzed by a chi-squared statistic based on the log-rank test; this analysis demonstrated that a cut-point of 39.5 most strongly separated patients in terms of their actual survival experience (chi-square, 21.39; adjusted p-value, 0.0002). Scores above 39.5 had a hazard ratio of 5.26 (95% confidence interval: 2.60, 10.62), i.e. for patients with scores above 39.5 compared to those with lower scores, the risk of death over the study period was 5 times higher. This hazard ratio for risk of death would need to be confirmed in a retrospective, independent cohort. Kaplan-Meier curves for patients above and below the cut-point were generated, as shown in FIG. 20.

The predictive value of individual features was further tested by Cox analysis. This analysis confirmed that performance status (hazard ratio: 4.4, 95% confidence interval 1.96 - 9.92) was a significant predictor of survival across all models. For IF features, which performed less robustly than performance status, often the same marker was selected (i.e. cyclin D1, pKDR, caspase-3), although different derived features, reflecting different attributes of the marker, were selected between and within the different models.

Table 26. Features selected in the final model, developed from clinical, histologic morphometry, immunohistochemical, and immunofluorescent feature domains

| Feature | Contribution |
| --- | --- |
| Performance status | -4.766 |
| Caspase-3 mean intensity level | -2.5079 |
| Cyclin D1 overall intensity | -2.3232 |
| pKDR variability | -2.1663 |
| Cyclin D1 mean intensity | -2.0788 |
| Relative area of lumen (alveolar) space | 1.9107 |

Discussion

There is consensus in the medical community on the critical importance of identifying NSCLC patients who can benefit from gefitinib, even when no objective response is seen on imaging studies. For this reason, we investigated response to gefitinib using overall survival as the end-point. It has been demonstrated that a predictive model for overall survival of patients treated with gefitinib is improved by addition of information in the patients' tumor samples. By integrating quantitative biomarker characteristics and histologic morphometry with clinical data the accuracy (CI) of such a predictive model has been increased from 62% (clinical features alone) to 74% (all domains).

Numerous variables have been proposed as affecting gefitinib sensitivity, including gender, tumor histology (e.g. adenocarcinoma/bronchoalveolar carcinoma), smoking history, ethnicity, EGFR gene copy number EGFR kinase domain mutations, and even type of EGFR mutations. Recently, it was reported that patients with an EGFR exon 19 deletion had longer median survival than patients with an L858R mutation. Additionally, germline polymorphisms in the EGFR promoter have been proposed as factors potentially relevant to response to gefitinib in patients who do not have tyrosine kinase domain mutations. Furthermore, responses to gefitinib have been observed in patients with NSCLC regardless of tumor histology or patient demographics.

In the current EAP cohort, EGFR mutations were identified in exons 19-21 in 4 out of the 87 patients. Although the frequency of mutation was too low to allow the inclusion of these mutations in a predictive model, it was consistent with prior studies involving predominantly Western patients. In this cohort, as in previous studies, the frequency of EGFR mutations was much lower than the probable frequency of clinical benefit from gefitinib. In an analysis of 124 patients from the current EAP cohort, 45% of the patients had no evidence of progression at first re-evaluation and 29% reported improvement in lung-cancer-related symptoms while receiving gefitinib.

The only clinical variable consistently identified as associated with survival was performance status. This is in agreement with an earlier study that demonstrated that good performance status (ECOG status 0-2) was associated with longer survival. Bronchoalveolar histology, which has been associated with good clinical outcome, was not selected in our predictive model; however, the selected histologic imaging feature, 'area of lumen divided by total tissue area,' can be interpreted as a surrogate morphologic feature of this subtype of adenocarcinoma.

The molecular mechanisms underlying the differing gefitinib sensitivities of subpopulations of patients with advanced NSCLC remain incompletely understood. In addition to EGFR mutations and EGFR gene copy number, other molecular variables that have been examined include an intact EGFR, phosphorylated EGFR, and downstream molecules such as PI3K, pAkt, and pERK.[31, 52-54] Unfortunately there is disagreement on the role, if any, that each of these factors plays in gefitinib sensitivity, and there is less agreement on their importance for overall survival. Even EGFR, as assessed by immunohistochemistry, has been the subject of conflicting reports of its association with gefitinib response.

In the current analysis several markers were identified by quantitative immunofluorescence that were associated with overall survival. Of significance, two cyclin D1 features were selected by the final model, with increased amounts associated with shorter overall survival.

The remaining biomarkers selected by the model, including activated caspase-3 and phosphorylated KDR, have not been widely investigated in lung cancer clinical specimens. Caspase-3 has been the subject of conflicting reports linking over expression with median survival. For pKDR; however, there are no recent published studies on immunohistochemistry or immunofluorescence in clinical NSCLC samples. The Cox models confirmed that over expression of VEGF (based on selected features) was associated with reduced survival, which is in agreement with earlier studies examining VEGF in conjunction with microvessel density and tumor invasion. The fact that cyclin D1, caspase-3, pKDR, and VEGF were selected repeatedly in various sub-models suggests the importance of cell cycle regulation and angiogenesis for tumor growth, metastasis, and survival.

Thus, a series of integrative, system-based models have been built that successfully predict overall survival in advanced NSCLC patients treated with gefitinib. The selected biomarkers are provocative in that they represent manifestations of cell cycle regulation, apoptosis, and angiogenesis.

## ADDITIONAL EMBODIMENTS

Thus it is seen that methods and systems are provided for predicting the occurrence of a medical condition. Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made without departing from the scope of the invention as defined by the claims. Other aspects, advantages, and modifications are considered to be within the scope of the following claims. The claims presented are representative of the inventions disclosed herein. Other, unclaimed inventions are also contemplated. Applicants reserve the right to pursue such inventions in later claims.

Insofar as embodiments of the invention described above are implementable, at least in part, using a computer system, it will be appreciated that a computer program for implementing at least part of the described methods and/or the described systems is envisaged as an aspect of the present invention. The computer system may be any suitable apparatus, system or device. For example, the computer system may be a programmable data processing apparatus, a general purpose computer, a Digital Signal Processor or a microprocessor. The computer program may be embodied as source code and undergo compilation for implementation on a computer, or may be embodied as object code, for example.

It is also conceivable that some or all of the functionality ascribed to the computer program or computer system aforementioned may be implemented in hardware, for example by means of one or more application specific integrated circuits.

Suitably, the computer program can be stored on a carrier medium in computer usable form, which is also envisaged as an aspect of the present invention. For example, the carrier medium may be solid-state memory, optical or magneto-optical memory such as a readable and/or writable disk for example a compact disk (CD) or a digital versatile disk (DVD), or magnetic memory such as disc or tape, and the computer system can utilize the program to configure it for operation. The computer program may also be supplied from a remote source embodied in a carrier medium such as an electronic signal, including a radio frequency carrier wave or an optical carrier wave.

## REFERENCES

The following references, some of which are referred to above, are all hereby incorporated by reference herein in their entireties:

[1] Scherr D., et al., Urology. 61 (2 Suppl 1): 14-24, Feb. 2003, Swindle P.W., et al., Urologic Clinics of North America. 30(2):377-401, May 2003.

[2] Wahlby C., et al., Analytical Cellular Pathology 24, 101-111, 2002.

[3] Street W.N., "Xcyt: A System for Remote Cytological Diagnosis and Prognosis of Breast Cancer," In Soft Computing Techniques in Breast Cancer Prognosis and Diagnosis, L.C. Jain (ed.), CRC Press, 1999

[4] Gleason D.F., "The Veteran's Administration Cooperative Urologic Research Group: Histologic Grading and Clinical Staging of Prostatic Carcinoma," In Urologic Pathology: The Prostate, Tannenbaum M. (ed.), 171-198, Lea and Febiger, Philadelphia, 1977.

[5] Cristianni et al., An Introduction to Support Vector Machines, Cambridge University Press (2000).

[6] Hastie, The Elements of Statistical Learning, Springer (2001).

[7] F.E. Harrell et al., "Evaluating the yield of medical tests," JAMA, 247(18):2543-2546, 1982.

[8] Bishop, C., Neural Networks for Pattern Recognition, Oxford University Press (1995).

[9] Fausett, L., Fundamentals of Neural Networks, New York, Prentice Hall (1994).

[10] Definiens Cellenger Architecture: A Technical Review, April 2004.

[11] Baatz M. and Schäpe A., "Multiresolution Segmentation - An Optimization Approach for High Quality Multi-scale Image Segmentation," In Angewandte Geographische Informationsverarbeitung XII, Strobl, J., Blaschke, T., Griesebner, G. (eds.), Wichmann- Verlag, Heidelberg, 12-23, 2000.

[12] Fukunaga K., Introduction to Statistical Pattern Recognition, 2nd Edition, Boston: Academic Press, 1990.

[13] Duda R.O. et al., Pattern Classification, 2nd Edition, John Wiley & Sons Inc., 2001.

[14] Holmberg L. et al., A randomized trial comparing radical prostatectomy with watchful waiting in early prostate cancer, N. Engl. M. Med., 347:781-789 (2002).

[15] Pound CR et al., Natural history of progression after PSA elevation following radical prostatectomy, JAMA 1999, 281:1591-1597.

[16] Kumar-Sinha C. et al., Molecular markers to identify patients at risk for recurrence after primary treatment for prostate cancer, Urology 2003; 62 Suppl. 1:19-35.

[17] Cox D.R., "Regression Models and Life Tables," Journal of the Royal Statistical Society, B 34, 187-220, 1972.

[18] Harrell F.E., Regression Modeling Strategies, Springer-Verlag 2001.

[19] Tuxhorn et al., "Reactive Stroma in Human Prostate Cancer: Induction of Myofibroblast Phenotype and Extracellular Matrix Remodeling" Clinical Cancer Research 2912 Vol. 8, 2912-2923, September 2002.

[20] Kattan et al., "Postoperative Nomogram for Disease Recurrence After Radical Prostatectomy for Prostate Cancer," Journal of Clinical Oncology, Vol. 17, No. 5 (May), 1999: pp 1499-1507.

[21] C. Vonesch, F. Aquet, J.L. Vonesch and M. Unser, "The colored revolution of bioimaging," IEEE Signal Proc. Mag., vol. 23, no. 3, pp. 20-31, May 2006.

[22] A. Krtolica, C. O. de Solorzano, S. Lockett and J. Campisi, "Quantification of epithelial cells in coculture with fibroblast by fluorescence image analysis," Cytometry, vol. 49, pp. 73-82, 2002.

[23] A. Gordon, A. Colman-Lerner, T. E. Chin, K. R. Benjamin, R. C. Yu, and R. Brent, "Single-cell quantification of molecules and rates using open-source microscope-based cytometry," Nature Methods, vol. 4, pp. 175-181, 2007.

[24] R. Camp, G. G. Chung, and D. L. Rimm, "Automated subcellular localization and quantification of protein expression in tissue microarrays," Nature Medicine, vol. 8, pp. 1323-1327, 2002.

[25] J. Y. Rao, D. Seligson, and G. P. Hemstreet, "Protein expression analysis using quantitative fluorescence image analysis on tissue microarray slides," BioTechniques, vol. 32, pp. 924-932, 2002.

[26] Definiens Understanding Images, Developer Version 6, 2007. http://www.definiens.com.

[27] http://www.cri-inc.com.

[28] A. Tabesh, M. Teverovskiy, H.-Y Pang, V. P. Kumar, D. Verbel, A. Kotsianti, and O. Saidi, "Multifeature prostate cancer diagnosis and Gleason grading of histological images," IEEE Trans. Medical Imag., vol. 26, pp. 1366-1378, 2007.

[29] Giaccone G, Herbst RS, Manegold C, et al: Gefitinib in combination with gemcitabine and cisplatin in advanced non-small-cell lung cancer: a phase III trial--INTACT 1. J Clin Oncol 22:777-84, 2004.

[30] Herbst RS, Giaccone G, Schiller JH, et al: Gefitinib in combination with paclitaxel and carboplatin in advanced non-small-cell lung cancer: a phase III trial--INTACT 2. J Clin Oncol 22:785-94, 2004.

[31] Thatcher N, Chang A, Parikh P, et al. Gefitinib plus best supportive care in previously treated patients with refractory advanced non-small-cell lung cancer: results from a randomised, placebo-controlled, multicentre study (Iressa Survival Evaluation in Lung Cancer). Lancet 366:1527-37, 2005.

[32] Lynch TJ, Bell DW, Sordella R, et al: Activating mutations in the epidermal growth factor receptor underlying responsiveness of non-small-cell lung cancer to gefitinib. N Engl J Med 350:2129-39, 2004.

[33] Dudek AZ, Kmak KL, Koopmeiners J, et al: Skin rash and bronchoalveolar histology correlates with clinical benefit in patients treated with gefitinib as a therapy for previously treated advanced or metastatic non-small cell lung cancer. Lung Cancer 51:89-96, 2006.

[34] Cappuzzo F, Hirsch FR, Rossi E, et al: Epidermal growth factor receptor gene and protein and gefitinib sensitivity in non-small-cell lung cancer. J Natl Cancer Inst 97:643-55, 2005.

[35] Oken MM, Creech RH, Tormey DC, Horton J, Davis TE, McFadden et al: Toxicity and response criteria of the Eastern Cooperative Oncology Group. Am J Clin Oncol 5:649-655, 1982. Cordon-Cardo C, Kotsianti A, Donovan M, et al: Improved prediction of PSA recurrence through systems pathology. J Clin Oncol 22:4591, 2004 (abstr.).

[36] Zubek V, Verbel D, Saidi O: Censored time trees for predicting time to PSA recurrence, in Proceedings of the Fourth International Conference on Machine Learning and Applications (ICMLA 2005). Washington, DC, IEEE Computer Society, 2005, pp 221-226.

[37] Paez JG, Janne PA, Lee JC, et al: EGFR mutations in lung cancer: correlation with clinical response to gefitinib therapy. Science 304:1497-500, 2004.

[38] Bell DW, Gore I, Okimoto RA, et al: Inherited susceptibility to lung cancer may be associated with the T790M drug resistance mutation in EGFR. Nat Genet 37:1315-6, 2005.

[39] Hirsch FR, Varella-Garcia M, Bunn PA, Jr., et al: Epidermal growth factor receptor in non-small-cell lung carcinomas: correlation between gene copy number and protein expression and impact on prognosis. J Clin Oncol 21:3798-807, 2003.

[40] Chan SK, Gullick WJ, Hill ME: Mutations of the epidermal growth factor receptor in non-small cell lung cancer -- search and destroy. Eur J Cancer 42:17-23, 2006.

[41] Riely GJ, Pao W, Pham D, et al: Clinical course of patients with non-small cell lung cancer and epidermal growth factor receptor exon 19 and exon 21 mutations treated with gefitinib or erlotinib. Clin Cancer Res 12:839-44, 2006.

[42] Sequist LV, Bell DW, Lynch TJ, et al: Molecular predictors of response to epidermal growth factor receptor antagonists in non-small-cell lung cancer. J Clin Oncol 25:587-95, 2007.

[43] Uchida A, Hirano S, Kitao H, et al: Activation of downstream epidermal growth factor receptor (EGFR) signaling provides gefitinib-resistance in cells carrying EGFR mutation. Cancer Sci 98:357-63, 2007.

[44] Sugio K, Uramoto H, Ono K, et al: Mutations within the tyrosine kinase domain of EGFR gene specifically occur in lung adenocarcinoma patients with a low exposure of tobacco smoking. Br J Cancer 94:896-903, 2006.

[45] Edelman MJ: An update on the role of epidermal growth factor receptor inhibitors in non-small cell lung cancer. Semin Oncol 32:S3-8, 2005.

[46] Kris MG, Natale RB, Herbst RS, et al: Efficacy of gefitinib, an inhibitor of the epidermal growth factor receptor tyrosine kinase, in symptomatic patients with non-small cell lung cancer: a randomized trial. Jama 290:2149-58, 2003.

[47] Al-Kuraya K, Siraj AK, Bavi P, et al: High epidermal growth factor receptor amplification rate but low mutation frequency in Middle East lung cancer population. Hum Pathol 37:453-7, 2006.

[48] Hainsworth JD, Mainwaring MG, Thomas M, et al: Gefitinib in the treatment of advanced, refractory non-small-cell lung cancer: results in 124 patients. Clin Lung Cancer 4:347-55, 2003.

[49] Su WP, Yang CH, Yu CJ, et al: Gefitinib treatment for non-small cell lung cancer -- a study including patients with poor performance status. J Formos Med Assoc 104:557-62, 2005.

[50] Erman M, Grunenwald D, Penault-Llorca F, et al: Epidermal growth factor receptor, HER-2/neu and related pathways in lung adenocarcinomas with bronchioloalveolar features. Lung Cancer 47:315-23, 2005.

[51] Massion PP, Taflan PM, Shyr Y, et al: Early involvement of the phosphatidylinositol 3-kinase/Akt pathway in lung cancer progression. Am J Respir Crit Care Med 170:1088-94, 2004.

[52] Cappuzzo F, Magrini E, Ceresoli GL, et al: Akt phosphorylation and gefitinib efficacy in patients with advanced non-small-cell lung cancer. J Natl Cancer Inst 96:1133-41, 2004.

[53] Han SW, Hwang PG, Chung DH, et al: Epidermal growth factor receptor (EGFR) downstream molecules as response predictive markers for gefitinib (Iressa, ZD1839) in chemotherapy-resistant non-small cell lung cancer. Int J Cancer 113:109-15, 2005.

[54] Bailey L, Kris M, Wolf M, et al: Tumor EGFR membrane staining is not clinically relevant for predicting response in patients receiving gifitinib (Iressa, ZD 1839) monotherapy for pretreated advanced non-small-cell lung cancer: IDEAL 1 and 2. Proc Am Assoc Cancer Res 44:1362, 2003 (abstr.).

[55] Bailey L, Janas M, Schmidt K, et al: Evaluation of epidermal growth factor receptor (EGFR) as a predictive marker in patients with non-small-cell lung cancer (NSCLC) receiving first-line gefitinib combined with platinum-based chemotherapy. J Clin Oncol 22:7013, 2004 (abstr.).

[56] Jin M, Inoue S, Umemura T, et al: Cyclin D1, p16 and retinoblastoma gene product expression as a predictor for prognosis in non-small cell lung cancer at stages I and II. Lung Cancer 34:207-18, 2001.

[57] Keum JS, Kong G, Yang SC, et al: Cyclin D1 overexpression is an indicator of poor prognosis in resectable non-small cell lung cancer. Br J Cancer 81:127-32, 1999.

[58] Kalish LH, Kwong RA, Cole IE, et al: Deregulated cyclin D1 expression is associated with decreased efficacy of the selective epidermal growth factor receptor tyrosine kinase inhibitor gefitinib in head and neck squamous cell carcinoma cell lines. Clin Cancer Res 10:7764-74, 2004.

[59] Koomagi R, Volm M: Relationship between the expression of caspase-3 and the clinical outcome of patients with non-small cell lung cancer. Anticancer Res 20:493-6, 2000.

[60] Takata T, Tanaka F, Yamada T, et al: Clinical significance of caspase-3 expression in pathologic-stage I, nonsmall-cell lung cancer. Int J Cancer 96 Suppl:54-60, 2001.

[61] Mineo TC, Ambrogi V, Baldi A, et al: Prognostic impact of VEGF, CD31, CD34, and CD105 expression and tumour vessel invasion after radical surgery for IB-IIA non-small cell lung cancer. J Clin Pathol 57:591-7, 2004.

**Table 1. Morphometric Features Script v1.0 (496 Features)**

| Feature |
| --- |
| Background.MaxAreaPxl |
| Background.MeanAreaPxl |
| Background.MinAreaPxl |
| Background.StdDevAreaPxl |
| Background. SumAreaPxl |
| Cytoplasm.Objects |
| Cytoplasm.ObjectsPct |
| Cytoplasm.MaxAreaPxl |
| Cytoplasm.MeanAreaPxl |
| Cytoplasm.MinAreaPxl |
| Cytoplasm.StdDevAreaPxl |

(continued)

| Feature |
| --- |
| Cytoplasm.SumAreaPxl |
| Cytoplasm.MaxAsymmetry |
| Cytoplasm.MeanAsymmetry |
| Cytoplasm.MinAsymmetry |
| Cytoplasm.StdDevAsymmetry |
| Cytoplasm.MaxBorderlengthPxl |
| Cytoplasm.MeanBorderlengthPxl |
| Cytoplasm.MinBorderlengthPxl |
| Cytoplasm.StdDevBorderlengthPxl |
| Cytoplasm.SumBorderlengthPxl |
| Cytoplasm.MaxBrightness |
| Cytoplasm.MeanBrightness |
| Cytoplasm.MinBrightness |
| Cytoplasm.StdDevBrightness |
| Cytoplasm.MaxCompactness |
| Cytoplasm. Mean Compactness |
| Cytoplasm.MinCompactness |
| Cytoplasm.StdDevCompactness |
| Cytoplasm.MaxDensity |
| Cytoplasm.Mean Density |
| Cytoplasm.MinDensity |
| Cytoplasm.StdDevDensity |
| Cytoplasm.MaxDiff.ofenclosing.enclo |
| Cytoplasm.MeanDiff.ofenclosing.encl |
| Cytoplasm.MinDiff.ofenclosing.enclo |
| Cytoplasm.StdDevDiff.ofenclosing.en |
| Cytoplasm.MaxEllipticFit |
| Cytoplasm.MeanEllipticFit |
| Cytoplasm.MinEllipticFit |
| Cytoplasm.StdDevEllipticFit |
| Cytoplasm.MaxLengthPxl |
| Cytoplasm.MeanLengthPxl |
| Cytoplasm.MinLengthPxl |
| Cytoplasm.StdDevLengthPxl |
| Cytoplasm.SumLengthPxl |
| Cytoplasm.MaxMax.Diff. |
| Cytoplasm.MeanMax.Diff. |
| Cytoplasm.MinMax.Diff. |
| Cytoplasm.StdDevMax.Diff. |
| Cytoplasm.MaxMeanChannel1 |
| Cytoplasm.MeanMeanChannel1 |
| Cytoplasm.MinMeanChannel1 |
| Cytoplasm.StdDevMeanChannel1 |
| Cytoplasm.MaxMeanChannel2 |
| Cytoplasm.MeanMeanChannel2 |
| Cytoplasm.MinMeanChannel2 |
| Cytoplasm.StdDevMeanChannel2 |
| Cytoplasm.MaxMeanChannel3 |
| Cytoplasm.MeanMeanChannel3 |

(continued)

| Feature |
| --- |
| Cytoplasm.MinMeanChannel3 |
| Cytoplasm.StdDevMeanChannel3 |
| Cytoplasm.MaxRadiusoflargestenclose |
| Cytoplasm.MeanRadiusoflargestenclos |
| Cytoplasm.MinRadiusoflargestenclose |
| Cytoplasm.StdDevRadiusoflargestencl |
| Cytoplasm.MaxRadiusofsmallestenclos |
| Cytoplasm.MeanRadiusofsmallestenclo |
| Cytoplasm.MinRadiusofsmallestenclos |
| Cytoplasm.StdDevRadiusofsmallestenc |
| Cytoplasm.MaxStdevChannel1 |
| Cytoplasm.MeanStdevChannel1 |
| Cytoplasm.MinStdevChannel1 |
| Cytoplasm.StdDevStdevChannel1 |
| Cytoplasm.MaxStdevChannel2 |
| Cytoplasm.MeanStdevChannel2 |
| Cytoplasm.MinStdevChannel2 |
| Cytoplasm.StdDevStdevChannel2 |
| Cytoplasm.MaxStdevChannel3 |
| Cytoplasm.MeanStdevChannel3 |
| Cytoplasm.MinStdevChannel3 |
| Cytoplasm.StdDevStdevChanne13 |
| Cytoplasm.MaxWidthPxl |
| Cytoplasm.MeanWidthPxl |
| Cytoplasm.MinWidthPxl |
| Cytoplasm.StdDevWidthPxl |
| Epithelial.Nuclei.Objects |
| Epithelial.Nuclei.ObjectsPct |
| Epithelial.Nuclei.MaxAreaPxl |
| Epithelial.Nuclei.MeanAreaPxl |
| Epithelial.Nuclei.MinAreaPxl |
| Epithelial.Nuclei.StdDevAreaPxl |
| Epithelial.Nuclei.SumAreaPxl |
| Epithelial.Nuclei.MaxAsymmetry |
| Epithelial.Nuclei.MeanAsymmetry |
| Epithelial.Nuclei.MinAsymmetry |
| Epithelial.Nuclei.StdDevAsymmetry |
| Epithelial.Nuclei.MaxBorderlengthPx |
| Epithelial.Nuclei.MeanBorderlengthP |
| Epithelial.Nuclei.MinBorderlengthPx |
| Epithelial.Nuclei.StdDevBorderlengt |
| Epithelial.Nuclei.SumBorderlengthPx |
| Epithelial.Nuclei.MaxBrightness |
| Epithelial.Nuclei.MeanBrightness |
| Epithelial.Nuclei.MinBrightness |
| Epithelial.Nuclei.StdDevBrightness |
| Epithelial.Nuclei.MaxCompactness |
| Epithelial.Nuclei.MeanCompactness |
| Epithelial.Nuclei.MinCompactness |

(continued)

| Feature |
| --- |
| Epithelial.Nuclei.StdDevCompactness |
| Epithelial.Nuclei.MaxDensity |
| Epithelial.Nuclei.MeanDensity |
| Epithelial.Nuclei.MinDensity |
| Epithelial.Nuclei.StdDevDensity |
| Epithelial.Nuclei.MaxDiff.ofenclosi |
| Epithelial.Nuclei.MeanDiff.ofenclos |
| Epithelial.Nuclei.MinDiff.ofenclosi |
| Epithelial.Nuclei.StdDevDiff.ofencl |
| Epithelial.Nuclei.MaxEllipticFit |
| Epithelial.Nuclei.MeanEllipticFit |
| Epithelial.Nuclei.MinEllipticFit |
| Epithelial.Nuclei.StdDevEllipticFit |
| Epithelial.Nuclei.MaxLengthPxl |
| Epithelial.Nuclei.MeanLengthPxl |
| Epithelial.Nuclei.MinLengthPxl |
| Epithelial.Nuclei.StdDevLengthPxl |
| Epithelial.Nuclei.SumLengthPxl |
| Epithelial.Nuclei.MaxMax.Diff. |
| Epithelial.Nuclei .MeanMax.Diff. |
| Epithelial.Nuclei.MinMax.Diff. |
| Epithelial.Nuclei.StdDevMax.Diff. |
| Epithelial.Nuclei.MaxMeanChannel1 |
| Epithelial.Nuclei.MeanMeanChannel1 |
| Epithelial.Nuclei.MinMeanChannel1 |
| Epithelial.Nuclei.StdDevMeanChannel |
| Epithelial.Nuclei.MaxMeanChannel2 |
| Epithelial.Nuclei.MeanMeanChannel2 |
| Epithelial.Nuclei.MinMeanChannel2 |
| Epithelial.Nuclei.StdDevMeanChannel |
| Epithelial.Nuclei.MaxMeanChannel3 |
| Epithelial.Nuclei.MeanMeanChannel3 |
| Epithelial.Nuclei.MinMeanChannel3 |
| Epithelial.Nuclei.StdDevMeanChannel |
| Epithelial.Nuclei.MaxRadiusoflarges |
| Epithelial.Nuclei.MeanRadiusoflarge |
| Epithelial.Nuclei.MinRadiusoflarges |
| Epithelial.Nuclei.StdDevRadiusoflar |
| Epithelial.Nuclei.MaxRadiusofsmalle |
| Epithelial.Nuclei.MeanRadiusofsmall |
| Epithelial.Nuclei.MinRadiusofsmalle |
| Epithelial.Nuclei.StdDevRadiusofsma |
| Epithelial.Nuclei.MaxStdevChannel1 |
| Epithelial.Nuclei.MeanStdevChannel1 |
| Epithelial.Nuclei.MinStdevChannell |
| Epithelial.Nuclei.StdDevStdevChanne |
| Epithelial.Nuclei.MaxStdevChannel2 |
| Epithelial.Nuclei.MeanStdevChannel2 |
| Epithelial.Nuclei.MinStdevChannel2 |

(continued)

| Feature |
| --- |
| Epithelial.Nuclei.StdEvStdevChanne |
| Epithelial.Nuclei.MaxStdevChannel3 |
| Epithelial.Nuclei.MeanStdevChannel3 |
| Epithelial.Nuclei.MinStdevChannel3 |
| Epithelial.Nuclei.StdEvStdevChanne |
| Epithelial.Nuclei.MaxWidthPx1 |
| Epithelial.Nuclei.MeanWidthPxl |
| Epithelial.Nuclei.MinWidthPxl |
| Epithelial.Nuclei.StdDevWidthPxl |
| Lumen.Objects |
| Lumen. Obj ectsPct |
| Lumen.MaxAreaPxl |
| Lumen.MeanAreaPxl |
| Lumen.MinAreaPxl |
| Lumen.StdDevAreaPxl |
| Lumen.SumAreaPxl |
| Lumen.MaxAsymmetry |
| Lumen.MeanAsymmetry |
| Lumen.MinAsymmetry |
| Lumen.StdDevAsymmetry |
| Lumen.MaxBorderlengthPxl |
| Lumen.MeanBorderlengthPxl |
| Lumen.MinBorderlengthPxl |
| Lumen.StdDevBorderlengthPxl |
| Lumen.SumBorderlengthPxl |
| Lumen.MaxBrightness |
| Lumen.MeanBrightness |
| Lumen.MinBrightness |
| Lumen.StdDevBrightness |
| Lumen.MaxCompactness |
| Lumen.MeanCompactness |
| Lumen.MinCompactness |
| Lumen.StdDevCompactness |
| Lumen.MaxDensity |
| Lumen.MeanDensity |
| Lumen.MinDensity |
| Lumen.StdDevDensity |
| Lumen.MaxDiff.ofenclosing.enclosede |
| Lumen.MeanDiff.ofenclosing.enclosed |
| Lumen.MinDiff.ofenclosing.enclosede |
| Lumen.StdDevDiff.ofenclosing.enclos |
| Lumen.MaxEllipticFit |
| Lumen.MeanEllipticFit |
| Lumen.MinEllipticFit |
| Lumen. StdDevEllipticFit |
| Lumen.MaxLengthPxl |
| Lumen.MeanLengthPxl |
| Lumen.MinLengthPxl |
| Lumen.StdDevLengthPxl |

(continued)

| Feature |
| --- |
| Lumen.SumLengthPxl |
| Lumen.MaxMax.Diff. |
| Lumen.MeanMax.Diff. |
| Lumen.MinMax.Diff. |
| Lumen.StdDevMax.Diff. |
| Lumen.MaxMeanChannel1 |
| Lumen.MeanMeanChannel1 |
| Lumen.MinMeanChannel1 |
| Lumen.StdDevMeanChannel1 |
| Lumen.MaxMeanChannel2 |
| Lumen.MeanMeanChannel2 |
| Lumen.MinMeanChannel2 |
| Lumen.StdDevMeanChannel2 |
| Lumen.MaxMeanChannel3 |
| Lumen.MeanMeanChannel3 |
| Lumen.MinMeanChannel3 |
| Lumen.StdDevMeanChannel3 |
| Lumen.MaxRadiusoflargestenclosedell |
| Lumen.MeanRadiusoflargestenclosedel |
| Lumen.MinRadiusoflargestenclosedell |
| Lumen. StdDevRadiusoflargestenclosed |
| Lumen.MaxRadi usofsmallestenclosinge |
| Lumen.MeanRadiusofsmallestenclosing |
| Lumen.MinRadiusofsmallestenclosinge |
| Lumen. StdDevRadiusofsmallestenclosi |
| Lumen.MaxStdevChannel1 |
| Lumen.MeanStdevChannel1 |
| Lumen.MinStdevChannel1 |
| Lumen.StdDevStdevChannel1 |
| Lumen.MaxStdevChannel2 |
| Lumen.MeanStdevChannel2 |
| Lumen.MinStdevChannel2 |
| Lumen.StdDevStdevChannel2 |
| Lumen.MaxStdevChannel3 |
| Lumen.MeanStdevChannel3 |
| Lumen.MinStdevChannel3 |
| Lumen.StdDevStdevChannel3 |
| Lumen.MaxWidthPxl |
| Lumen.MeanWidthPxl |
| Lumen.MinWidthPxl |
| Lumen.StdDevWidthPxl |
| Red.Blood. Cell. Objects |
| Red.Blood.Cell.ObjectsPct |
| Red.Blood.Cell.MaxAreaPxl |
| Red.Blood.Cell.MeanAreaPxl |
| Red.Blood.Cell.MinAreaPxl |
| Red.Blood.Cell.StdDevAreaPxl |
| Red.Blood.Cell.SumAreaPxl |
| Red.Blood.Cell.MaxAsymmetry |

(continued)

| Feature |
| --- |
| Red.Blood.Cell.MeanAsymmetry |
| Red.Blood.Cell.MinAsymmetry |
| Red.Blood.Cell.StdDevAsymmetry |
| Red.Blood.Cell.MaxBorderlengthPxl |
| Red.Blood.Cell.MeanBorderlengthPx |
| Red.Blood.Cell.MinBorderlengthPxl |
| Red.Blood.Cell.StdDevBorderlengthPx |
| Red.Blood.Cell.SumBorderlengthPxl |
| Red.Blood.Cell.MaxBrightness |
| Red.Blood.Cell.MeanBrightness |
| Red.Blood.Cell.MinBrightness |
| Red.Blood.Cell.StdDevBrightness |
| Red.Blood.Cell.MaxCompactness |
| Red.Blood.Cell.MeanCompactness |
| Red.Blood.Cell.MinCompactness |
| Red.Blood.Cell.StdDevCompactness |
| Red.Blood.Cell.MaxDensity |
| Red.Blood.Cell.MeanDensity |
| Red.Blood.Cell.MinDensity |
| Red.Blood.Cell.StdDevDensity |
| Red.Blood.Cell.MaxDiff.ofenclosing. |
| Red.Blood.Cell.MeanDiff.ofenclosing |
| Red.Blood.Cell.MinDiff.ofenclosing. |
| Red.Blood.Cell.StdDevDiff.ofenclosi |
| Red.Blood.Cell.MaxEllipticFit |
| Red.Blood.Cell.MeanEllipticFit |
| Red.Blood.Cell.MinEllipticFit |
| Red.Blood.Cell.StdDevEllipticFit |
| Red.Blood.Cell.MaxLengthPxl |
| Red.Blood.Cell.MeanLengthPxl |
| Red.Blood.Cell.MinLengthPxl |
| Red.Blood.Cell.StdDevLengthPxl |
| Red.Blood.Cell.SumLengthPxl |
| Red.Blood.Cell.MaxMax.Diff. |
| Red.Blood.Cell.MeanMax.Diff. |
| Red.Blood.Cell.MinMax.Diff. |
| Red.Blood.Cell.StdDevMax.Diff. |
| Red.Blood.Cell.MaxMeanChannel1 |
| Red.Blood.Cell.MeanMeanChannel1 |
| Red.Blood.Cell.MinMeanChannel1 |
| Red.Blood.Cell.StdDevMeanChannel1 |
| Red.Blood.Cell.MaxMeanChannel2 |
| Red.Blood.Cell.MeanMeanChannel2 |
| Red.Blood.Cell.MinMeanChannel2 |
| Red.Blood.Cell.StdDevMeanChannel2 |
| Red.Blood.Cell.MaxMeanChannel3 |
| Red.Blood.Cell.MeanMeanChannel3 |
| Red.Blood.Cell.MinMeanChannel3 |
| Red.Blood.Cell.StdDevMeanChannel3 |

(continued)

| Feature |
| --- |
| Red.Blood.Cell.MaxRadiusoflargesten |
| Red.Blood.Cell.MeanRadiusoflargeste |
| Red.Blood.Cell.MinRadiusoflargesten |
| Red.Blood.Cell.StdDevRadiusoflarges |
| Red.Blood.Cell.MaxRadiusofsmalleste |
| Red.Blood.Cell.MeanRadiusofsmallest |
| Red.Blood.Cell.MinRadiusofsmalleste |
| Red.Blood.Cell.StdDevRadiusofsmalle |
| Red.Blood.Cell.MaxStdevChannel1 |
| Red.Blood.Cell.MeanStdevChannel1 |
| Red.Blood.Cell.MinStdevChannel1 |
| Red.Blood.Cell.StdDevStdevChannel1 |
| Red.Blood.Cell.MaxStdevChannel2 |
| Red.Blood.Cell.MeanStdevChannel2 |
| Red.Blood.Cell.MinStdevChannel2 |
| Red.Blood.Cell.StdDevStdevChannel2 |
| Red.Blood.Cell.MaxStdevChannel3 |
| Red.Blood.Cell.MeanStdevChannel3 |
| Red.Blood.Cell.MinStdevChannel3 |
| Red.Blood.Cell.StdDevStdevChannel3 |
| Red.Blood.Cell.MaxWidthPxl |
| Red.Blood.Cell.MeanWidthPxl |
| Red.Blood.Cell.MinWidthPxl |
| Red.Blood.Cell.StdDevWidthPxl |
| Stroma.Objects |
| Stroma.ObjectsPct |
| Stroma.MaxAreaPxl |
| Stroma.MeanAreaPxl |
| Stroma.MinAreaPxl |
| Stroma.StdDevAreaPxl |
| Stroma.SumAreaPxl |
| Stroma.MaxAsymmetry |
| Stroma.MeanAsymmetry |
| Stroma. MinAsymmetry |
| Stroma.StdDevAsymmetry |
| Stroma.MaxBorderlengthPxl |
| Stroma. MeanBorderlengthPxl |
| Stroma.MinBorderlengthPxl |
| Stroma. StdDevBorderlengthPxl |
| Stroma.SumBorderlengthPxl |
| Stroma.MaxBrightness |
| Stroma. Mean Brightness |
| Stroma.MinBrightness |
| Stroma. StdDev Brightness |
| Stroma.MaxCompactness |
| Stroma. Mean Compactness |
| Stroma.MinCompactness |
| Stroma.StdDevCompactness |
| Stroma.MaxDensity |

(continued)

| Feature |
| --- |
| Stroma.MeanDensity |
| Stroma.MinDensity |
| Stroma.StdDevDensity |
| Stroma.MaxDiff.ofenclosing.enclosed |
| Stroma.MeanDiff.ofenclosing.enclose |
| Stroma.MinDiff.ofenclosing.enclosed |
| Stroma. StdDevDiff.ofenclosing.enclo |
| Stroma.MaxEllipticFit |
| Stroma.MeanEllipticFit |
| Stroma.MinEllipticFit |
| Stroma.StdDevEllipticFit |
| Stroma.MaxLengthPxl |
| Stroma.MeanLengthPxl |
| Stroma.MinLengthPxl |
| Stroma.StdDevLengthPxl |
| Stroma.SumLengthPxl |
| Stroma. MaxMax.Diff. |
| Stroma.MeanMax.Diff. |
| Stroma.MinMax.Diff. |
| Stroma.StdDevMax.Diff. |
| Stroma.MaxMeanChannel 1 |
| Stroma.MeanMeanChannel1 |
| Stroma.MinMeanChannel 1 |
| Stroma.StdDevMeanChannel1 |
| Stroma.MaxMeanChannel2 |
| Stroma.MeanMeanChannel2 |
| Stroma.MinMeanChannel2 |
| Stroma.StdDevMeanChannel2 |
| Stroma.MaxMeanChannel3 |
| Stroma.MeanMeanChannel3 |
| Stroma.MinMeanChannel3 |
| Stroma.StdDevMeanChannel3 |
| Stroma. MaxRadiusoflargestenclosedel |
| Stroma.MeanRadiusoflargestenclosede |
| Stroma.MinRadiusoflargestenclosedel |
| Stroma. StdDevRadiusoflargestenclose |
| Stroma.MaxRadiusofsmallestenclosing |
| Stroma.MeanRadiusofsmallestenclosin |
| Stroma.MinRadiusofsmallestenclosing |
| Stroma. StdDev Radiusofsmallestenclos |
| Stroma.MaxStdevChannel 1 |
| Stroma.MeanStdevChannel 1 |
| Stroma.MinStdevChannel 1 |
| Stroma.StdDevStdevChannel 1 |
| Stroma.MaxStdevChannel2 |
| Stroma.MeanStdevChannel2 |
| Stroma.MinStdevChannel2 |
| Stroma.StdDevStdevChannel2 |
| Stroma.MaxStdevChannel3 |

(continued)

| Feature |
| --- |
| Stroma.MeanStdevChannel3 |
| Stroma.MinStdevChannel3 |
| Stroma.StdDevStdevChannel3 |
| Stroma.MaxWidthPxl |
| Stroma.MeanWidthPxl |
| Stroma.MinWidthPxl |
| Stroma.StdDevWidthPxl |
| Stroma.Nuclei .Objects |
| Stroma.Nuclei.ObjectsPct |
| Stroma.Nuclei.MaxAreaPxl |
| Stroma.Nuclei .MeanAreaPxl |
| Stroma.Nuclei.MinAreaPxl |
| Stroma.Nuclei.StdDevAreaPxl |
| Stroma.Nuclei.SumAreaPxl |
| Stroma.Nuclei.MaxAsymmetry |
| Stroma.Nuclei .MeanAsymmetry |
| Stroma.Nuclei.MinAsymmetry |
| Stroma.Nuclei.StdDevAsymmetry |
| Stroma.Nuclei.MaxBorderlengthPxl |
| Stroma.Nuclei.MeanBorderlengthPxl |
| Stroma.Nuclei.MinBorderlengthPxl |
| Stroma.Nuclei.StdDevBorderlengthPxl |
| Stroma.Nuclei.SumBorderlengthPxl |
| Stroma.Nuclei .MaxBrightness |
| Stroma.Nuclei .MeanBrightness |
| Stroma.Nuclei .MinBrightness |
| Stroma.Nuclei .StdDevBrightness |
| Stroma.Nuclei .MaxCompactness |
| Stroma.Nuclei .MeanCompactness |
| Stroma.Nuclei.MinCompactness |
| Stroma.Nuclei .StdDevCompactness |
| Stroma.Nuclei .MaxDensity |
| Stroma.Nuclei .MeanDensity |
| Stroma.Nuclei.MinDensity |
| Stroma. Nuclei .StdDevDensity |
| Stroma.Nuclei.MaxDiff.ofenclosing.e |
| Stroma.Nuclei .MeanDiff.ofenclosing. |
| Stroma.Nuclei .MinDiff.ofenclosing.e |
| Stroma.Nuclei .StdDevDiff.ofenclosin |
| Stroma.Nuclei.MaxEllipticFit |
| Stroma.Nuclei.MeanEllipticFit |
| Stroma.Nuclei .MinEllipticFit |
| Stroma.Nuclei.StdDevEllipticFit |
| Stroma.Nuclei.MaxLengthPxl |
| Stroma.Nuclei.MeanLengthPxl |
| Stroma.Nuclei.MinLengthPxl |
| Stroma.Nuclei.StdDevLengthPxl |
| Stroma. Nuclei .SumLengthPxl |
| Stroma.Nuclei .MaxMax.Diff. |

(continued)

| Feature |
| --- |
| Stroma.Nuclei.MeanMax.Diff. |
| Stroma.Nuclei .MinMax.Diff. |
| Stroma.Nuclei .StdDevMax.Diff. |
| Stroma.Nuclei.MaxMeanChannel1 |
| Stroma.Nuclei.MeanMeanChannel1 |
| Stroma.Nuclei.MinMeanChannel1 |
| Stroma.Nuclei.StdDevMeanChannel1 |
| Stroma.Nuclei.MaxMeanChannel2 |
| Stroma.Nuclei.MeanMeanChannel2 |
| Stroma.Nuclei.MinMeanChannel2 |
| Stroma.Nuclei.StdDevMeanChannel2 |
| Stroma.Nuclei.MaxMeanChannel3 |
| Stroma.Nuclei.MeanMeanChannel3 |
| Stroma.Nuclei.MinMeanChannel3 |
| Stroma.Nuclei.StdDevMeanChannel3 |
| Stroma.Nuclei.MaxRadiusoflargestenc |
| Stroma.Nuclei.MeanRadiusoflargesten |
| Stroma.Nuclei.MinRadiusoflargestenc |
| Stroma.Nuclei.StdDevRadiusoflargest |
| Stroma.Nuclei.MaxRadiusofsmallesten |
| Stroma.Nuclei.MeanRadiusofsmalleste |
| Stroma.Nuclei .MinRadiusofsmallesten |
| Stroma.Nuclei.StdDevRadiusofsmalles |
| Stroma.Nuclei.MaxStdevChannel1 |
| Stroma.Nuclei.MeanStdevChannel1 |
| Stroma.Nuclei.MinStdevChannel1 |
| Stroma.Nuclei.StdDevStdevChannel1 |
| Stroma.Nuclei.MaxStdevChannel2 |
| Stroma.Nuclei.MeanStdevChannel2 |
| Stroma.Nuclei.MinStdevChannel2 |
| Stroma.Nuclei.StdDevStdevChannel2 |
| Stroma.Nuclei.MaxStdevChannel3 |
| Stroma.Nuclei.MeanStdevChannel3 |
| Stroma.Nuclei.MinStdevChannel3 |
| Stroma.Nuclei.StdDevStdevChannel3 |
| Stroma.Nuclei.MaxWidthPx1 |
| Stroma.Nuclei.MeanWidthPxl |
| Stroma.Nuclei.MinWidthPxl |
| Stroma.Nuclei.StdDevWidthPxl |
| C2EN |
| EN2SN |
| L2Core |
| C2L |
| CEN2L |

**Table 2. Morphometric Features Script v2.0 (350 features)**

| Feature |
| --- |
| Artifact Mean Area Pxl |

(continued)

| Feature |
| --- |
| Artifact StdDev Area Pxl |
| Artifact Mean Asymmetry |
| Artifact StdDev Asymmetry |
| Artifact Mean Border index |
| Artifact StdDev Border index |
| Artifact Mean Border length Pxl |
| Artifact StdDev Border length Pxl |
| Artifact Mean Brightness |
| Artifact StdDev Brightness |
| Artifact Mean Compactness |
| Artifact StdDev Compactness |
| Artifact Mean Density |
| Artifact StdDev Density |
| Artifact Mean Diff. of enclosing/enclosed ellipse |
| Artifact StdDev Diff. of enclosing/enclosed ellipse |
| Artifact Mean Elliptic Fit |
| Artifact StdDev Elliptic Fit |
| Artifact Mean Length Pxl |
| Artifact StdDev Length Pxl |
| Artifact Mean Length/width |
| Artifact StdDev Length/width |
| Artifact Mean Main direction |
| Artifact StdDev Main direction |
| Artifact Mean Max.Diff. |
| Artifact StdDev Max.Diff. |
| Artifact Mean Mean Channel 1 |
| Artifact StdDev Mean Channel 1 |
| Artifact Mean Mean Channel 2 |
| Artifact StdDev Mean Channel 2 |
| Artifact Mean Mean Channel 3 |
| Artifact StdDev Mean Channel 3 |
| Artifact Mean Radius of largest enclosed ellipse |
| Artifact StdDev Radius of largest enclosed ellipse |
| Artifact Mean Radius of smallest enclosing ellipse |
| Artifact StdDev Radius of smallest enclosing ellipse |
| Artifact Mean Rectangular Fit |
| Artifact StdDev Rectangular Fit |
| Artifact Mean Shape index |
| Artifact StdDev Shape index |
| Artifact Mean Stddev Channel 1 |
| Artifact StdDev Stddev Channel 1 |
| Artifact Mean Stddev Channel 2 |
| Artifact StdDev Stddev Channel 2 |
| Artifact Mean Stddev Channel 3 |
| Artifact StdDev Stddev Channel 3 |
| Artifact Mean Width Pxl |
| Artifact StdDev Width Pxl |
| Cytoplasm Mean Area Pxl |
| Cytoplasm StdDev Area Pxl |

| Feature |
| --- |
| Cytoplasm Mean Asymmetry |
| Cytoplasm StdDev Asymmetry |
| Cytoplasm Mean Border index |
| Cytoplasm StdDev Border index |
| Cytoplasm Mean Border length Pxl |
| Cytoplasm StdDev Border length Pxl |
| Cytoplasm Mean Brightness |
| Cytoplasm StdDev Brightness |
| Cytoplasm Mean Compactness |
| Cytoplasm StdDev Compactness |
| Cytoplasm Mean Density |
| Cytoplasm StdDev Density |
| Cytoplasm Mean Diff. of enclosing/enclosed ellipse |
| Cytoplasm StdDev Diff. of enclosing/enclosed ellipse |
| Cytoplasm Mean Elliptic Fit |
| Cytoplasm StdDev Elliptic Fit |
| Cytoplasm Mean Length Pxl |
| Cytoplasm StdDev Length Pxl |
| Cytoplasm Mean Length/width |
| Cytoplasm StdDev Length/width |
| Cytoplasm Mean Main direction |
| Cytoplasm StdDev Main direction |
| Cytoplasm Mean Max.Diff. |
| Cytoplasm StdDev Max.Diff. |
| Cytoplasm Mean Mean Channel 1 |
| Cytoplasm StdDev Mean Channel 1 |
| Cytoplasm Mean Mean Channel 2 |
| Cytoplasm StdDev Mean Channel 2 |
| Cytoplasm Mean Mean Channel 3 |
| Cytoplasm StdDev Mean Channel 3 |
| Cytoplasm Mean Radius of largest enclosed ellipse |
| Cytoplasm StdDev Radius of largest enclosed ellipse |
| Cytoplasm Mean Radius of smallest enclosing ellipse |
| Cytoplasm StdDev Radius of smallest enclosing ellipse |
| Cytoplasm Mean Rectangular Fit |
| Cytoplasm StdDev Rectangular Fit |
| Cytoplasm Mean Shape index |
| Cytoplasm StdDev Shape index |
| Cytoplasm Mean Stddev Channel 1 |
| Cytoplasm StdDev Stddev Channel 1 |
| Cytoplasm Mean Stddev Channel 2 |
| Cytoplasm StdDev Stddev Channel 2 |
| Cytoplasm Mean Stddev Channel 3 |
| Cytoplasm StdDev Stddev Channel 3 |
| Cytoplasm Mean Width Pxl |
| Cytoplasm StdDev Width Pxl |
| Epithelial Nuclei Mean Area Pxl |
| Epithelial Nuclei StdDev Area Pxl |
| Epithelial Nuclei Mean Asymmetry |

(continued)

| Feature |
| --- |
| Epithelial Nuclei StdDev Asymmetry |
| Epithelial Nuclei Mean Border index |
| Epithelial Nuclei StdDev Border index |
| Epithelial Nuclei Mean Border length Pxl |
| Epithelial Nuclei StdDev Border length Pxl |
| Epithelial Nuclei Mean Brightness |
| Epithelial Nuclei StdDev Brightness |
| Epithelial Nuclei Mean Compactness |
| Epithelial Nuclei StdDev Compactness |
| Epithelial Nuclei Mean Density |
| Epithelial Nuclei StdDev Density |
| Epithelial Nuclei Mean Diff. of enclosing/enclosed ellipse |
| Epithelial Nuclei StdDev Diff. of enclosing/enclosed ellipse |
| Epithelial Nuclei Mean Elliptic Fit |
| Epithelial Nuclei StdDev Elliptic Fit |
| Epithelial Nuclei Mean Length Pxl |
| Epithelial Nuclei StdDev Length Pxl |
| Epithelial Nuclei Mean Length/width |
| Epithelial Nuclei StdDev Length/width |
| Epithelial Nuclei Mean Main direction |
| Epithelial Nuclei StdDev Main direction |
| Epithelial Nuclei Mean Max.Diff. |
| Epithelial Nuclei StdDev Max.Diff. |
| Epithelial Nuclei Mean Mean Channel 1 |
| Epithelial Nuclei StdDev Mean Channel 1 |
| Epithelial Nuclei Mean Mean Channel 2 |
| Epithelial Nuclei StdDev Mean Channel 2 |
| Epithelial Nuclei Mean Mean Channel 3 |
| Epithelial Nuclei StdDev Mean Channel 3 |
| Epithelial Nuclei Mean Radius of largest enclosed ellipse |
| Epithelial Nuclei StdDev Radius of largest enclosed ellipse |
| Epithelial Nuclei Mean Radius of smallest enclosing ellipse |
| Epithelial Nuclei StdDev Radius of smallest enclosing ellipse |
| Epithelial Nuclei Mean Rectangular Fit |
| Epithelial Nuclei StdDev Rectangular Fit |
| Epithelial Nuclei Mean Shape index |
| Epithelial Nuclei StdDev Shape index |
| Epithelial Nuclei Mean Stddev Channel 1 |
| Epithelial Nuclei StdDev Stddev Channel 1 |
| Epithelial Nuclei Mean Stddev Channel 2 |
| Epithelial Nuclei StdDev Stddev Channel 2 |
| Epithelial Nuclei Mean Stddev Channel 3 |
| Epithelial Nuclei StdDev Stddev Channel 3 |
| Epithelial Nuclei Mean Width Pxl |
| Epithelial Nuclei StdDev Width Pxl |
| Lumen Mean Area Pxl |
| Lumen StdDev Area Pxl |
| Lumen Mean Asymmetry |
| Lumen StdDev Asymmetry |

(continued)

| Feature |
|---|
| Lumen Mean Border index |
| Lumen StdDev Border index |
| Lumen Mean Border length Pxl |
| Lumen StdDev Border length Pxl |
| Lumen Mean Brightness |
| Lumen StdDev Brightness |
| Lumen Mean Compactness |
| Lumen StdDev Compactness |
| Lumen Mean Density |
| Lumen StdDev Density |
| Lumen Mean Diff. of enclosing/enclosed ellipse |
| Lumen StdDev Diff. of enclosing/enclosed ellipse |
| Lumen Mean Elliptic Fit |
| Lumen StdDev Elliptic Fit |
| Lumen Mean Length Pxl |
| Lumen StdDev Length Pxl |
| Lumen Mean Length/width |
| Lumen StdDev Length/width |
| Lumen Mean Main direction |
| Lumen StdDev Main direction |
| Lumen Mean Max.Diff. |
| Lumen StdDev Max.Diff. |
| Lumen Mean Mean Channel 1 |
| Lumen StdDev Mean Channel 1 |
| Lumen Mean Mean Channel 2 |
| Lumen StdDev Mean Channel 2 |
| Lumen Mean Mean Channel 3 |
| Lumen StdDev Mean Channel 3 |
| Lumen Mean Radius of largest enclosed ellipse |
| Lumen StdDev Radius of largest enclosed ellipse |
| Lumen Mean Radius of smallest enclosing ellipse |
| Lumen StdDev Radius of smallest enclosing ellipse |
| Lumen Mean Rectangular Fit |
| Lumen StdDev Rectangular Fit |
| Lumen Mean Shape index |
| Lumen StdDev Shape index |
| Lumen Mean Stddev Channel 1 |
| Lumen StdDev Stddev Channel 1 |
| Lumen Mean Stddev Channel 2 |
| Lumen StdDev Stddev Channel 2 |
| Lumen Mean Stddev Channel 3 |
| Lumen StdDev Stddev Channel 3 |
| Lumen Mean Width Pxl |
| Lumen StdDev Width Pxl |
| Stroma Mean Area Pxl |
| Stroma StdDev Area Pxl |
| Stroma Mean Asymmetry |
| Stroma StdDev Asymmetry |
| Stroma Mean Border index |

(continued)

| Feature |
|---|
| Stroma StdDev Border index |
| Stroma Mean Border length Pxl |
| Stroma StdDev Border length Pxl |
| Stroma Mean Brightness |
| Stroma StdDev Brightness |
| Stroma Mean Compactness |
| Stroma StdDev Compactness |
| Stroma Mean Density |
| Stroma StdDev Density |
| Stroma Mean Diff. of enclosing/enclosed ellipse |
| Stroma StdDev Diff. of enclosing/enclosed ellipse |
| Stroma Mean Elliptic Fit |
| Stroma StdDev Elliptic Fit |
| Stroma Mean Length Pxl |
| Stroma StdDev Length Pxl |
| Stroma Mean Length/width |
| Stroma StdDev Length/width |
| Stroma Mean Main direction |
| Stroma StdDev Main direction |
| Stroma Mean Max.Diff. |
| Stroma StdDev Max.Diff. |
| Stroma Mean Mean Channel 1 |
| Stroma StdDev Mean Channel 1 |
| Stroma Mean Mean Channel 2 |
| Stroma StdDev Mean Channel 2 |
| Stroma Mean Mean Channel 3 |
| Stroma StdDev Mean Channel 3 |
| Stroma Mean Radius of largest enclosed ellipse |
| Stroma StdDev Radius of largest enclosed ellipse |
| Stroma Mean Radius of smallest enclosing ellipse |
| Stroma StdDev Radius of smallest enclosing ellipse |
| Stroma Mean Rectangular Fit |
| Stroma StdDev Rectangular Fit |
| Stroma Mean Shape index |
| Stroma StdDev Shape index |
| Stroma Mean Stddev Channel 1 |
| Stroma StdDev Stddev Channel 1 |
| Stroma Mean Stddev Channel 2 |
| Stroma StdDev Stddev Channel 2 |
| Stroma Mean Stddev Channel 3 |
| Stroma StdDev Stddev Channel 3 |
| Stroma Mean Width Pxl |
| Stroma StdDev Width Pxl |
| Stroma Nuclei Mean Area Pxl |
| Stroma Nuclei StdDev Area Pxl |
| Stroma Nuclei Mean Asymmetry |
| Stroma Nuclei StdDev Asymmetry |
| Stroma Nuclei Mean Border index |
| Stroma Nuclei StdDev Border index |

(continued)

| Feature |
| --- |
| Stroma Nuclei Mean Border length Pxl |
| Stroma Nuclei StdDev Border length Pxl |
| Stroma Nuclei Mean Brightness |
| Stroma Nuclei StdDev Brightness |
| Stroma Nuclei Mean Compactness |
| Stroma Nuclei StdDev Compactness |
| Stroma Nuclei Mean Density |
| Stroma Nuclei StdDev Density |
| Stroma Nuclei Mean Diff. of enclosing/enclosed ellipse |
| Stroma Nuclei StdDev Diff. of enclosing/enclosed ellipse |
| Stroma Nuclei Mean Elliptic Fit |
| Stroma Nuclei StdDev Elliptic Fit |
| Stroma Nuclei Mean Length Pxl |
| Stroma Nuclei StdDev Length Pxl |
| Stroma Nuclei Mean Length/width |
| Stroma Nuclei StdDev Length/width |
| Stroma Nuclei Mean Main direction |
| Stroma Nuclei StdDev Main direction |
| Stroma Nuclei Mean Max.Diff. |
| Stroma Nuclei StdDev Max.Diff. |
| Stroma Nuclei Mean Mean Channel 1 |
| Stroma Nuclei StdDev Mean Channel 1 |
| Stroma Nuclei Mean Mean Channel 2 |
| Stroma Nuclei StdDev Mean Channel 2 |
| Stroma Nuclei Mean Mean Channel 3 |
| Stroma Nuclei StdDev Mean Channel 3 |
| Stroma Nuclei Mean Radius of largest enclosed ellipse |
| Stroma Nuclei StdDev Radius of largest enclosed ellipse |
| Stroma Nuclei Mean Radius of smallest enclosing ellipse |
| Stroma Nuclei StdDev Radius of smallest enclosing ellipse |
| Stroma Nuclei Mean Rectangular Fit |
| Stroma Nuclei StdDev Rectangular Fit |
| Stroma Nuclei Mean Shape index |
| Stroma Nuclei StdDev Shape index |
| Stroma Nuclei Mean Stddev Channel 1 |
| Stroma Nuclei StdDev Stddev Channel 1 |
| Stroma Nuclei Mean Stddev Channel 2 |
| Stroma Nuclei StdDev Stddev Channel 2 |
| Stroma Nuclei Mean Stddev Channel 3 |
| Stroma Nuclei StdDev Stddev Channel 3 |
| Stroma Nuclei Mean Width Pxl |
| Stroma Nuclei StdDev Width Pxl |
| Area of Artifact Pxl |
| Area of Cytoplasm Pxl |
| Area of Epithelial Nuclei Pxl |
| Area of Lumen Pxl |
| Area of Red Blood Cell Pxl |
| Area of Stroma Pxl |
| Area of Stroma Nuclei Pxl |

(continued)

| Feature |
| --- |
| Number of objects of Artifact |
| Number of objects of Cytoplasm |
| Number of objects of Epithelial Nuclei |
| Number of objects of Lumen |
| Number of objects of Red Blood Cell |
| Number of objects of Stroma |
| Number of objects of Stroma Nuclei |
| Red Blood Cell Mean Area Pxl |
| Red Blood Cell StdDev Area Pxl |
| Red Blood Cell Mean Asymmetry |
| Red Blood Cell StdDev Asymmetry |
| Red Blood Cell Mean Border index |
| Red Blood Cell StdDev Border index |
| Red Blood Cell Mean Border length Pxl |
| Red Blood Cell StdDev Border length Pxl |
| Red Blood Cell Mean Brightness |
| Red Blood Cell StdDev Brightness |
| Red Blood Cell Mean Compactness |
| Red Blood Cell StdDev Compactness |
| Red Blood Cell Mean Density |
| Red Blood Cell StdDev Density |
| Red Blood Cell Mean Diff. of enclosing/enclosed ellipse |
| Red Blood Cell StdDev Diff. of enclosing/enclosed ellipse |
| Red Blood Cell Mean Elliptic Fit |
| Red Blood Cell StdDev Elliptic Fit |
| Red Blood Cell Mean Length Pxl |
| Red Blood Cell StdDev Length Pxl |
| Red Blood Cell Mean Length/width |
| Red Blood Cell StdDev Length/width |
| Red Blood Cell Mean Main direction |
| Red Blood Cell StdDev Main direction |
| Red Blood Cell Mean Max.Diff. |
| Red Blood Cell StdDev Max.Diff. |
| Red Blood Cell Mean Mean Channel 1 |
| Red Blood Cell StdDev Mean Channel 1 |
| Red Blood Cell Mean Mean Channel 2 |
| Red Blood Cell StdDev Mean Channel 2 |
| Red Blood Cell Mean Mean Channel 3 |
| Red Blood Cell StdDev Mean Channel 3 |
| Red Blood Cell Mean Radius of largest enclosed ellipse |
| Red Blood Cell StdDev Radius of largest enclosed ellipse |
| Red Blood Cell Mean Radius of smallest enclosing ellipse |
| Red Blood Cell StdDev Radius of smallest enclosing ellipse |
| Red Blood Cell Mean Rectangular Fit |
| Red Blood Cell StdDev Rectangular Fit |
| Red Blood Cell Mean Shape index |
| Red Blood Cell StdDev Shape index |
| Red Blood Cell Mean Stddev Channel 1 |
| Red Blood Cell StdDev Stddev Channel 1 |

(continued)

| Feature |
| --- |
| Red Blood Cell Mean Stddev Channel 2 |
| Red Blood Cell StdDev Stddev Channel 2 |
| Red Blood Cell Mean Stddev Channel 3 |
| Red Blood Cell StdDev Stddev Channel 3 |
| Red Blood Cell Mean Width Pxl |
| Red Blood Cell StdDev Width Pxl |

**Table 17. Morphometric Features - Script v5.0 (38 features)**

| Feature Name | Description |
| --- | --- |
| CytoplasmMeanMeanChannel40058 | Mean cytoplasm intensity value in the red color channel |
| CytoplasmMeanMeanChannel50059 | Mean cytoplasm intensity value in the green color channel |
| CytoplasmMeanMeanChannel60060 | Mean cytoplasm intensity value in the blue color channel |
| CytoplasmMeanStddevChannel40066 | Mean cytoplasm intensity standard deviation in the red color channel. |
| CytoplasmMeanStddevChannel50067 | Mean cytoplasm intensity standard deviation in the green color channel |
| CytoplasmMeanStddevChannel60068 | Mean cytoplasm intensity standard deviation in the blue color channel |
| CytoplasmStddevMeanChannel40081 | Standard deviation of cytoplasm intensity in the red color channel |
| CytoplasmStddevMeanChannel50082 | Standard deviation of cytoplasm intensity in the green color channel |
| CytoplasmStddevMeanChannel60083 | Standard deviation of cytoplasm intensity in the blue color channel |
| EpithelialNucleiMeanAreaPxl0101 | Mean value of the epithelial nuclei area, pixels |
| EpithelNucleiMeanMeanChanne40112 | Mean epithelial nuclei intensity in the red color channel |
| EpithelNucleiMeanMeanChanne50113 | Mean epithelial nuclei intensity in the green color channel |
| EpithelNucleiMeanMeanChanne60114 | Mean epithelial nuclei intensity in the blue color channel |
| EpitheNucleiMeanStddevChann40120 | Mean epithelial nuclei intensity standard deviation in the red color channel |
| EpitheNucleiMeanStddevChann50121 | Mean epithelial nuclei intensity standard deviation in the green color channel |
| EpitheNucleiMeanStddevChann60122 | Mean epithelial nuclei intensity standard deviation in the blue color channel |
| EpitheliaNucleiStddevAreaPxl0124 | Standard deviation of the epithelial nuclei area |
| EpitheNucleiStddevMeanChann40135 | Standard deviation of epithelial nuclei intensity in the red color channel |
| EpitheNucleiStddevMeanChann50136 | Standard deviation of epithelial nuclei intensity in the green color channel |
| EpitheNucleiStddevMeanChann60137 | Standard deviation of epithelial nuclei intensity in the blue color channel |
| StromaMeanMeanChannel40262 | Mean stroma intensity in the red color channel |
| StromaMeanMeanChannel50263 | Mean stroma intensity in the green color channel |
| StromaMeanMeanChannel60264 | Mean stroma intensity in the blue color channel |
| StromaMeanStddevChannel40270 | Mean stroma intensity standard deviation in the red color channel |
| StromaMeanStddevChannel50271 | Mean stroma intensity standard deviation in the green color channel |
| StromaMeanStddevChannel60272 | Mean stroma intensity standard deviation in the blue color channel |
| St$_T$omaStddevMeanChannel40331 | Standard deviation of stroma intensity in the red color channel |
| StromaStddevMeanChannel50332 | Standard deviation of stroma intensity in the green color channel |
| StromaStddevMeanChannel60333 | Standard deviation of stroma intensity in the blue color channel |
| AreaOfCytoplasmPxl0345 | Total area of cytoplasm, pixels |
| AreaOfEpithelialNucleiPxl0350 | Total area of epithelial nuclei, pixels |
| AreaOfLumenPxl10357 | Total area of lumens, pixels |
| NumberOfObjectOfEpitheNuclei0364 | Total number of epithelial nuclei objects |
| AreaCytopdivTotTissueArea | Relative area of cytoplasm with respect to the tissue area, % |
| AreaEpitNucdivTotTissueArea | Relative area of epithelial nuclei with respect to the tissue area, % |

(continued)

| Feature Name | Description |
|---|---|
| AreaLumendivTotTissueArea | Relative area of lumens with respect to the tissue area, % |
| AreaStromadivTotTissueArea | Relative area of stroma with respect to the tissue area, % |
| NumObjEpitNucdivTotNumberNuc | Relative number of epithelial nuclei with respect to the total number of nuclei |

**Table 18. Morphometric Features**

| Feature Name | Description |
|---|---|
| CytoplasmMeanMeanChannel40058 | Mean of cytoplasm intensity mean value with the red filter |
| CytoplasmMeanMeanChannel50059 | Mean of cytoplasm intensity mean value with the green filter |
| CytoplasmMeanMeanChannel60060 | Mean of cytoplasm intensity mean value with the blue filter |
| CytoplasmMeanStddevChannel40066 | Mean of cytoplasm intensity standard deviation with the red filter |
| CytoplasmMeanStddevChannel50067 | Mean of cytoplasm intensity standard deviation with the green filter |
| CytoplasmMeanStddevChannel60068 | Mean of cytoplasm intensity standard deviation with the blue filter |
| CytoplasmStddevMeanChannel40081 | Standard deviation of the mean cytoplasm intensity with the red filter |
| CytoplasmStddevMeanChannel50082 | Standard deviation of the mean cytoplasm intensity with the green filter |
| CytoplasmStddevMeanChannel60083 | Standard deviation of the mean cytoplasm intensity with the blue filter |
| EpithelNucleiMeanMeanChanne40112 | Mean of epithelial nuclei intensity with the red filter |
| EpithelNucleiMeanMeanChanne50113 | Mean of epithelial nuclei intensity with the green filter |
| EpithelNucleiMeanMeanChanne60114 | Mean of epithelial nuclei intensity with the blue filter |
| EpitheNucleiMeanStddevChann40120 | Mean of epithelial nuclei intensity standard deviation with the red filter |
| EpitheNucleiMeanStddevChann50121 | Mean of epithelial nuclei intensity standard deviation with the green filter |
| EpitheNucleiMeanStddevChann60122 | Mean of epithelial nuclei intensity standard deviation with the blue filter |
| EpitheliaNucleiStddevAreaPxl0124 | Standard deviation of the epithelial nuclei area |
| EpitheNucleiStddevMeanChann40135 | Standard deviation of the mean epithelial nuclei intensity with the red filter |
| EpitheNucleiStddevMeanChann50136 | Standard deviation of the mean epithelial nuclei intensity with the green filter |
| EpitheNucleiStddevMeanChann60137 | Standard deviation of the mean epithelial nuclei intensity with the blue filter |
| StromaMeanMeanChannel40262 | Mean of stroma intensity with the red filter |
| StromaMeanMeanChannel50263 | Mean of stroma intensity with the green filter |
| StromaMeanMeanChannel60264 | Mean of stroma intensity with the blue filter |
| StromaMeanStddevChannel40270 | Mean of stroma intensity standard deviation with the red filter |
| StromaMeanStddevChannel50271 | Mean of stroma intensity standard deviation with the green filter |
| StromaMeanStddevChannel60272 | Mean of stroma intensity standard deviation with the blue filter |
| StromaStddevMeanChannel40331 | Standard deviation of the mean stroma intensity with the red filter |
| StromaStddevMeanChannel50332 | Standard deviation of the mean stroma intensity with the green filter |
| StromaStddevMeanChannel60333 | Standard deviation of the stroma intensity with the blue filter |
| AreaCytopdivTotTissueArea | Area of cytoplasm relative to the tissue area, % |
| AreaEpitNucdivTotTissueArea | Area of epithelial nuclei relative to the tissue area, % |
| AreaLumendivTotTissueArea | Area of lumen relative to the tissue area, % |
| AreaRBCdivTotTissueArea | Area of red blood cells relative to the tissue area, % |
| AreaStromadivTotTissueArea | Area of stroma relative to the tissue area, % |

**Table 19. Example 8 Clinical Information**

| Characteristic | Training | Validation |
|---|---|---|
| N | 342 | 340 |
| Pre-operative PSA (ng/mL) | | |
| Mean | 10.3 | 10.8 |

(continued)

| Pre-operative PSA (ng/mL) | | |
|---|---|---|
| Median | 7.5 | 7.9 |
| Range | 0.8-68.5 | 1.01-00.0 |
| **Lymph Node Involvement** | | |
| Negative | 339 (99.1%) | 335 (98.5%) |
| Positive | 3 (0.9%) | 5 (1.5%) |
| **Seminal Vesicle Involvement** | | |
| No | 320 (93.6%) | 321 (94.4%) |
| Yes | 22 (6.4%) | 19 (5.6%) |
| **Surgical Margins** | | |
| Negative | 219 (64.0%) | 240 (71.0%) |
| Positive | 123 (36.0%) | 100 (29.0%) |
| **Extracapsular Involvement** | | |
| No | 245 (71.6.0%) | 254 (74.7%) |
| Yes | 97 (28.4%) | 86 (25.3%) |
| **Dominant Biopsy Gleason Grade** | | |
| 1 | 1 (0.3%) | 2 (0.6%) |
| 2 | 17 (5.0%) | 23 (6.8%) |
| 3 | 279 (81.6%) | 273 (80.3%) |
| 4 | 45 (13.2%) | 42 (12.4%) |
| 5 | 0 (0.0%) | 0 (0.0%) |
| **Biopsy Gleason Score** | | |
| 2 | 0 (0.0%) | 1 (0.3%) |
| 3 | 1 (0.3%) | 2 (0.6%) |
| 4 | 11 (3.2%) | 8 (2.4%) |
| 5 | 23 (6.7%) | 30 (8.8%) |
| 6 | 190 (55.6%) | 187 (55.0%) |
| 7 | 101 (29.5%) | 94 (27.7%) |
| 8 | 15 (4.4%) | 13 (3.8%) |
| 9 | 1 (0.3%) | 5 (1.5%) |
| **Dominant Specimen Gleason Grade** | | |
| 2 | 8 (2.3%) | 5 (1.5%) |
| 3 | 283 (82.8%) | 276 (81.2%) |
| 4 | 49 (14.3%) | 55 (16.2%) |
| 5 | 2 (0.6%) | 4 (1.2%) |
| **Specimen Gleason Score** | | |
| 5 | 9 (2.6%) | 15 (4.4%) |
| 6 | 116 (33.9%) | 110 (32.4%) |
| 7 | 193 (56.4%) | 187 (55.0%) |
| 8 | 18 (5.3%) | 17 (5.0%) |
| 9 | 6 (1.8%) | 10 (2.9%) |
| 10 | 0 (0.0%) | 1 (0.3%) |

**Table 20. Example 9 Features Evaluated and Selected**

| Feature | Aggregating function | Univariate CI* | Weight in CF model |
|---|---|---|---|
| **Clinical Features** | | | |
| Clinical TNM stage | | 032917 | |
| Preoperative PSA | | 030974 | |
| Dominant biopsy Gleason grade | | 03265 | |

(continued)

| Feature | Aggregating function | Univariate CI* | Weight in CF model |
|---|---|---|---|
| **Clinical Features** | | | |
| Biopsy Gleason score | | 031104 | |
| Dominant prostatectomy Gleason grade | | 0.26804 | -16.4 |
| Prostatectomy Gleason sum | | 031311 | |
| Extracapsular extension | | 03358 | |
| Seminal vesicle status | | 037022 | |
| Lymph node involvement | | 0.42033 | -25.938 |
| Surgical margin status | | 037596 | |
| | | | |
| **Morphometric Features** | | | |
| Relative area of epi nuclei | | 03778 | |
| Epithelial nuclear texture feature 1 norm | | 033503 | |
| Epithelial nuclear texture feature 2 | | 039107 | |
| Epithelial nuclear texture feature 3 | | 036808 | |
| Cytoplasm texture feature 1 | | 0.72432 | |
| Cytoplasm texture feature 2 | | 0.74648 | 20.728 |
| Cytoplasm texture feature 3 | | 0.73593 | |
| Relative area of lumen | | 0.67302 | 8.9663 |
| Lumen mean borderlength | | 0.7646 | 17.217 |
| Isolated epithelial nuclei density | | 030897 | |
| Well defined epithelial nuclei | | 034273 | |
| Isolated epithelial nuclear area Norm | | 0.3098 | |
| | | 0.42957 | |
| **Molecular (Imunofluorescence) Features** | | | |
| Relative area of epi nuclei | min | 0.54152 | |
| Relative area of AR+ epi nuclei | mean | 0.3778 | |
| Relative area of AMACR+ epi cells | max | 0.45243 | |
| Relative area AR+AMACR+/ AR+ epi nuclei | max | 0.50219 | |
| Relative area AR+AMACR+/ epi nuclei | max | 0.4362 | |
| Normalized sum AR Intensity Bin 3-9/total nuclear area | mean | 0.33266 | |
| Normalized sum AR Intensity Bin 1-2/total nuclear area | max | 0.60088 | |
| Normalized sum AR Intensity Bin 3-5/total nuclear area | mean | 0.38799 | |
| Normalized sum AR Intensity Bin 6-10/total nuclear area | min | 0.45605 | |
| Mean AR Intensity in AMACR- epi nuclei | mean | 0.34475 | -5.2075 |
| Mean AR Intensity in AMACR+ epi nuclei | mean | 0.30174 | |
| Total AR Intensity in epi nuclei | mean | 0.32532 | |
| Normalized AR Intensity in epi nuclei | mean | 0.3406 | |
| Total AR Intensity in AMACR+ epi nuclei | max | 0.42957 | |
| Total AR Intensity in AMACR - epi nuclei | max | 0.44616 | |
| Total AR Intensity x area, normalized | mean | 0.34167 | |
| Total AR positive Intensity x area, normalized | mean | 0.33562 | |
| Total area of AR+ intensity within epi nuclei | mean | 0.37898 | |

* Concordance index as a single-variable predictor of clinical failure in the training set.

**Table 21. Example 9 Clinical characteristics of the patients**

| | Training | Training | Validation | Validation |
|---|---|---|---|---|
| **Number of Patients** | 373 | | 385 | |
| **Preoperative PSA** | Frequency | Percent (%) | Frequency | Percent (%) |
| <4 | 26 | 6.97 | 30 | 7.79 |
| [4,10) | 216 | 57.91 | 215 | 55.84 |
| [10,20) | 92 | 24.66 | 101 | 26.23 |
| >20 | 39 | 10.46 | 39 | 10.13 |
| **Clinical TNM stage** | Frequency | Percent | Frequency | Percent |
| 1 | 3 | 0.8 | 4 | 1.04 |
| 2 | 187 | 50.13 | 187 | 48.57 |
| 3 | 69 | 18.5 | 61 | 15.84 |
| 4 | 36 | 9.65 | 30 | 7.79 |
| 5 | 72 | 19.3 | 98 | 25.45 |
| 6 | 6 | 1.61 | 5 | 1.3 |
| **Biopsy Gleason score** | Frequency | Percent | Frequency | Percent |
| 2 | 0 | 0 | 1 | 0.26 |
| 3 | 1 | 0.27 | 2 | 0.52 |
| 4 | 11 | 2.95 | 9 | 2.34 |
| 5 | 23 | 6.17 | 31 | 8.05 |
| 6 | 202 | 54.16 | 205 | 53.25 |
| 7 | 113 | 30.29 | 108 | 28.05 |
| 8 | 22 | 5.9 | 23 | 5.97 |
| 9 | 1 | 0.27 | 6 | 1.56 |
| **Prostatectomy Gleason sum** | Frequency | Percent | Frequency | Percent |
| 5 | 9 | 2.41 | 16 | 4.16 |
| 6 | 119 | 31.9 | 116 | 30.13 |
| 7 | 214 | 57.37 | 212 | 55.06 |
| 8 | 21 | 5.63 | 23 | 5.97 |
| 9 | 10 | 2.68 | 17 | 4.42 |
| 10 | 0 | 0 | 1 | 0.26 |
| **Seminal vesicle status** | Frequency | Percent | Frequency | Percent |
| 0 | 342 | 91.69 | 349 | 90.65 |
| 1 | 31 | 8.31 | 36 | 9.35 |
| **Surgical margin status** | Frequency | Percent | Frequency | Percent |
| 0 | 234 | 62.73 | 258 | 67.01 |
| 1 | 139 | 37.27 | 127 | 32.99 |
| **Extracapsular extension** | Frequency | Percent | Frequency | Percent |
| 0 | 254 | 68.1 | 276 | 71.69 |
| 1 | 29 | 7.77 | 33 | 8.57 |
| 2 | 90 | 24.13 | 76 | 19.74 |
| **Lymph node involvement** | Frequency | Percent | Frequency | Percent |
| 0 | 364 | 97.59 | 373 | 96.88 |
| 1 | 9 | 2.41 | 12 | 3.12 |
| **censored** | Frequency | Percent | Frequency | Percent |
| 0 (no CF yet) | 340 | 91.15 | 356 | 92.47 |
| 1 (CF) | 33 | 8.85 | 29 | 7.53 |

**Table 22. Morphometric Features - Script v6.0 (27 features)**

| Feature Name | Description |
| --- | --- |
| Cytoplasm Mean MeanChannel40058 | Mean of cytoplasm intensity mean value with the red filter |
| Cytoplasm Mean MeanChannel50059 | Mean of cytoplasm intensity mean value with the green filter |
| Cytoplasm Mean MeanChannel60060 | Mean of cytoplasm intensity mean value with the blue filter |
| CytoplasmMeanStddevChannel40066 | Mean of cytoplasm intensity standard deviation with the red filter |
| CytoplasmMeanStddevChannel50067 | Mean of cytoplasm intensity standard deviation with the green filter |
| CytoplasmMeanStddevChannel60068 | Mean of cytoplasm intensity standard deviation with the blue filter |
| CytoplasmStddevMeanChannel40081 | Standard deviation of the mean cytoplasm intensity with the red filter |
| CytoplasmStddevMeanChannel50082 | Standard deviation of the mean cytoplasm intensity with the green filter |
| CytoplasmStddevMeanChannel60083 | Standard deviation of the mean cytoplasm intensity with the blue filter |
| EpitheNucleiMeanStddevChann40142 | Mean of epithelial nuclei intensity standard deviation with the red filter |
| EpitheNucleiMeanStddevChann50143 | Mean of epithelial nuclei intensity standard deviation with the green filter |
| EpitheNucleiMeanStddevChann60144 | Mean of epithelial nuclei intensity standard deviation with the blue filter |
| EpitheNucleiStddevMeanChann40157 | Standard deviation of the mean epithelial nuclei intensity with the red filter |
| EpitheNucleiStddevMeanChann50158 | Standard deviation of the mean epithelial nuclei intensity with the green filter |
| EpitheNucleiStddevMeanChann60159 | Standard deviation of the mean epithelial nuclei intensity with the blue filter |
| StromaMeanStddevChannel40310 | Mean of stroma intensity standard deviation with the red filter |
| StromaMeanStddevChannel50311 | Mean of stroma intensity standard deviation with the green filter |
| StromaMeanStddevChannel60312 | Mean of stroma intensity standard deviation with the blue filter |
| AreaCytopdivTotTissueArea | Area of cytoplasm relative to the tissue area, % |
| Area EpitNucdivTotTissueArea | Area of epithelial nuclei relative to the tissue area, % |
| AreaLumendivTotTissueArea | Area of lumen relative to the tissue area, % |
| AreaStromadivTotTissueArea | Area of stroma relative to the tissue area, % |
|  | Nuclei density is defined as relative area of the epithelial nuclei within circle centered at image object and with radius of 27 pixels. The image objects were classified on to 10 bins based on the value of the nuclei density. For every bin total areas of objects comprising the bin were calculated. |
| AreaOfLowNucleiDensityNorm | The feature represents relative area of the image objects having up to 40% of the epithelial nuclei area within 27 pixel circle. |
| AreaOfMedNucleiDensityNorm | The feature represents relative area of the image objects having more than 40% and less than 60% of the epithelial nuclei area within 27 pixel circle. |
| AreaOfHighNucleiDensityNorm | The feature represents relative area of the image objects having more than 60% of the epithelial nuclei area within 27 pixel circle. |
| DensityPercent_Bins3_4 | Relative area of the image object having more than 20% and less than 40% of epithelial nuclei within circle of 27 pixel radius |
| DensityPercent_Bin1 | Relative area of the image object having less than 10% of epithelial nuclei within circle of 27 pixel radius |

**Claims**

1. Apparatus for evaluating a risk of prostate cancer recurrence in a patient, the apparatus comprising:

   (a) one or more data storage devices configured to store a patient dataset and a population dataset, wherein the population dataset includes data relating to patients where the final health outcome was known (uncensored) and data relating to patients where the final health outcome was unknown (censored); and both the population dataset and patient dataset include a common set of data values corresponding to at least a biopsy Gleason score; seminal vesicle invasion; extracapsular extension; preoperative PSA; dominant prostatectomy Gleason grade; the relative area of AR+ epithelial nuclei; a morphometric measurement of the texture of tumour epithelial nuclei and epithelial cytoplasm, wherein the data in at least the patient dataset is derived in part from an image of a tissue sample subject to immunofluorescence; and
(b) a computer configured to:

(i) generate, from the population dataset, a prognostic application configured to output one or more values corresponding to the risk of prostate recurrence in a patient by performing support vector regression utilizing a first loss function on the common set of data values in the censored dataset and support vector regression utilizing a second loss function on the common set of data values in the uncensored dataset, and store the prognostic application within the memory of the computer;
(ii) access the patient dataset;
(iii) evaluate the common set of data values in the patient dataset with the prognostic application to generate a value corresponding to the probability of prostate cancer reoccurrence in the patient, and
(iv) generate a digital file suitable for transmission to a remote computer system that includes at least the subject data and the one or more values indicative of the risk of prostate recurrence,

**characterised in that**
the tissue sample image is analyzed using at least a quad-tree image analysis function and multiresolution re-segmentation to differentiate the tissue sample image into background and non-background objects, where the background objects have an average pixel intensity below a predetermined threshold, and wherein the non-background objects comprise biomarker compartments within which biomarker signals are discriminated from background by intensity thresholding, wherein the intensity thresholds for signal discrimination are a function of image characteristics of the background objects.

2. The apparatus of claim 1, wherein the population dataset and patient dataset include at least one additional morphometric measurement.

3. The apparatus of claim 2, wherein the population dataset and patient dataset includes a value corresponding to the average intensity of AR in AR+/AMACR- epithelial nuclei.

**Patentansprüche**

1. Vorrichtung zur Bewertung eines Risikos für das Wiederauftreten von Prostatakrebs bei einem Patienten, wobei die Vorrichtung Folgendes umfasst:

a) eine oder mehrere Datenspeichervorrichtungen, die konfiguriert sind, einen Patientendatensatz und einen Populationsdatensatz zu speichern, wobei der Populationsdatensatz Daten betreffend Patienten, bei denen der endgültige Gesundheitszustand bekannt war (unzensuriert), und Daten betreffend Patienten, bei denen der endgültige Gesundheitszustand unbekannt war (zensuriert), umfasst; und wobei sowohl der Populationsdatensatz als auch der Patientendatensatz einen gemeinsamen Satz von Datenwerten umfassen, die Folgenden entsprechen: zumindest einem Biopsie-Gleason-Score; Invasion der Samenblase; extrakapsulärer Ausdehnung; präoperativem PSA-Wert; dominantem Prostatektomie-Gleason-Grad; relativer Fläche von AR+-Epithelkernen; morphometrischer Messung der Struktur von Tumorepithelkernen und Epithel-Zytoplasma, wobei die Daten in zumindest dem Patientendatensatz teilweise von einem Bild einer Gewebeprobe abgeleitet ist, die Immunfluoreszenz ausgesetzt wurde; und
b) einen Computer, der für Folgendes konfiguriert ist:

i) anhand des Populationsdatensatzes Erzeugen einer Prognose-Anwendung, die konfiguriert ist, einen oder mehrere Werte auszugeben, die dem Risiko für das Wiederauftreten von Prostatakrebs bei einem Patienten entsprechen, durch Durchführen von Stützvektorregression unter Verwendung einer ersten Verlustfunktion am gemeinsamen Satz von Datenwerten im zensurierten Datensatz und von Stützvektorregression unter Verwendung einer zweiten Verlustfunktion am gemeinsamen Satz von Datenwerten im unzensurierten Datensatz, und Speichern der Prognose-Anwendung im Speicher des Computers;

ii) Zugreifen auf den Patientendatensatz;

iii) Bewerten des gemeinsamen Satzes von Datenwerten im Patientendatensatz mit der Prognose-Anwendung, um einen Wert zu erzeugen, der der Wahrscheinlichkeit für das Wiederauftreten von Prostatakrebs beim Patienten entspricht, und

iv) Erzeugen einer digitalen Datei, die zur Übertragung an ein Ferncomputersystem geeignet ist und zumindest die Daten der Individuen sowie des einen oder der mehreren Werte umfasst, die auf das Risiko für das Wiederauftreten von Prostatakrebs hindeuten,

**dadurch gekennzeichnet, dass**

das Bild der Gewebeprobe unter Verwendung von zumindest einer Quadtree-Bildanalysefunktion und Resegmentierung mit mehrfacher Auflösung analysiert wird, um das Bild der Gewebeprobe in Hintergrund- und Nichthintergrundobjekte zu unterteilen, wobei die Hintergrundobjekte eine mittlere Pixelintensität unterhalb einer vordefinierten Schwelle aufweisen und die Nichthintergrundobjekte Biomarkerbereiche aufweisen, in denen Biomarkersignale durch Intensitätsschwellenwertbildung vom Hintergrund unterschieden werden, wobei die Intensitätsschwellenwerte zur Signalunterscheidung eine Funktion von Bildeigenschaften der Hintergrundobjekte sind.

2. Vorrichtung nach Anspruch 1, wobei der Populationsdatensatz und der Patientendatensatz zumindest eine zusätzliche morphometrische Messung umfassen.

3. Vorrichtung nach Anspruch 2, wobei der Populationsdatensatz und der Patientendatensatz einen Wert umfasst, der der mittleren Intensität von AR in AR+/AMACR-Epithelkernen entspricht.

**Revendications**

1. Appareil pour évaluer un risque de récidive du cancer de la prostate chez un patient, l'appareil comprenant :

(a) un ou plusieurs dispositifs de stockage de données configurés pour stocker un ensemble de données de patient et un ensemble de données de population, dans lequel l'ensemble de données de population comprend des données relatives à des patients dont le résultat de santé final était connu (non censuré) et des données relatives à des patients dont le résultat de santé final était inconnu (censuré) ; et à la fois l'ensemble de données de population et l'ensemble de données de patient comprennent un ensemble commun de valeurs de données correspondant à au moins un score de Gleason de biopsie ; une invasion des vésicules séminales ; une extension extracapsulaire ; un PSA préopératoire ; un grade de Gleason dominant de la prostatectomie ; l'aire relative de noyaux épithéliaux AR+ ; une mesure morphométrique de la texture de noyaux épithéliaux tumoraux et du cytoplasme épithélial, dans lequel les données dans au moins l'ensemble de données de patient sont dérivées en partie d'une image d'un échantillon de tissu soumis à une immunofluorescence ; et
(b) un ordinateur configuré pour :

(i) générer, à partir de l'ensemble de données de population, une application de pronostique configurée pour produire une ou plusieurs valeurs correspondant au risque de récidive du cancer de la prostate chez un patient en effectuant une régression de vecteur de soutien en utilisant une première fonction de perte sur l'ensemble commun de valeurs de données dans l'ensemble de données censuré et une régression de vecteur de soutien en utilisant une seconde fonction de perte sur l'ensemble commun de valeurs de données dans l'ensemble de données non censuré, et stocker l'application de pronostique dans la mémoire de l'ordinateur ;
(ii) accéder à l'ensemble de données de patient ;
(iii) évaluer l'ensemble commun de valeurs de données dans l'ensemble de données de patient avec l'application de pronostique pour générer une valeur correspondant à la probabilité de récidive du cancer de la prostate chez le patient, et
(iv) générer un fichier numérique apte à être transmis à un système informatique distant qui comprend au moins les données de sujet et les une ou plusieurs valeurs indicatives du risque de récidive du cancer de la prostate,

**caractérisé en ce que**
l'image d'échantillon de tissu est analysée en utilisant au moins une fonction d'analyse d'image à quatre arbres et une re-segmentation multirésolution pour différencier l'image d'échantillon de tissu en objets d'arrière-plan et non d'arrière-plan, où les objets d'arrière-plan ont une intensité de pixel moyenne inférieure à un seuil prédéterminé, et dans lequel les objets non d'arrière-plan comprennent des compartiments de biomarqueur dans lesquels les signaux de biomarqueur sont discriminés de l'arrière-plan par seuillage d'intensité, dans lequel les seuils d'intensité pour une discrimination de signal sont fonction de caractéristiques d'image des objets d'arrière-plan.

2. Appareil selon la revendication 1, dans lequel l'ensemble de données de population et l'ensemble de données de patient comprennent au moins une mesure morphométrique supplémentaire.

3. Appareil selon la revendication 2, dans lequel l'ensemble de données de population et l'ensemble de données de patient comprennent une valeur correspondant à l'intensité moyenne de AR dans des noyaux épithéliaux AR+/AMA-CR-.

# FIG. 1A

104

Diagnostics Facility

Predictive
Model — 102

— 110

Internet
Service
Provider — 108

— 112

Remote access
device with
data for a
patient — 106

# FIG. 1B

124

Facility (e.g., hospital or physician's office)

Test kit including predictive model ~ 122

# FIG. 1C

# FIG. 2

Predicted Risk of Outcome for Edward Jones Over Time
BioScore= 520

High Risk (Patient Edward Jones)

Intermediate Risk

Low Risk

Time to Outcome in Months

—— Patient BioScore < 490 (Low Risk)
--- Patient BioScore 490-509 (Intermediate Risk)
—— Patient BioScore > 509 (High Risk)

EP 2 145 276 B1

# FIG. 3

# FIG. 4

400

```
┌─────────────────────┐
│  Obtain a first     │
│  dataset            │
│  including at least │
│  one of clinical,   │  ～ 402
│  morphometric and   │
│  molecular data     │
│  for a patient      │
└─────────────────────┘
          │
          ▼
┌─────────────────────┐
│  Administer a test  │
│  compound           │  ～ 404
│  to the patient     │
└─────────────────────┘
          │
          ▼
┌─────────────────────┐
│  Obtain a second    │  ～ 406
│  dataset            │
│  from the patient   │
└─────────────────────┘
          │
          ▼
┌─────────────────────┐
│  Compare the first  │
│  and                │
│  second datasets,   │
│  where a            │
│  change in the      │  ～ 408
│  datasets           │
│  indicates that the │
│  test               │
│  compound is an     │
│  inhibitor          │
└─────────────────────┘
```

# FIG. 5A

Image of healthy prostate tissue

Prostate Gland and Lumen

Epithelial Cells (cytoplasm and nuclei)

Stroma

# FIG. 5B

Image of abnormal prostate tissue

Epithelial cytoplasm and nuclei

Stroma

Gland Lumen

# FIG. 6

Features Selected for Prediction of PSA Recurrence (Example 2, Study 1)

| Feature | Description |
| --- | --- |
| Clinical | |
| | Biopsy Gleason Score |
| | Race |
| | UICC Stage |
| | Ploidy Result |
| | DRE Result |
| | Lymph Node Involvement |
| | Dominant Biopsy Gleason Grade |
| | Percent Ploidy in S Phase |
| | Post-operative Gleason Score |
| | TNM Stage |
| | Dominant Post-operative Gleason Grade |
| | Age |
| | Seminal Vesicle Involvement |
| | Pre-operative PSA |
| | Percent Ploidy Fraction |
| | Surgical Margin Involvement |
| | Extracapsular Involvement |
| Molecular | |
| | AR-tumor |
| | AR-gland |
| | CD34-tumor/PIN |
| | Ki67-tumor 2 |
| | CD45-Pin 3 |
| | CD34-tumor/stroma |
| | Ki-67-tumor 3 |
| | p27-tumor |
| | C14-PIN |
| | CD34-tumor |
| | PSA-gland |
| | PSMA-PIN |
| | CD34-PIN/stroma |
| | CD45-tumor 3 |
| Morphometric | |
| | Red Blood Cell Minimum Length in Pixels |
| | Epitheial Nuclei Maximum Compactness |
| | Lumen Minimum Radius of Smallest Enclosure |
| | Epithelial Nuclei Minimum Width in Pixels |
| | Stroma Maximum Density |
| | Lumen Maximum Border Length in Pixels |
| | Epithelial Nuclei Minimum Standard Deviation Channel 2 |
| | Epithelial Nuclei Maximum Radius of Smallest Enclosure |
| | Cytoplasm Standard Deviation of Border Length in Pixels |
| | Background Standard Deviation of Area in Pixels |

# FIG. 7A

AR

# FIG. 7B

CD34

# FIG. 8

Risk Groups for PSA Recurrence Using NNci Model Score

Low-Risk (33 Pts, 33 Recurrence-Free)

Intermediate-Risk (66 Pts, 61 Recurrence-Free)

High-Risk (33 Pts, 18 Recurrence-Free)

$p < 0.0001$

Actual Time to PSA Recurrence (months)
(Low: <25%, Intermediate: 25-75%, High: > 75%)

# FIG. 9

Features Selected for Prediction of PSA Recurrence (Example 2, Study 2)

| | | |
|---|---|---|
| Clinical | TNM Clinical Stage | 0.7431 |
| | Surgical Margins | 0.7937 |
| | Lymph Nodes | 0.8376 |
| Molecular | | |
| | AR Staining Index (tumor) | 0.8528 |
| Morphometric | | |
| | EpithelialNucleiMinCompactne0215 | 0.8222 |
| | StromaMaxStddevChannel30569 | 0.8483 |
| | CytoplasmStddevMaxDiff0148 | 0.8569 |
| | RedBloodCellMeanAreaPxl0386 | 0.8596 |
| | RedBloodCellStddevAreaPxl0388 | 0.8621 |
| | LumenMinAsymmetry0295 | 0.8635 |

# FIG. 10

Risk Groups for PSA Recurrence Using SVRc Model Score

Actual Time to PSA Recurrence (months)
Tick mark ( · ) indicates censored observation
(Low: <25%, Intermediate: 25-75%, High: > 75%)

# FIG. 11

## Features Selected for Prediction of Overall Survival (Example 2, Study 3)

| | | |
|---|---|---|
| Clinical | tnm | 0.7362 |
| | age | 0.7906 |
| Molecular | | |
| | psapsi | 0.7595 |
| Morphometric | | |
| | StromaMinMeanChannel10535 | 0.6804 |
| | RedBloodCellMeanStddevChann30474 | 0.7475 |
| | StromaMinMeanChannel20539 | 0.7722 |
| | RedBloodCellMinMeanChannel20443 | 0.7772 |
| | RedBloodCellStddeStddeChann20472 | 0.7809 |
| | StromaMaxMaxDiff0529 | 0.7852 |
| | EpitheNucleMeanBordeLengtPxl0206 | 0.7888 |
| | EpithelialNucleMeanAreaPxl0194 | 0.7921 |
| | EpithelNucleiStddevElliptFit0228 | 0.7951 |
| | RedBloodCellStddeStddeChann30476 | 0.7964 |
| | RedBloodCellStddevElliptiFit0420 | 0.7976 |

# FIG. 12

Risk Groups for Overall Survival Using SVRc Model Score

Low-Risk (77 Pts, 77 Alive)

Intermediate-Risk (122 Pts, 116 Alive)

High-Risk (69 Pts, 56 Alive)

$p < 0.0001$

Probability of Remaining Alive

Actual Time to Death (months)
Tick mark ( · ) indicates censored observation
(Low: <25%, Intermediate: 25-75%, High: > 75%)

# FIG. 13

### Features Selected for Prediction of Clinical Failure (Example 3)

| Clinical | Extracapsular Extension |
| --- | --- |
| | Seminal Vesicle Invasion |
| | Dominant Prostatectomy Gleason Grade |
| | Lymph Node Invasion |
| Morphometric | |
| | Cytoplasm Area Divided by Total Tissue Area |
| | Cytoplasm Standard Deviation of Mean Red Channel |
| | Lumen Area Divided by Total Tissue Area |

# FIG. 14

Features Selected for Prediction of PSA Recurrence (Example 5)

| Clinical | Lymph Nodes | -9.3742 |
|---|---|---|
| | Surgical Margin Involvement | -7.3159 |
| | Seminal Vesicle Involvement | -5.2103 |
| Molecular | AR (Tumor) Staining Index | -3.5404 |
| Morphometric | AreaStromadivTotTissueArea | -.34225 |
| | AreaEpitNucdivTotTissueArea | 3.2975 |

# FIG. 15

Features Selected for Prediction of PSA Recurrence (Example 6)

| Clinical | Lymph Nodes | -23.32 |
|---|---|---|
| | Surgical Margin Involvement | -11.73 |
| | Biopsy Gleason Score | -10.60 |
| | Seminal Vesicle Involvement | -6.40 |
| Molecular | AR (Tumor) Staining Index | -10.49 |
| Morphometric | AreaStromadivTotTissueArea | -16.15 |
| | AreaEpitNucdivTotTissueArea | 11.54 |
| | StromaMeanStddevRedChann | -11.26 |

# FIG. 16

Features Selected for Prediction of Clinical Failure
Post Prostatectomy (Example 7)

| | | |
|---|---|---|
| Clinical | Biopsy Gleason Score | -18.08 |
| | Lymph Node Involvement | -11.87 |
| | Specimen (Prostatectomy) Gleason Score | -6.36 |
| Molecular | AR intensity within AMACR epithelial cells | -6.20 |
| Morphometric | CytoplasmMeanMeanChannel | 19.68 |
| | StromaMeanStddevChannel | -10.06 |
| | EpitheNucleiStddevMeanChann | -8.87 |

# FIG. 17

Features Selected for Prediction of PSA Recurrence (Example 8)

| Clinical | Biopsy Gleason Score | -25.60 |
|---|---|---|
| | Seminal Vesicle Invasion | -22.69 |
| | Extracapsular Extension | -4.37 |
| | Preoperative PSA | -13.81 |
| | Dominant Prostatectomy Gleason Grade | -10.93 |
| | | |
| Molecular | Relative Area of AR+ Epithelial Nuclei | -10.64 |
| | | |
| Morphometric | Texture of Tumor Epithelial Nuclei | -19.21 |
| | Texture of Tumor Epithelial Cytoplasm | 18.19 |

# FIG. 18

4b. Nuclear Map: DAPI

4e. Epithelial (Nuclear) AR

4c. Epithelial Map: CK18

4a. M-PLEX: CK18, AR and DAPI

4d. AR Map

# FIG. 19

Features Selected for Prediction of Clinical Failure
Post Prostatectomy (Example 9)

| Clinical | Dominant Prostatectomy Gleason Grade | -16.4 |
|---|---|---|
| | Lymph Node Invasion Status | -25.938 |
| | | |
| Molecular | Average Intensity of the AR Channel in AR+/AMACR- | |
| | Epithelial Nuclei | -5.2075 |
| | | |
| Morphometric | Average Perimeter Length of Lumen | 17.217 |
| | Variation in Texture within Cytoplasm in Green Channel | 20.728 |
| | Relative Area of Lumen | 8.9663 |

## FIG. 20

**EP 2 145 276 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6409664 B, Kattan
- US 20050262031 A1
- US 991240
- US 99124004
- US 52093903

- US 06706605
- US 7321881 B
- US 6472415 B, Sovak
- US 6821767 B, French
- US 43878906

### Non-patent literature cited in the description

- **SCHERR D. et al.** *Urology,* February 2003, vol. 61, 14-24
- **SWINDLE P.W. et al.** *Urologic Clinics of North America,* May 2003, vol. 30 (2), 377-401
- **WAHLBY C. et al.** *Analytical Cellular Pathology,* 2002, vol. 24, 101-111
- Xcyt: A System for Remote Cytological Diagnosis and Prognosis of Breast Cancer. **STREET W.N.** In Soft Computing Techniques in Breast Cancer Prognosis and Diagnosis. CRC Press, 1999
- The Veteran's Administration Cooperative Urologic Research Group: Histologic Grading and Clinical Staging of Prostatic Carcinoma. **GLEASON D.F.** Urologic Pathology: The Prostate. Lea and Febiger, 1977, 171-198
- **CRISTIANNI et al.** An Introduction to Support Vector Machines. University Press, 2000
- **HASTIE.** The Elements of Statistical Learning. Springer, 2001
- **F.E. HARRELL et al.** Evaluating the yield of medical tests. *JAMA,* 1982, vol. 247 (18), 2543-2546
- **BISHOP, C.** Neural Networks for Pattern Recognition. Oxford University Press, 1995
- **FAUSETT, L.** Fundamentals of Neural Networks. Prentice Hall, 1994
- *Definiens Cellenger Architecture: A Technical Review,* April 2004
- Multiresolution Segmentation - An Optimization Approach for High Quality Multi-scale Image Segmentation. **BAATZ M. ; SCHÄPE A.** Angewandte Geographische Informationsverarbeitung. Wichmann-Verlag, 2000, 12-23
- **FUKUNAGA K.** Introduction to Statistical Pattern Recognition. Academic Press, 1990
- **DUDA R.O. et al.** Pattern Classification. John Wiley & Sons Inc, 2001
- **HOLMBERG L. et al.** A randomized trial comparing radical prostatectomy with watchful waiting in early prostate cancer. *N. Engl. M. Med.,* 2002, vol. 347, 781-789

- **POUND CR et al.** Natural history of progression after PSA elevation following radical prostatectomy. *JAMA,* 1999, vol. 281, 1591-1597
- **KUMAR-SINHA C. et al.** Molecular markers to identify patients at risk for recurrence after primary treatment for prostate cancer. *Urology,* 2003, vol. 62 (1), 19-35
- **COX D.R.** Regression Models and Life Tables. *Journal of the Royal Statistical Society,* 1972, vol. B 34, 187-220
- **HARRELL F.E.** Regression Modeling Strategies. Springer-Verlag, 2001
- **TUXHORN et al.** Reactive Stroma in Human Prostate Cancer: Induction of Myofibroblast Phenotype and Extracellular Matrix Remodeling. *Clinical Cancer Research,* September 2002, vol. 8, 2912-2923
- **KATTAN et al.** Postoperative Nomogram for Disease Recurrence After Radical Prostatectomy for Prostate Cancer. *Journal of Clinical Oncology,* May 1999, vol. 17 (5), 1499-1507
- **C. VONESCH ; F. AQUET ; J.L. VONESCH ; M. UNSER.** The colored revolution of bioimaging. *IEEE Signal Proc. Mag.,* May 2006, vol. 23 (3), 20-31
- **A. KRTOLICA ; C. O. DE SOLORZANO ; S. LOCKETT ; J. CAMPISI.** Quantification of epithelial cells in coculture with fibroblast by fluorescence image analysis. *Cytometry,* 2002, vol. 49, 73-82
- **A. GORDON ; A. COLMAN-LERNER ; T. E. CHIN ; K. R. BENJAMIN ; R. C. YU ; R. BRENT.** Single-cell quantification of molecules and rates using open-source microscope-based cytometry. *Nature Methods,* 2007, vol. 4, 175-181
- **R. CAMP ; G. G. CHUNG ; D. L. RIMM.** Automated subcellular localization and quantification of protein expression in tissue microarrays. *Nature Medicine,* 2002, vol. 8, 1323-1327
- **J. Y. RAO ; D. SELIGSON ; G. P. HEMSTREET.** Protein expression analysis using quantitative fluorescence image analysis on tissue microarray slides. *BioTechniques,* 2002, vol. 32, 924-932

**114**

- *Definiens Understanding Images, Developer Version 6,* 2007, http://www.definiens.com
- **A. TABESH ; M. TEVEROVSKIY ; H.-Y PANG ; V. P. KUMAR ; D. VERBEL ; A. KOTSIANTI ; O. SAIDI.** Multifeature prostate cancer diagnosis and Gleason grading of histological images. *IEEE Trans. Medical Imag.,* 2007, vol. 26, 1366-1378
- **GIACCONE G ; HERBST RS ; MANEGOLD C et al.** Gefitinib in combination with gemcitabine and cisplatin in advanced non-small-cell lung cancer: a phase III trial--INTACT 1. *J Clin Oncol,* 2004, vol. 22, 777-84
- **HERBST RS ; GIACCONE G ; SCHILLER JH et al.** Gefitinib in combination with paclitaxel and carboplatin in advanced non-small-cell lung cancer: a phase III trial--INTACT 2. *J Clin Oncol,* 2004, vol. 22, 785-94
- **THATCHER N ; CHANG A ; PARIKH P et al.** Gefitinib plus best supportive care in previously treated patients with refractory advanced non-small-cell lung cancer: results from a randomised, placebo-controlled, multicentre study (Iressa Survival Evaluation in Lung Cancer). *Lancet,* 2005, vol. 366, 1527-37
- **LYNCH TJ ; BELL DW ; SORDELLA R et al.** Activating mutations in the epidermal growth factor receptor underlying responsiveness of non-small-cell lung cancer to gefitinib. *N Engl J Med,* 2004, vol. 350, 2129-39
- **DUDEK AZ ; KMAK KL ; KOOPMEINERS J et al.** Skin rash and bronchoalveolar histology correlates with clinical benefit in patients treated with gefitinib as a therapy for previously treated advanced or metastatic non-small cell lung cancer. *Lung Cancer,* 2006, vol. 51, 89-96
- **CAPPUZZO F ; HIRSCH FR ; ROSSI E et al.** Epidermal growth factor receptor gene and protein and gefitinib sensitivity in non-small-cell lung cancer. *J Natl Cancer Inst,* 2005, vol. 97, 643-55
- **OKEN MM ; CREECH RH ; TORMEY DC ; HORTON J ; DAVIS TE ; MCFADDEN et al.** Toxicity and response criteria of the Eastern Cooperative Oncology Group. *Am J Clin Oncol,* 1982, vol. 5, 649-655
- **CORDON-CARDO C ; KOTSIANTI A ; DONOVAN M et al.** Improved prediction of PSA recurrence through systems pathology. *J Clin Oncol,* 2004, vol. 22, 4591
- **ZUBEK V ; VERBEL D ; SAIDI O.** Censored time trees for predicting time to PSA recurrence, in Proceedings of the Fourth International Conference on Machine Learning and Applications (ICMLA 2005). IEEE Computer Society, 2005, 221-226
- **PAEZ JG ; JANNE PA ; LEE JC et al.** EGFR mutations in lung cancer: correlation with clinical response to gefitinib therapy. *Science,* 2004, vol. 304, 1497-500

- **BELL DW ; GORE I ; OKIMOTO RA et al.** Inherited susceptibility to lung cancer may be associated with the T790M drug resistance mutation in EGFR. *Nat Genet,* 2005, vol. 37, 1315-6
- **HIRSCH FR ; VARELLA-GARCIA M ; BUNN PA, JR. et al.** Epidermal growth factor receptor in non-small-cell lung carcinomas: correlation between gene copy number and protein expression and impact on prognosis. *J Clin Oncol,* 2003, vol. 21, 3798-807
- **CHAN SK ; GULLICK WJ ; HILL ME.** Mutations of the epidermal growth factor receptor in non-small cell lung cancer -- search and destroy. *Eur J Cancer,* 2006, vol. 42, 17-23
- **RIELY GJ ; PAO W ; PHAM D et al.** Clinical course of patients with non-small cell lung cancer and epidermal growth factor receptor exon 19 and exon 21 mutations treated with gefitinib or erlotinib. *Clin Cancer Res,* 2006, vol. 12, 839-44
- **SEQUIST LV ; BELL DW ; LYNCH TJ et al.** Molecular predictors of response to epidermal growth factor receptor antagonists in non-small-cell lung cancer. *J Clin Oncol,* 2007, vol. 25, 587-95
- **UCHIDA A ; HIRANO S ; KITAO H et al.** Activation of downstream epidermal growth factor receptor (EGFR) signaling provides gefitinib-resistance in cells carrying EGFR mutation. *Cancer Sci,* 2007, vol. 98, 357-63
- **SUGIO K ; URAMOTO H ; ONO K et al.** Mutations within the tyrosine kinase domain of EGFR gene specifically occur in lung adenocarcinoma patients with a low exposure of tobacco smoking. *Br J Cancer,* 2006, vol. 94, 896-903
- **EDELMAN MJ.** An update on the role of epidermal growth factor receptor inhibitors in non-small cell lung cancer. *Semin Oncol,* 2005, vol. 32, 3-8
- **KRIS MG ; NATALE RB ; HERBST RS et al.** Efficacy of gefitinib, an inhibitor of the epidermal growth factor receptor tyrosine kinase, in symptomatic patients with non-small cell lung cancer: a randomized trial. *Jama,* 2003, vol. 290, 2149-58
- **AL-KURAYA K ; SIRAJ AK ; BAVI P et al.** High epidermal growth factor receptor amplification rate but low mutation frequency in Middle East lung cancer population. *Hum Pathol,* 2006, vol. 37, 453-7
- **HAINSWORTH JD ; MAINWARING MG ; THOMAS M et al.** Gefitinib in the treatment of advanced, refractory non-small-cell lung cancer: results in 124 patients. *Clin Lung Cancer,* 2003, vol. 4, 347-55
- **SU WP ; YANG CH ; YU CJ et al.** Gefitinib treatment for non-small cell lung cancer -- a study including patients with poor performance status. *J Formos Med Assoc,* 2005, vol. 104, 557-62
- **ERMAN M ; GRUNENWALD D ; PENAULT-LLORCA F et al.** Epidermal growth factor receptor, HER-2/neu and related pathways in lung adenocarcinomas with bronchioloalveolar features. *Lung Cancer,* 2005, vol. 47, 315-23

- **MASSION PP ; TAFLAN PM ; SHYR Y et al.** Early involvement of the phosphatidylinositol 3-kinase/Akt pathway in lung cancer progression. *Am J Respir Crit Care Med,* 2004, vol. 170, 1088-94
- **CAPPUZZO F ; MAGRINI E ; CERESOLI GL et al.** Akt phosphorylation and gefitinib efficacy in patients with advanced non-small-cell lung cancer. *J Natl Cancer Inst,* 2004, vol. 96, 1133-41
- **HAN SW ; HWANG PG ; CHUNG DH et al.** Epidermal growth factor receptor (EGFR) downstream molecules as response predictive markers for gefitinib (Iressa, ZD1839) in chemotherapy-resistant non-small cell lung cancer. *Int J Cancer,* 2005, vol. 113, 109-15
- **BAILEY L ; KRIS M ; WOLF M et al.** Tumor EGFR membrane staining is not clinically relevant for predicting response in patients receiving gifitinib (Iressa, ZD 1839) monotherapy for pretreated advanced non-small-cell lung cancer: IDEAL 1 and 2. *Proc Am Assoc Cancer Res,* 2003, vol. 44, 1362
- **BAILEY L ; JANAS M ; SCHMIDT K et al.** Evaluation of epidermal growth factor receptor (EGFR) as a predictive marker in patients with non-small-cell lung cancer (NSCLC) receiving first-line gefitinib combined with platinum-based chemotherapy. *J Clin Oncol,* 2004, vol. 22, 7013
- **JIN M ; INOUE S ; UMEMURA T et al.** Cyclin D1, p16 and retinoblastoma gene product expression as a predictor for prognosis in non-small cell lung cancer at stages I and II. *Lung Cancer,* 2001, vol. 34, 207-18
- **KEUM JS ; KONG G ; YANG SC et al.** Cyclin D1 overexpression is an indicator of poor prognosis in resectable non-small cell lung cancer. *Br J Cancer,* 1999, vol. 81, 127-32
- **KALISH LH ; KWONG RA ; COLE IE et al.** Deregulated cyclin D1 expression is associated with decreased efficacy of the selective epidermal growth factor receptor tyrosine kinase inhibitor gefitinib in head and neck squamous cell carcinoma cell lines. *Clin Cancer Res,* 2004, vol. 10, 7764-74
- **KOOMAGI R ; VOLM M.** Relationship between the expression of caspase-3 and the clinical outcome of patients with non-small cell lung cancer. *Anticancer Res,* 2000, vol. 20, 493-6
- **TAKATA T ; TANAKA F ; YAMADA T et al.** Clinical significance of caspase-3 expression in pathologic-stage I, nonsmall-cell lung cancer. *Int J Cancer,* 2001, vol. 96, 54-60
- **MINEO TC ; AMBROGI V ; BALDI A et al.** Prognostic impact of VEGF, CD31, CD34, and CD105 expression and tumour vessel invasion after radical surgery for IB-IIA non-small cell lung cancer. *J Clin Pathol,* 2004, vol. 57, 591-7